# EUROPEAN PATENT APPLICATION

(11) **EP 4 800 029 A1**
(43) Date of publication of application: **02.09.2026**
(21) Application number: 24898200.1
(22) Date of filing: 29.11.2024
(51) Int. Cl.: C07K 14/52, C12N 15/86, A61K 48/00, A61P 27/02, A61K 38/00

(54) **NOVEL CHEMOKINE-LIKE PROTEIN FRAGMENTS**

(30) Priority: 30.11.2023 KR 20230171078; 26.11.2024 KR 20240171247
(71) Applicant: Elisigen Inc., Seoul 02841 (KR)
(72) Inventor: KIM, Youyoung, Seoul 02577 (KR); KIM, Jee Yong, Gwacheon-si, Gyeonggi-do 13836 (KR); SHIM, Juwon, Seoul 04702 (KR); LEE, Jaewoo, Seoul 01913 (KR); LEE, Junwoo, Seoul 03935 (KR); JANG, Sejeong, Seoul 08796 (KR); HAN, Joo Seok, Seoul 02725 (KR)
(74) Representative: V.O.
(86) International application number: PCT/KR2024/019273
(87) International publication number: WO 2025/116606

(57) **Abstract**

The present disclosure relates to a TAFA protein, a fragment thereof, or a variant thereof having the ability to increase neurite length or the number of branch points. The present disclosure also relates to a pharmaceutical composition comprising a polypeptide comprising the amino acid sequence of the TAFA protein, the fragment, or the variant, a nucleic acid molecule encoding the polypeptide, a vector comprising the nucleic acid molecule, a recombinant viral particle comprising the vector and a capsid protein, a cell comprising the vector, a cell transformed with the vector, or any combination thereof, and to therapeutic uses thereof. The composition of the present disclosure can be usefully employed for preventing or treating retinal neurodegenerative diseases and/or neuropathic pain by restoring damaged retina (for example, cone cells of photoreceptors, etc.) or by increasing the length of neurites of neurons.

## Description

### BACKGROUND OF THE INVENTION

The present disclosure relates to TAFA proteins, TAFA protein fragments, or variants thereof which have the ability to increase neurite length and/or the number of branch points.

The TAFA protein family is a group of proteins that are abundantly expressed throughout brain regions and consists of five proteins, TAFA1 to TAFA5. These proteins contain structurally conserved cysteine residues, and C, CC and CXC motifs between the cysteine residues are associated with chemokines. TAFA1 to TAFA4 have a high degree of similarity in the number and spacing of cysteine residues, whereas TAFA5 has fewer cysteine residues than these.

These proteins are evolutionarily highly conserved in vertebrates and have been shown to be important for the normal functioning of the central nervous system. In mice in which TAFA1 is knocked out, weight loss, decreased anxiety-like behavior, and impairment of fear memory have been reported (Lei X, Liu L, Terrillion CE, et al., FASEB J., 2019, 33(12):14734-14747 and Yong HJ, Ha N, Cho EB, et al., Sci Rep., 2020, 10(1):3969). Conversely, when TAFA2 and TAFA3 are knocked out, an increase in anxiety-like behaviors has been reported (Choi JH, Jeong YM, Kim S, et al., Proc Natl Acad Sci U S A, 2018, 115(5):E1041-E1050 and Kim S, Lee B, Choi JH, Kim JH, Kim CH, Shin HS, Sci Rep., 2017, 7(1):16503). In TAFA4-null mice, it has been reported that allodynia and hyperalgesia occur (Delfini MC, Mantilleri A, Gaillard S, et al., Cell Rep., 2013, 5(2):378-388). In mice in which TAFA5 is knocked out, behavioral changes such as increased depressive-like behavior and loss of spatial memory have been reported (Huang S, Zheng C, Xie G, et al., "FAM19A5/TAFA5, a novel neurokine, plays a crucial role in depressive-like and spatial memory-related behaviors in mice", Mol Psychiatry., 2021, 26(6):2363-2379).

Despite the fact that the roles of these proteins in the central nervous system are important, what is known about the functions of each protein and their pathophysiological or therapeutic functions is very limited, and what has been elucidated about their roles outside the central nervous system is also limited.

The matters described above as background art are provided only for the purpose of improving the understanding of the background of the present invention, and should not be construed as an acknowledgement that they correspond to prior art already known to those skilled in the art.

### BRIEF SUMMARY OF THE INVENTION

An object of the present disclosure is to provide a polypeptide having the ability to increase neurite length and/or the number of branch points.

The polypeptide may reverse or restore loss of or functional abnormalities of cone cells, which are one type of photoreceptor cell.

The polypeptide may comprise an amino acid sequence of a TAFA (TAFA Chemokine Like Family Member) protein, a fragment thereof, or a variant thereof.

The polypeptide may comprise, from the N-terminus to the C-terminus, an amino acid sequence of General Formula 7 below:

General Formula 1 X1-X2-X3-G-T-C-E-V-X4-A-X5-H-X6-C-C-N-X7-N-X8-I-E-E-X9-S-Q-T-X10-X11-C-S-C- X12-X13-G-X14-V-A-G-T-T-X15-X16-X17-P-S-C-V-X18-A-X19-I-V-X20-X21-X22-W-W- C-X23-M-X24-P-C-X25-X26-G-E-X27-C-K-X28-L-P-D-X29-X30-G-W-X31-C-X32-X33- G-X34-K-X35-K-T-T-X36-X37-X38-X39

In General Formula 7,
X1 is nonexistent, V, I or L;
X2 is K, E, R or Q;
X3 is G, T, Q, P or A;
X4 is V or I;
X5 is A, L, V or I;
X6 is R or L;
X7 is K, R or Q;
X8 is R or K;
X9 is R or L;
X10 is V or G;
X11 is K or N;
X12 is F or L;
X13 is P or S;
X14 is Q or K;
X15 is R, H or Q;
X16 is A, N, S or T;
X17 is A, Q, R, K or T;
X18 is D or E;
X19 is S or A;
X20 is I, E, L, A or V;
X21 is Q, G or E;
X22 is K or R;
X23 is H, Q or E;
X24 is E, Q, N, D, S or H;
X25 is L, V or M;
X26 is E, D, P, L or A;
X27 is E or D;
X28 is V, T, A or I;
X29 is L, N, R, Y, S or Q;
X30 is S, K or T;
X31 is S or M;
X32 is S, A or Y;
X33 is S, T or R;
X34 is N or H;
X35 is V or I;
X36 is R or K;
X37 is nonexistent, V, A, G, M or N;
X38 is nonexistent, T, I, N, F or S; and
X39 is nonexistent, R, H, V, K, I or Q.

The polypeptide may comprise one or more amino acid sequences selected from the group consisting of the amino acid sequences set forth in SEQ ID NOs: 87 to 141.

The polypeptide may consist of 8 to 61 amino acid residues.

The polypeptide may comprise an amino acid sequence having at least 50% sequence identity with the amino acid sequence of SEQ ID NO: 1, SEQ ID NO: 15 or SEQ ID NO: 87.

The amino acid sequence of the polypeptide may comprise, from the N-terminus to the C-terminus, an amino acid sequence of General Formula 1 below:

General Formula 1 X1-G-E-X2-C-K-X3-L

In General Formula 1,
X1 is E, D, P, L or A;
X2 is D or E; and
X3 is T, V, I or A.

The amino acid sequence of the polypeptide comprising the amino acid sequence of General Formula 1 may comprise the amino acid sequence of SEQ ID NO: 142.

The amino acid sequence of the polypeptide comprising the amino acid sequence of General Formula 1 may comprise an amino acid sequence selected from the group consisting of the amino acid sequences of SEQ ID NOs: 1 to 14.

The amino acid sequence of the polypeptide comprising the amino acid sequence of General Formula 1 may consist of 8 to 43 amino acid residues.

The amino acid sequence of the polypeptide may comprise, from the N-terminus to the C-terminus, an amino acid sequence of General Formula 2 below:

General Formula 2 I-V-X4-X5-X6-W-W-C-X7-M-X8-P-C-X9-X1-G-E-X2-C-K-X3-L

In General Formula 2,
X1 is E, D, P, L or A;
X2 is D or E;
X3 is T, V, I or A;
X4 is I, E, A, L or V;
X5 is Q, E or G;
X6 is K or R;
X7 is E, H or Q;
X8 is E, Q, N, D, S or H; and
X9 is L, M or V.

The amino acid sequence of the polypeptide comprising the amino acid sequence of General Formula 2 may comprise the amino acid sequence of SEQ ID NO: 143. The amino acid sequence of the polypeptide comprising the amino acid sequence of General Formula 2 may comprise an amino acid sequence selected from the group consisting of the amino acid sequences of SEQ ID NOs: 28 to 51.

The amino acid sequence of the polypeptide may comprise, from the N-terminus to the C-terminus, an amino acid sequence of General Formula 3 below:

General Formula 3 X1-G-E-X2-C-K-X3-L-P-D-X4-X5-G-W-S-C-S-X6-G-N-K-X7-K-T-T-K-V-T-R

In General Formula 3,
X1 is E, D, P, L or A;
X2 is D or E;
X3 is T, V, I or A;
X4 is Y, S or L;
X5 is S or T;
X6 is S or T; and
X7 is V or I.

The amino acid sequence of the polypeptide comprising the amino acid sequence of General Formula 3 may comprise the amino acid sequence of SEQ ID NO: 144.

The amino acid sequence of the polypeptide comprising the amino acid sequence of General Formula 3 may comprise an amino acid sequence selected from the group consisting of the amino acid sequences of SEQ ID NOs: 52 to 58.

The amino acid sequence of the polypeptide may comprise one or more amino acid sequences selected from the group consisting of the amino acid sequences of SEQ ID NOs: 152 to 171.

The amino acid sequence of the polypeptide may comprise, from the N-terminus to the C-terminus, an amino acid sequence of General Formula 4 below:

General Formula 4 G-X1-V-A-G-T-T-X2-X3-X4-P-S-C-V-X5-X6-X7

In General Formula 4,
X1 is Q or K;
X2 is R, H or Q;
X3 is A, N, S or T;
X4 is R, A, Q, K or T;
X5 is D or E;
X6 is A or nonexistent; and
X7 is S, A or nonexistent.

The amino acid sequence of the polypeptide comprising the amino acid sequence of General Formula 4 may comprise the amino acid sequence of SEQ ID NO: 145.

The amino acid sequence of the polypeptide comprising the amino acid sequence of General Formula 4 may comprise an amino acid sequence selected from the group consisting of the amino acid sequences of SEQ ID NOs: 15 to 27.

The amino acid sequence of the polypeptide comprising the amino acid sequence of General Formula 4 may consist of 15 to 46 amino acid residues.

The amino acid sequence of the polypeptide may comprise, from the N-terminus to the C-terminus, an amino acid sequence of General Formula 5 below:

General Formula 5 X8-I-E-E-X9-S-Q-T-X10-X11-C-S-C-X12-X13-G-X1-V-A-G-T-T-X2-X3-X4-P-S-C-V-X5-X 6-X7

In General Formula 5,
X1 is Q or K;
X2 is R, H or Q;
X3 is A, N, S or T;
X4 is R, A, Q, K or T;
X5 is D or E;
X6 is A or nonexistent;
X7 is S, A or nonexistent;
X8 is R or K;
X9 is R or L;
X10 is V or G;
X11 is K or N;
X12 is F or L; and
X13 is P or S.

The amino acid sequence of the polypeptide comprising the amino acid sequence of General Formula 5 may comprise the amino acid sequence of SEQ ID NO: 146.

The amino acid sequence of the polypeptide comprising the amino acid sequence of General Formula 5 may comprise an amino acid sequence selected from the group consisting of the amino acid sequences of SEQ ID NOs: 59 to 74.

The amino acid sequence of the polypeptide may comprise, from the N-terminus to the C-terminus, an amino acid sequence of General Formula 6 below:

General Formula 6 G-X1-V-A-G-T-T-X2-X3-X4-P-S-C-V-X5-X6-X7-I-V-X8-X9-K-W-W-C-X10-M-X11-P-C-X 12

In General Formula 6,
X1 is Q or K;
X2 is R, H or Q;
X3 is A, N, S or T;
X4 is R, A, Q, K or T;
X5 is D or E;
X6 is A or nonexistent;
X7 is S, A or nonexistent;
X8 is I, A, V or L;
X9 is Q or E;
X10 is H or Q;
X11 is N, D, S or H; and
X12 is L or M.

The amino acid sequence of the polypeptide comprising the amino acid sequence of General Formula 6 may comprise the amino acid sequence of SEQ ID NO: 147.

The amino acid sequence of the polypeptide comprising the amino acid sequence of General Formula 6 may consist of 75 to 85 amino acid residues.

The amino acid sequence of the polypeptide comprising the amino acid sequence of General Formula 6 may comprise one or more amino acid sequences selected from the group consisting of the amino acid sequences of SEQ ID NOs: 172 to 184.

Another object of the present disclosure is to provide a nucleic acid molecule encoding the polypeptide.

A further object of the present disclosure is to provide a vector comprising the nucleic acid molecule.

A further object of the present disclosure is to provide a recombinant virus particle comprising the vector and a capsid protein.

The virus may be an AAV.

A further object of the present disclosure is to provide a cell comprising the vector.

A further object of the present disclosure is to provide a cell transformed with the vector.

A further object of the present disclosure is to provide a composition comprising the polypeptide, the nucleic acid molecule encoding the polypeptide, the vector comprising the nucleic acid molecule, the recombinant virus particle comprising the vector and the capsid protein, the cell comprising the vector, the cell transformed with the vector, or any combination thereof.

The composition may be a pharmaceutical composition.

The pharmaceutical composition may be a composition for preventing or treating a retinal neurodegenerative disease.

The retinal neurodegenerative disease may comprise retinopathy, choroidal neovascularization, maculopathy, macular degeneration, retinal degeneration, macular edema, retinal edema, macular swelling, retinal swelling, color vision deficiency, retinal cell degeneration, retinal vascular occlusion, retinal detachment, an inherited retinal disease, or any combination thereof.

The pharmaceutical composition may be a composition for preventing or treating neuropathic pain.

The neuropathic pain may be allodynia, hyperalgesia, hyperesthesia, or dysesthesia.

The neuropathic pain may be central neuropathic pain or peripheral neuropathic pain.

The neuropathic pain may be neuralgia, deafferentation pain syndrome, complex regional pain syndrome, or a (central or peripheral) neuropathy.

A further object of the present disclosure is to provide a method for producing a composition, comprising a step of producing a composition comprising the polypeptide, the nucleic acid molecule encoding the polypeptide, the vector comprising the nucleic acid molecule, the recombinant virus particle comprising the vector and the capsid protein, the cell comprising the vector, the cell transformed with the vector, or any combination thereof.

A further object of the present disclosure is to provide the therapeutic use of the polypeptide, the nucleic acid molecule encoding the polypeptide, the vector comprising the nucleic acid molecule, the recombinant virus particle comprising the vector and the capsid protein, the cell comprising the vector, the cell transformed with the vector, or any combination thereof, for the manufacture of a medicament.

A further object of the present disclosure is to provide a method for preventing or treating a disease or disorder in a subject in need thereof, the method comprising administering to the subject a composition comprising the polypeptide, the nucleic acid molecule encoding the polypeptide, the vector comprising the nucleic acid molecule, the recombinant virus particle comprising the vector and the capsid protein, the cell comprising the vector, the cell transformed with the vector, or any combination thereof.

Other objects and advantages of the present invention will become more apparent from the following detailed description of the invention, the claims, and the drawings.

### [Technical Solution]

### I. Definitions

Throughout the present disclosure, the term "at least" preceding a single number or a series of numbers is understood to include the number that follows the term "at least" as well as all subsequent numbers or integers that can logically be included in view of the context. For example, the number of nucleotides in a nucleic acid molecule must be an integer. Thus, "at least 18 nucleotides of a 21-nucleotide nucleic acid molecule" means that 18, 19, 20, or 21 nucleotides have the indicated property. When "at least" appears before a series of numbers or a range, it is understood that "at least" can modify each of the numbers in the series or range. "At least" is also not limited to integers for example, "at least 5%" includes 5.0%, 5.1%, and 5.18% regardless of the number of significant figures.

Where aspects are described in the present specification with the term "comprising", it is understood that other analogous aspects described in terms of "consisting of" and/or "consisting essentially of" are also provided.

Unless defined otherwise, all technical and scientific terms used herein have the same meaning as commonly understood by one of ordinary skill in the art to which the present disclosure pertains. For example, the Concise Dictionary of Biomedicine and Molecular Biology, Juo, Pei-Show, 2nd ed., 2002, CRC Press the Dictionary of Cell and Molecular Biology, 3rd ed., 1999, Academic Press and the Oxford Dictionary of Biochemistry and Molecular Biology, Revised, 2000, Oxford University Press provide those of skill in the art with a general dictionary of many of the terms used in the present disclosure.

Units, prefixes, and symbols are denoted in their Systeme International de Unites (SI) accepted form. Numeric ranges are inclusive of the numbers defining the range. Unless otherwise indicated, amino acid sequences are written left to right in amino to carboxy orientation. The headings provided herein are not limitations on the various aspects of the disclosure, which can be had by reference to the specification as a whole. Accordingly, the terms defined immediately below are more fully defined by reference to the specification in its entirety.

As used herein, the term "about" means approximately, roughly, around, or in the region of. When the term "about" is used in conjunction with a numerical range, it modifies that range by extending the boundaries above and below the numerical values set forth. In general, the term "about" can modify a numerical value above and below the stated value by, for example, a variance of 10 percent, higher or lower.

As used herein, the term "family with sequence similarity 19" or "FAM19" or "TAFA" refers to a protein that belongs to the TAFA family of five proteins (also known as the FAM19 family) and is primarily expressed in the brain and the spinal cord. FAM19A1 is also known as TAFA1, FAM19A2 as TAFA2, FAM19A3 as TAFA3, FAM19A4 as TAFA4, and FAM19A5 as TAFA5.

The human TAFA1 gene encodes a sequence of 133 amino acids, and although there is disagreement over the length of the signal peptide and mature protein, the human TAFA1 protein is expected to consist of a signal sequence of 35 amino acids and a mature protein of 98 amino acids. TAFA1 is highly expressed in the frontal, temporal, occipital and parietal cortices, and expressed at lower levels in the basal ganglia, lateral tubes and cerebellum. Through cell experiments, TAFA1 has been shown to influence the determination of the differentiation fate of neural stem cells, inhibit the differentiation of neural stem cells into astrocytes, and promote their differentiation into nerve cells. Experiments using TAFA1 knock-out (KO) mice have shown that TAFA1 can regulate locomotor activity, anxiety-like behaviors, learning and memory, and somatosensory functions.

The human TAFA2 gene encodes a sequence of 131 amino acids, and although there is disagreement over the length of the signal peptide and mature protein, the human TAFA2 protein is expected to consist of a signal sequence of 30 amino acids and a mature protein of 101 amino acids. TAFA2 is expressed most abundantly in the occipital, frontal cortices, and medulla oblongata within the central nervous system. It is known that, when recombinant TAFA2 protein is injected into the third ventricle of mice, the food intake and meal frequency, energy expenditure, and respiratory exchange rate are increased. This suggests that TAFA2 may play a role in regulating food intake and energy metabolism. It is also known that inhibition of TAFA2 in zebrafish and mice increases anxiety-like behaviors.

The human TAFA3 gene encodes a sequence of 133 amino acids, and although there is disagreement over the length of the signal peptide and mature protein, the human TAFA3 protein is expected to consist of a signal sequence of 30 amino acids and a mature protein of 103 amino acids. It is known that, in a mouse model of transient focal cerebral ischemia, the expression of TAFA3 is increased in microglia and the microglia treated with TAFA3 are polarized into anti-inflammatory microglia. In addition, when TAFA3 is knocked out in a mouse model, three major behavioral deficits are observed on the autism spectrum disorders, such as decreased response to social novelty, impaired social communication, and increased repetitive behavior. This suggests that TAFA3 is involved in normal functioning of social relationships formation.

The human TAFA4 gene encodes a sequence of 140 amino acids, and although there is disagreement over the length of the signal peptide and mature protein, the human TAFA4 protein is expected to consist of a signal sequence of 45 amino acids and a mature protein of 95 amino acids. TAFA4 is expressed mainly in sensory neurons of the peripheral nervous system. The TAFA4 protein is expressed specifically in C low-threshold mechanoreceptors and appears to reduce pain by regulating the activity of interneurons, especially GABAergic neurons.

The human TAFA5 gene encodes a sequence of 132 amino acids, and although there is disagreement over the length of the signal peptide and mature protein, the human TAFA5 protein is expected to consist of a signal peptide of 43 amino acids and a mature protein of 89 amino acids. TAFA5 is highly expressed in the basal ganglia region and cerebellum. It is known that the expression of TAFA5 in the hypothalamus of mice is increased by inflammatory stimuli such as TNF-α and that, when TAFA5 is knocked out, suppression of food intake, body weight loss and increased inflammatory cytokines induced by TNF-α are partially reversed.In the case of TAFA1 to TAFA4 mature proteins, they exhibit high sequence identity (e.g., human TAFA4 has 73.7% sequence identity with TAFA1, 85.3% sequence identity with TAFA2, and 81.1% sequence identity with TAFA3). On the other hand, TAFA5 is expected to have a completely different function since it exhibits low sequence identity with TAFA1 to 4 (e.g., human TAFA5 has 48.9% sequence identity with TAFA1, 51.1% sequence identity with TAFA2, 47.7% sequence identity with TAFA3, and 50.0% sequence identity with TAFA4).

Human TAFA4 (TAFA chemokine-like family member 4) protein (SEQ ID NO 299) is a member of the TAFA protein family (TAFA1-5) that is abundantly expressed throughout brain regions. TAFA4, consisting of 140 amino acids, has 95 or 93 amino acids, except for the 45 or 47 amino acids in the preceding part, which are evolutionarily very well conserved among vertebrates. In addition, among the TAFA protein groups, TAFA1 to 4 are found to have very well conserved interspecies sequence identity within the 93 amino acid sequence in vertebrates. i) The human mature TAFA4 amino acid sequence shows sequence identity 90% or higher compared to TAFA4 in mammals and amphibians, 95% or higher compared to TAFA4 in birds, 85% or higher compared to TAFA4 in reptiles and fish. ii) The human TAFA1 amino acid sequence shows sequence identity 90% or higher compared to TAFA1 in mammals and amphibians, 95% or higher compared to TAFA1 in birds, and 85% or higher compared to TAFA1 in reptiles and fish. iii) The human TAFA2 amino acid sequence shows sequence identity 90% or higher compared to TAFA2 in mammals and amphibians, 95% or higher compared to TAFA2 in birds, and 80% or higher compared to TAFA2 in reptiles and fish. And, iv) the human TAFA3 amino acid sequence shows sequence identity 75% or higher compared to TAFA3 in mammals and amphibians, 80% or higher compared to TAFA3 in birds, and 75% or higher compared to TAFA3 in reptiles and fish. It can be seen that they exhibit very high sequence identity regardless of the species.

As used herein, the term "TAFA protein fragment" refers to a fragment of a TAFA1 to TAFA4 protein that has the ability to increase neurite length and/or the number of branch points. The fragment may consist of 8 (for example, TAFA fragment 3.7), 9, 10, 11, 12, 13, 14, 15, 16, 17 (for example, TAFA fragment 2.5), 18, 19, 20, 21, 22 (for example, TAFA fragment 7), 23, 24, 25 (for example, TAFA fragment 2.5+3.7), 26, 27, 28, 29 (for example, TAFA fragment 3), 30, 31 (for example, TAFA fragment 2), 32 (for example, TAFA fragment 5), 33, 34, 35, 36, 37, 38, 39 (for example, TAFA fragment 2.5+7 or TAFA fragment 2+3.7), 40, 41, 42, 43, 44, 45, 46, 47, 48, 49, 50, 51, 52, 53, 54 (for example, TAFA fragment 5+7), 55, 56, 57, 58, 59, 60 (for example, TAFA fragment 2+3) or 61 (for example, TAFA fragment 5+3) amino acid residues, but is not limited thereto.

As used herein, the term "variant of a TAFA protein fragment" refers to a polypeptide in which some of the amino acids of a polypeptide constituting a TAFA1 to TAFA4 protein fragment are substituted with other amino acids. The variant is preferably a functional variant. The term "functional" refers to a variant derived from a fragment of a TAFA1 to TAFA4 protein that, like the "TAFA protein fragment", has the ability to increase neurite length and/or the number of branch points. The variant is preferably an interspecies variant (for example, a polypeptide comprising any one of the amino acid sequences of SEQ ID NOs: 142 to 147). Those skilled in the art can readily prepare a polypeptide in which one or more amino acids are substituted with other amino acids by using differences in non-conserved sequences, excluding conserved sequences, among the amino acid sequences of TAFA1 to TAFA4 fragments for each known species. For example, a polypeptide can be produced by substituting the fourth residue D with E in the human TAFA4 fragment 3.7 sequence EGEDCKVL (SEQ ID NO: 1) by reference to the rabbit TAFA2 fragment 3.7 sequence EGEECKVL (SEQ ID NO: 6), or by substituting the first residue E with P and the fourth residue D with E in the pig TAFA3 fragment 3.7 sequence PGEECKVL (SEQ ID NO: 9).

The number of amino acid substitutions may be from 1 to 22 amino acids. For example, when the TAFA protein fragment simultaneously comprises fragment 5 and fragment 7, 1 to 22 amino acid substitutions are possible; when the TAFA protein fragment simultaneously comprises fragment 2 and fragment 3, 1 to 19 amino acid substitutions are possible; when the TAFA protein fragment comprises only fragment 2, 1 to 12 amino acid substitutions are possible; when the TAFA protein fragment comprises only fragment 5, 1 to 13 amino acid substitutions are possible; when the TAFA protein fragment comprises only fragment 3, 1 to 7 amino acid substitutions are possible; when the TAFA protein fragment comprises only fragment 7, 1 to 9 amino acid substitutions are possible; when the TAFA protein fragment comprises only fragment 2.5, 1 to 7 amino acid substitutions are possible; and when the TAFA protein fragment comprises only fragment 3.7, 1 to 3 amino acid substitutions are possible.

Preferably, the number of amino acid substitutions is 1 to 20, 1 to 19, 1 to 18, 1 to 17, 1 to 16, 1 to 15, 1 to 14, 1 to 13, 1 to 12, 1 to 11, 1 to 10, 1 to 9, 1 to 8, 1 to 7, 1 to 6, 1 to 5, 1 to 4, or 1 to 3.

As used herein, the term "adeno-associated virus" or "AAV" refers to a single-stranded DNA virus that is a helper-dependent human parvovirus. The genome size is about 4.6 kbp, the N-terminal portion of the genome encodes the rep gene involved in viral replication and expression of viral genes, and the C-terminal portion encodes the cap gene encoding the capsid protein of the virus, and comprises repeat regions ITRs in which about 145 bases are inserted at both ends. The ITRs (inverted terminal repeats) having a T-shaped structure and a size of 145 bp function as the origin of replication during viral genome replication and act as the primary packaging signal. Since the ITR is the only cis-acting nucleotide sequence required to make recombinant AAV constructs, and has enhancer activity in the presence of the Rep protein but has very weak activity in the absence of the Rep protein, it is necessary, when cloning a transgene into a recombinant AAV construct, to take this into account and construct an expression construct by appropriately arranging enhancers, promoters, pA, etc. (RJ Samulski and N Muzyczka, Annu. Rev. Virol., 2014, 1:427-451). Four proteins are translated from the rep gene, which are classified according to their molecular weights as rep78, rep68, rep52, and rep40, and they perform important functions in AAV DNA replication. Four proteins are translated from the cap gene, of which the VP1, VP2, and VP3 proteins are structural proteins that constitute AAV particles, and the assembly-activating protein (AAP) promotes formation of AAV particles by the structural proteins. For the adeno-associated virus to replicate efficiently, some proteins and RNAs derived from helper viruses such as adenovirus or herpes simplex virus are required (Muzyczka N., Curr Top Microbiol Immunol, 158:97-129, 1992).

AAV includes, but is not limited to, AAV type 1, AAV type 2, AAV type 3 (including types 3A and 3B), AAV type 4, AAV type 5, AAV type 6, AAV type 7, AAV type 8, AAV type 9, AAV type 10, AAV type 11, AAV type 12, AAV type 13, AAVrh.74, snake AAV, avian AAV, bovine AAV, canine AAV, equine AAV, ovine AAV, goat AAV, shrimp AAV, those AAV serotypes and clades disclosed by Gao et al., (J. Virol., 78:6381 2004) and Moris et al., (Virol., 33:375 2004), and any other AAV now known or later discovered. See, for example, FIELDS et al., VIROLOGY, volume 2, chapter 69, 4th ed., Lippincott-Raven Publishers. In some aspects, "AAV" includes derivatives of known AAVs. In some aspects, "AAV" includes modified or artificial AAVs. In some aspects, "AAV" includes AAVs having a modified capsid.

As used herein, the terms "administration", "administering" and grammatical variants thereof refer to introducing a composition (for example, a polypeptide comprising an amino acid sequence of a TAFA protein, a fragment thereof, or a variant thereof as described herein) into a subject via a pharmaceutically acceptable route. The composition is introduced into a subject by any suitable route, including intratumorally, orally, pulmonarily, intranasally, parenterally (for example, intravenously, intra-arterially, intramuscularly, intraperitoneally, or subcutaneously), rectally, intralymphatically, intrathecally, periocularly, intraocularly, or topically (for example, eye drops, nasal administration, etc.). Administration includes self-administration and administration by another. A composition or formulation exerts its intended function by a suitable route of administration. For example, if a suitable route is intravenous, the composition is administered by introducing the composition or formulation into a vein of the subject. The pharmaceutical composition can be formulated for topical or local application, for example as eye drops, gels, creams, or lotions for application to the eye, skin, or mucosa, and for intrathecal or intracisternal administration. Topical administration may be considered for ocular, transdermal, or mucosal (for example, nasal, oral, rectal, etc.) administration or for inhalation therapy. The composition can be administered alone or together with other pharmaceutically acceptable excipients.

As used herein, the term "intraocular" refers to within or under ocular tissues. As used herein, the term "intraocular administration" refers to any administration capable of delivering a composition to a sub-Tenon, subconjunctival, suprachoroidal, subretinal, intravitreal, or similar site in the eye. In some aspects, intraocular administration includes suprachoroidal, subretinal, and intravitreal administration.

As used herein, the term "conserved" refers to each nucleotide or amino acid residue of a polynucleotide or polypeptide sequence that appears unchanged at the same position in each of two or more sequences being compared. Relatively conserved nucleotides or amino acids are those that are conserved among related sequences rather than other nucleotides or amino acids appearing at other positions in the sequence.

As used herein, the term "amino acid" as used herein includes not only the 20 standard amino acids that are naturally incorporated into peptides (arginine (R), lysine (K), histidine (H), glutamic acid (E), aspartic acid (D), glutamine (Q), asparagine (N), leucine (L), isoleucine (I), valine (V), methionine (M), phenylalanine (F), tryptophan (W), tyrosine (Y), glycine (G), alanine (A), serine (S), threonine (T), proline (P), and cysteine (C)), but also D-isomers and modified amino acids. In addition, the peptide may include non-standard amino acids that have undergone post-translational modification. Post-translational modifications may include, but are not limited to, phosphorylation, glycosylation, acylation (for example, acetylation, myristoylation, and palmitoylation), alkylation, carboxylation, hydroxylation, glycation, biotinylation, ubiquitinylation, changes in chemical properties (for example, beta-elimination deimidation, and deamidation), and structural changes (for example, formation of disulfide bridges). The peptide may be a wild-type peptide identified and isolated from a natural source. Alternatively, the peptide may be an artificial variant comprising an amino acid sequence in which one or more amino acids are substituted, deleted, and/or inserted. Amino acid modifications in the artificial variants as well as in the wild-type polypeptide may include conservative amino acid substitutions that do not significantly affect protein folding and/or activity. For example, such conservative substitutions may occur among basic amino acids (arginine (R), lysine (K), and histidine (H)), acidic amino acids (glutamic acid (E) and aspartic acid (D)), polar amino acids (glutamine (Q) and asparagine (N)), hydrophobic amino acids (leucine (L), isoleucine (I), valine (V), and methionine (M)), aromatic amino acids (phenylalanine (F), tryptophan (W), and tyrosine (Y)), and small amino acids (glycine (G), alanine (A), serine (S), and threonine (T)). In general, amino acid substitutions that do not alter specific activity are well known in the art. The most frequently occurring substitutions may include Ala/Ser, Val/Ile, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/Ile, Leu/Val, Ala/Glu, and Asp/Gly.

In some aspects, when two or more sequences are 100% identical to one another, they are said to be "fully conserved" or "identical". In some aspects, when two or more sequences are at least 70% identical, at least 80% identical, at least 90% identical, or at least 95 identical to one another, they are said to be "highly conserved". In some aspects, when two or more sequences are about 70% identical, about 80% identical, about 90% identical, about 95% identical, about 98% identical, or about 99% identical to one another, they are said to be "highly conserved". In some aspects, when two or more sequences are at least 30% identical, at least 40% identical, at least 50% identical, at least 60% identical, at least 70% identical, at least 80% identical, at least 90% identical, or at least 95% identical to one another, they are said to be "conserved". In some aspects, when two or more sequences are about 30% identical, about 40% identical, about 50% identical, about 60% identical, about 70% identical, about 80% identical, about 90% identical, about 95% identical, about 98% identical, or about 99% identical to one another, they are said to be "conserved". Conservation of a sequence can apply to the full length of a polynucleotide or polypeptide or to a portion, region, or feature thereof.

The terms "complementary" and "complementarity" refer to the relationship between two or more oligomers that each comprise a nucleotide sequence, or between an oligomer and a target gene, as determined by Watson-Crick base-pairing rules. For example, the nucleotide sequence "T-G-A (5'→3')" is complementary to the nucleotide sequence "A-C-T (3'→5')". When fewer than all of the bases of a given nucleotide sequence match those of another nucleotide sequence according to base-pairing rules, the complementarity can be "partial". For example, in some aspects, complementarity between a given nucleotide sequence and another nucleotide sequence can be about 70%, about 75%, about 80%, about 85%, about 90%, or about 95%. Accordingly, in certain aspects, the term "complementary" refers to at least about 80%, at least about 85%, at least about 90%, at least about 91%, at least about 92%, at least about 93%, at least about 94%, at least about 95%, at least about 96%, at least about 97%, at least about 98%, or at least about 99% identity or complementarity with a target nucleic acid sequence. Alternatively, to continue the example, there may be "complete" or "perfect" (100%) complementarity between a given nucleotide sequence and another nucleotide sequence. In some aspects, the degree of complementarity between nucleotide sequences has a substantial effect on the efficiency and strength of hybridization between the sequences.

The term "downstream" refers to a nucleotide sequence that is located at the 3' side relative to a reference nucleotide sequence. In certain aspects, a downstream nucleotide sequence is related to a sequence located after the transcription start site. For example, the translation initiation codon of a gene is located downstream of the transcription start site.

As used herein, the term "enhancer" refers to a portion of DNA that contains a sequence capable of providing enhanced transcription and, in some cases, can act independently of its orientation relative to another regulatory sequence. An enhancer can act in cooperation with, or in addition to, a promoter and/or other enhancer elements.

The terms "excipient" and "carrier" are used interchangeably and refer to an inert substance that is added to a pharmaceutical composition to facilitate administration of a compound, for example a polynucleotide comprising a transgene and untranslated nucleic acid sequence as described herein.

The term "exon" refers to a specific portion of a nucleic acid that encodes a protein, or to a nucleic acid sequence that appears in the mature form of an RNA molecule after removal by splicing of a portion of a pre-processed or precursor RNA. The mature RNA molecule may be messenger RNA (mRNA) or a functional form of a non-coding RNA such as rRNA or tRNA.

The term "expression" as used herein refers to the process by which a polynucleotide produces a gene product, for example an RNA or a polypeptide. This includes, but is not limited to, transcription of the polynucleotide into messenger RNA (mRNA) and translation of the mRNA into a polypeptide. Expression produces a "gene product".

As used herein, the gene product may be a nucleic acid, for example an RNA produced by transcription of a gene. The gene product as used herein may be a nucleic acid or a polypeptide translated from a transcript. The gene products described herein further include nucleic acids that have undergone post-transcriptional modification, for example polyadenylation or splicing, or polypeptides that have undergone post-translational modification, for example phosphorylation, methylation, glycosylation, addition of a lipid, association with other protein subunits, or proteolytic cleavage of the protein.

The term "identity" as used herein refers to overall monomer conservation between polymer molecules, for example between polynucleotide molecules. The term "identical" when used without any additional modifier, for example "polynucleotide A is identical to polynucleotide B", means that the polynucleotide sequences are 100% identical (i.e., 100 sequence identity). Describing two sequences as "70% identical", for example, is the same as describing them as having "70% sequence identity".

For example, the identity (percent) between two polypeptide or polynucleotide sequences can be calculated by aligning the two sequences for optimal comparison. For optimal alignment, a gap can be introduced into one or both of the first and second polypeptide or polynucleotide sequences, and non-matching sequences can be ignored for comparison purposes. In certain aspects, the length of the aligned sequences for comparison is at least 30%, at least 40%, at least 50%, at least 60%, at least 70%, at least 80%, at least 90%, at least 95%, or 100% of the length of the reference sequence. The amino acids at corresponding positions for polypeptides, or the bases at corresponding positions for polynucleotides, are then compared.

When the same amino acid or nucleotide occupies a particular position in the first sequence and the corresponding position in the second sequence, the molecules are identical at that position. The percent identity between two sequences is a function of the number of positions shared by identical residues between the two sequences, taking into account the number of gaps that must be introduced to achieve optimal alignment of the two sequences and the length of each such gap. Sequence comparison and determination of percent identity between two sequences can be performed using mathematical algorithms.

Suitable software programs that can be used to align different sequences (for example, polynucleotide sequences) are available from various sources. One suitable program for determining sequence identity (percent) is bl2seq, which is part of the BLAST suite of programs available from the BLAST website of the National Center for Biotechnology Information NCBI of the U.S. government (blast.ncbi.nlm.nih.gov). bl2seq uses the BLASTN or BLASTP algorithms to perform pairwise sequence comparisons. BLASTN is used to compare nucleic acid sequences, whereas BLASTP is used to compare amino acid sequences. Other suitable programs include, for example, Needle, Stretcher, Water, or Matcher, which are part of the EMBOSS suite of bioinformatics programs and are also available from the European Bioinformatics Institute (EBI) at www.ebi.ac.uk/Tools/psa.

Sequence alignment can be performed using methods known in the art, such as MAFFT, Clustal (for example, ClustalW, Clustal X, or Clustal Omega), and MUSCLE.

Different regions within a single polynucleotide or polypeptide target sequence that is aligned with a reference polynucleotide or polypeptide sequence can each have their own percent sequence identity. It is noted that percent sequence identity values are rounded to the nearest first decimal place. For example, 80.11, 80.12, 80.13, and 80.14 are rounded down to 80.1, and 80.15, 80.16, 80.17, 80.18, and 80.19 are rounded up to 80.2. It is also noted that length values are always integers.

In certain aspects, the percent identity (%ID) of a first amino acid sequence (or nucleic acid sequence) to a second amino acid sequence (or nucleic acid sequence) is calculated as %ID = 100 × (Y/Z), where Y is the number of amino acid residues (or nucleotides) scored as identical matches in an alignment of the first and second sequences (as determined by visual inspection or using a particular sequence alignment program), and Z is the total number of residues in the second sequence. If the length of the first sequence is longer than that of the second sequence, the percent identity of the first sequence to the second sequence will be higher than the percent identity of the second sequence to the first sequence.

Those skilled in the art will understand that generation of a sequence alignment for calculating sequence identity (percent) is not limited to binary sequence-to-sequence comparisons in which only primary sequence data are considered. It will also be understood that sequence alignments can be generated by integrating sequence data with data from heterogeneous sources, such as structural data (for example, crystallographic protein structures), functional data (for example, locations of mutations), or phylogenetic data. A suitable program for generating multiple sequence alignments by integrating heterogeneous data is T-Coffee, which is available at www.tcoffee.org and also, for example, from EBI. It will further be understood that the final alignment used to calculate sequence identity (percent) can be curated either automatically or manually.

As used herein, the term "intron" refers to a portion intervening sequence of DNA within a gene that does not encode part of the protein produced by the gene and that is spliced from the mRNA transcribed from the gene before export from the nucleus. The term "intron sequence" refers to the nucleic acid sequence of an intron. Such sequences are also referred to herein as "untranslated nucleic acid sequences". Accordingly, introns are regions of DNA that are transcribed together with coding sequences exons but are removed during formation of mature mRNA.

As used herein, the term "intron fragment" refers to a fragment derived from a full-length intron sequence (for example, a full-length EF-1α intron A sequence). The fragment excludes the full-length intron. In some aspects, the "intron fragment" comprises the minimum number of nucleotides or configuration necessary to achieve an expression level that exceeds the expression level achieved by a corresponding construct in which all nucleotides of EF-1α intron A are absent. Accordingly, the intron fragments of the present disclosure also referred to herein as "untranslated nucleic acid sequences" are not particularly limited as long as they comprise a fragment of an EF-1α intron and can increase expression of a transgene. As demonstrated herein, in some aspects, an intron fragment (i.e., an untranslated nucleic acid sequence) can increase transcription of a transgene and thereby increase expression of the transgene. Thus, in some aspects, the intron fragments described herein may be untranslated regulatory elements.

As used herein, the terms "isolated", "purified", "extracted" and grammatical variants thereof are used interchangeably and refer to the state of a preparation of a desired composition of the present disclosure, for example a polypeptide comprising an amino acid sequence of a TAFA protein, a fragment thereof or a variant thereof, or a polynucleotide comprising a nucleic acid sequence encoding the same, that has undergone one or more purification steps. In some aspects, isolation or purification as used herein is a process of removing or partially removing (for example, fractionating) a composition of the present disclosure, such as a polypeptide or polynucleotide described herein, from a sample containing impurities.

In some aspects, the isolated composition has no detectable undesirable activity, or alternatively has a level or amount of undesirable activity that is at or below an acceptable level or amount. In other aspects, the isolated composition has an amount and/or concentration and/or activity of the desired composition of the present disclosure that is above an acceptable amount and/or concentration and/or activity. In other aspects, the isolated composition is enriched relative to the starting material from which the composition is obtained. Such enrichment can be at least about 10%, at least about 20%, at least about 30%, at least about 40%, at least about 50%, at least about 60%, at least about 70%, at least about 80%, at least about 90%, at least about 95%, at least about 96%, at least about 97%, at least about 98%, at least about 99%, at least about 99.9%, at least about 99.99%, at least about 99.999%, at least about 99.9999%, or greater than 99.9999%, relative to the starting material.

In some aspects, the isolated preparation contains substantially no residual biological products. In some aspects, the isolated preparation contains no biological contaminants in an amount of 100%, at least about 99%, at least about 98%, at least about 97%, at least about 96%, at least about 95%, at least about 95%, at least about 94%, at least about 93%, at least about 92%, at least about 91%, or at least about 90%. Residual biological products may include abiotic substances including chemicals or undesirable nucleic acids, proteins, lipids, or metabolites.

As used herein, the term "linked" refers to a first amino acid sequence or polynucleotide sequence that is joined, either covalently or non-covalently, to a second amino acid sequence or polynucleotide sequence. The first amino acid or polynucleotide sequence can be directly bonded to or juxtaposed with the second amino acid or polynucleotide sequence, or alternatively, an intervening sequence can covalently link the first sequence to the second sequence. For example, since a TAFA protein is a polypeptide including fragments 1, 2, and 3 joined together, the fragments of the TAFA protein may be in the form of fragment 2 linked to fragment 3, or fragment 5 linked to fragment 7. The term "linked" not only means fusion of a first polynucleotide sequence to the 5'- or 3'-end of a second polynucleotide sequence, but also includes insertion of the entire first polynucleotide sequence or the entire second polynucleotide sequence between any two nucleotides within the second polynucleotide sequence or the first polynucleotide sequence, respectively. The first polynucleotide sequence can be linked to the second polynucleotide sequence via a phosphodiester bond or via a linker. The linker can be, for example, a polynucleotide.

As used herein, the term "retinal neurodegenerative disease" refers to any disease, disorder, or condition that affects, or is associated with, a part or region of nerves in the retina or macula of the eye. The retinal neurodegenerative disease may be a disease, disorder, or condition caused by damage to all or part of the nerves of the retina or macula. The retinal neurodegenerative disease may be caused by dysfunction or damage of neural cells of the retina or macula.

NaIO₃ (sodium iodate) has been reported to induce damage and/or degeneration of the retina over part or all of its outer and inner layers and to induce damage to the retinal pigment epithelium (RPE) and photoreceptors (for example, the rod cell and cone cell layers) (A.E.-H. Koh, et al., Journal of Photochemistry & Photobiology, B: Biology, 196 (2019) 111514). Thus, by using a NaIO₃ model, it is possible to confirm preventive or therapeutic effects on diseases caused by damage to nerves in the retina or macula, or on damage to the retinal pigment epithelium (RPE) and photoreceptors (for example, the rod cell and cone cell layers). Examples of such diseases include retinopathy, choroidal neovascularization, maculopathy, macular degeneration, retinal degeneration, macular edema, retinal edema, macular swelling, retinal swelling, color vision deficiency, retinal cell degeneration, retinal vascular occlusion, retinal detachment, inherited retinal diseases, or any combination thereof.

As used herein, the term "retinopathy" refers to a disease or damage of the retina (for example, the tissue lining the inner surface of the back of the eye that captures images passing through the cornea and lens) or of retinal cells.

As used herein, the term "diabetic retinopathy" or "DR" refers to retinopathy caused by complications associated with diabetes. Depending on the severity of the disease, DR may be asymptomatic, may cause mild vision problems, or may lead to blindness. DR is a consequence of microvascular retinal changes. Hyperglycemia-induced intramural pericyte death and thickening of the basement membrane can lead to failure of the vascular wall. These injuries alter formation of the blood-retinal barrier and render retinal vessels more permeable. Pericyte death can be induced when hyperglycemia persistently activates PKC-δ encoded by protein kinase C-δ and p38 mitogen-activated protein kinase (MAPK) and increases expression of Src homology-2 domain-containing phosphatase-1 (SHP-1), a protein tyrosine phosphatase that is a previously unrecognized target of PKC-δ signaling. This signaling cascade leads to dephosphorylation of the PDGF receptor and reduced downstream signaling from this receptor, resulting in "pericyte apoptosis". Small vessels, such as the small vessels in the eye, may be particularly vulnerable to poor control of blood glucose. Excess accumulation of glucose and/or fructose can damage the small vessels in the retina.

DR can be classified into two distinct stages (Wu L., et al., World J Diabetes, 4 6 290-294 (2013)). The first stage, referred to as non-proliferative diabetic retinopathy (NPDR), is associated with early diabetic retinopathy. NPDR is generally asymptomatic or associated with mild vision distortion caused by vessels from which fluid leaks into the surrounding tissue. The only way to detect NPDR is by fundus photography, which can reveal microaneurysms (tiny bulges in the arterial wall filled with blood). If left untreated, patients with DR can progress to a more advanced, second stage called proliferative diabetic retinopathy (PDR). PDR is characterized by abnormal new blood vessel formation (i.e., neovascularization), which can rupture and bleed, causing blurred vision. Other symptoms of PDR include floating spots or dark lines in the visual field (floaters), vision changes, defects in color vision, dark or empty areas in the visual field, pain, uneven vision, and complete loss of vision.

The term "diabetic retinopathy" includes all types of diabetic retinopathy, including but not limited to non-proliferative diabetic retinopathy (NPDR), proliferative diabetic retinopathy (PDR), diabetic maculopathy, and diabetic macular edema.

In some aspects, PDR develops after the onset of NPDR (for example, a subject is first diagnosed with NPDR and the disease progresses to PDR). In other aspects, PDR develops independently of NPDR. As used herein, the term "diabetic retinopathy" also encompasses all types of diabetic retinopathy and any and all symptoms of diabetic retinopathy, regardless of cause. Non-limiting examples of risk factors for diabetic retinopathy include duration of diabetes, genetics, excessive alcohol consumption, smoking, hypertension, obesity, dyslipidemia, high cholesterol, kidney disease, pregnancy, and renal impairment.

As used herein, the term "maculopathy" refers to any pathological condition of the macula, the central region of the retina associated with very sensitive and accurate vision. In some aspects, the terms "maculopathy" and "retinopathy" can be used interchangeably (for example, when only the macula is affected). In some aspects, the maculopathy is diabetic maculopathy.

"Diabetic maculopathy" occurs when the macula is affected by retinal changes caused by diabetes. The term includes two distinct ocular conditions, diabetic macular edema and diabetic ischemic maculopathy. The two types of maculopathy are often comorbid, that is, a person with macular edema often also has ischemic maculopathy. Ischemic maculopathy occurs together with macular edema and may occur even when macular edema is mild. In some aspects, retinal changes associated with diabetic maculopathy include, within the retina of a subject, a decrease in retinal potentials, loss of pericytes, formation of acellular capillaries, vascular congestion, vascular dysfunction, vascular leakage, vascular occlusion, tissue swelling (edema), tissue ischemia, or any combination thereof.

As used herein, the term "acellular capillary" refers to a vessel tube of capillary size that has no nucleus anywhere along its length.

As used herein, the term "vascular congestion" refers to a type of vascular damage that is an important factor in the pathogenesis of various ocular diseases disclosed herein (for example, diabetic macular edema). Vascular congestion is associated with accumulation of fluid (for example, blood within the vasculature). In some aspects, vascular congestion can result from hyperglycemia (i.e., high blood glucose).

As used herein, the term "macular degeneration" refers to any of a number of disorders and conditions in which the central region of the retina (i.e., the macula) is degenerated or has lost functional activity. The degeneration or loss of functional activity can occur, for example, as a result of cell death, reduced cell proliferation, loss of normal biological function, or any combination thereof. Macular degeneration can lead to, and/or manifest as, changes in the structural integrity of cells and/or extracellular matrix of the macula, changes in the composition of normal cells and/or extracellular matrix, and/or loss of function of cells of the macula. The cells can be any cell type normally present in or near the macula, including RPE cells, photoreceptors (for example, the rod cell and cone cell layers), and/or capillary endothelial cells. Age-related macular degeneration is the most common form of macular degeneration, but the term "macular degeneration" does not necessarily exclude macular degeneration in non-elderly patients. Non-limiting examples of macular degeneration include: age-related macular degeneration (wet or dry), Best macular dystrophy, Sorsby fundus dystrophy, Malattia Leventinese, Doyne honeycomb retinal dystrophy, Stargardt disease (also referred to as Stargardt macular dystrophy, juvenile macular degeneration, or fundus flavimaculatus), and pigment epithelial detachment-related macular degeneration.

As used herein, the term "age-related macular degeneration" AMD generally refers to a retinopathy that affects elderly individuals and is associated with loss of central vision due to damage to the central region of the retina (i.e., the macula). AMD is generally characterized by gradual accumulation or aggregation of yellow-ish insoluble extracellular deposits known as drusen (accumulation of extracellular proteins such as amyloid-beta and lipids) within the macula (primarily between the retinal pigment epithelium (RPE) and the underlying choroid). Accumulation or aggregation of these deposits within the macula can progressively damage the macula and lead to loss of central vision. As used herein, the term "macula" refers to the central region of the retina responsible for central high-resolution color vision.

Although several theories, including oxidative stress, mitochondrial dysfunction, and inflammatory processes, have been proposed, the pathogenesis of age-related macular degeneration is not well understood. An imbalance between production and degradation of damaged cellular components leads, for example, to accumulation of harmful products such as intracellular lipofuscin and extracellular drusen. Early atrophy is characterized in early AMD by areas of thinning or depigmentation of the retinal pigment epithelium (RPE) that precede geographic atrophy. In advanced stages of AMD, atrophy of the RPE (i.e., geographic atrophy) and/or development of new vessels (i.e., neovascularization) lead to death of photoreceptors and loss of central vision. In dry (non-exudative) AMD, cellular debris called drusen accumulates between the retina and the choroid, leading to atrophy and scarring of the retina. In the more severe wet (exudative) AMD, blood vessels grow from the choroid behind the retina (i.e., neovascularization) and leak exudate and fluid, which can also cause hemorrhage.

Depending on the extent of drusen present, AMD can be classified into three major stages: (i) early, (ii) intermediate, and (iii) advanced or late. Early AMD is characterized by the presence of multiple small (for example, less than about 63 microns in diameter) drusen or a few medium-sized (for example, about 63 to 124 microns in diameter) drusen. During the early stage, patients typically have no obvious symptoms and no vision loss. The intermediate stage is characterized by the presence of many medium-sized drusen or one or more large (for example, greater than about 125 microns in diameter) drusen. During this stage, some patients may begin to experience blurred spots in the center of their visual field. Advanced or late AMD is characterized by damage to a large area of retinal tissue, resulting in a central scotoma and eventually loss of central vision. Based on the type of damage (for example, the presence or absence of neovascularization), advanced or late AMD can be further divided into two subtypes: (i) geographic atrophy (also referred to as atrophic AMD) and (ii) wet AMD (also referred to as neovascular or exudative AMD).

AMD has two main forms: (i) dry AMD and (ii) wet AMD. Unless otherwise specified, the term "age-related macular degeneration" includes both dry AMD and wet AMD. As used herein, the term "age-related macular degeneration" also encompasses all types of age-related macular degeneration and any and all symptoms of age-related macular degeneration, regardless of cause. Non-limiting examples of symptoms associated with macular degeneration (for example, age-related macular degeneration) include: loss of central vision, distortion, decreased contrast sensitivity, blurred vision, difficulty adapting to low light, sudden onset and rapid worsening of symptoms, and decreased color vision. In some aspects, macular degeneration (for example, age-related macular degeneration) can cause macular edema (for example, swelling of the macula due to accumulation of fluid and protein deposits on or under the macula).

As used herein, the term "dry AMD" (also referred to as atrophic age-related macular degeneration or non-exudative AMD) refers to all forms of AMD that are not wet (neovascular) AMD. This includes early and intermediate forms of AMD as well as the advanced form of dry AMD known as geographic atrophy. Patients with dry AMD tend to have minimal symptoms at earlier stages, and loss of visual function occurs more frequently when the condition progresses to geographic atrophy.

As used herein, the term "wet AMD" (also referred to as neovascular age-related macular degeneration or exudative AMD) refers to a retinal condition characterized by the presence of retinal neovascularization and represents the most advanced form of AMD. In wet AMD, blood vessels grow from the choroidal capillaries and, in some cases, from the underlying retinal pigment epithelium through defects in Bruch's membrane (choroidal neovascularization or neovascularization). Organization of serous or hemorrhagic exudates leaking from these vessels can lead to secondary degeneration of the neurosensory retina, detachment and rupture of the retinal pigment epithelium, vitreous hemorrhage, and formation of a fibrovascular scar in the macular region, together with permanent loss of central vision.

As used herein, the term "neovascularization" refers to growth of new, abnormal blood vessels in different parts of the eye that can cause bleeding and result in loss of vision. As used herein, the term "choroidal neovascularization" refers to abnormal growth of new blood vessels in the choroid (i.e., the vascular layer of the eye containing connective tissue and located between the retina and the sclera). In wet AMD, new vessels can grow through the retinal pigment epithelium (RPE) and the choroid into the retina and impair visual function due to leakage of blood and lipids. As used herein, the term "retinal neovascularization" refers to abnormal development, proliferation, and/or growth of blood vessels on or within the retina, for example on the retinal surface. Retinal neovascularization can occur in many retinopathies associated with retinal ischemia (for example, diabetic retinopathy, sickle cell retinopathy, Eales disease, ocular ischemic syndrome, carotid-cavernous fistula, familial exudative vitreoretinopathy, hyperviscosity syndrome, radiation retinopathy, retinal vein occlusion, retinal artery occlusion, retinal embolism, birdshot retinochoroidopathy, choroidal melanoma, chronic retinal detachment, anterior ischemic optic neuropathy (AION), non-arteritic anterior ischemic optic neuropathy (NAION), and incontinentia pigmenti). Methods for detecting neovascularization are known in the art and include, but are not limited to, measuring expression of CD31 (platelet endothelial cell adhesion molecule, also known as PECAM-1) and vascular endothelial growth factor (VEGF) in tissues; see, for example, Schluter A. et al., BMC Cancer, 18(1):272 (2018).

As used herein, the term "dyschromatopsia" or "color vision deficiency" refers to a condition in which color discrimination is impaired due to dysfunction of cone cells, which are one type of photoreceptor in the retina. Cone cells are a type of photoreceptor, and functional defects or loss of cone cells lead to color vision deficiency (Bennett J., Gene therapy for color blindness, N. Engl. J. Med., 2009, 361(25):2483-2484). In patients with AMD, it has also been reported that decreased function or loss of cone cells is accompanied in many cases by color vision deficiency (O'Neill-Biba M. et al., Loss of chromatic sensitivity in AMD and diabetes: a comparative study, Ophthalmic Physiol. Opt., 2010, 30(5):705-716). A model for assessing such color vision deficiency can employ NaIO₃ (sodium iodate), which induces oxidative stress in photoreceptors and causes apoptosis of cone cells, thereby inducing color vision deficiency (Wang J. et al., Direct effect of sodium iodate on neurosensory retina, Invest. Ophthalmol. Vis. Sci., 2014, 55(3):1941-1953; Takeda A. et al., New Insights Into Immunological Therapy for Retinal Disorders, Front. Immunol., 2020, 11:1431). In one embodiment, a polypeptide comprising an amino acid sequence of TAFA, a fragment thereof, or a variant thereof as described herein protects cone cells and photoreceptors containing the same from oxidative stress caused by NaIO₃, and this mechanism indicates that the polypeptide and a polynucleotide encoding the same can function as an agent for preventing or treating the above-described color vision deficiency.

The color vision deficiency can be classified into color blindness and color weakness. Color blindness is a condition in which one of the three primary colors cannot be perceived at all, whereas color weakness is a condition in which the color can be perceived but is seen differently due to a defect in the corresponding receptor. Color vision deficiency is common and occurs in about 5-8% of the male population. Among color vision deficiencies, green weakness (deuteranomaly) is generally the most frequent and accounts for about 25-45% of all cases. This is followed, in approximate order, by green blindness (deuteranopia), red blindness (protanopia), and red weakness (protanomaly), each occurring in about 1% of the male population. Tritan color defects and complete achromatopsia are very rare, with a prevalence of about 0.005%.

As used herein, the term "inherited retinal disease" refers to a retinal disease in which structural and functional abnormalities of retinal cells occur due to defects or abnormalities in genes. The time of onset and symptoms differ depending on the causative gene of the inherited retinal disease. Non-limiting examples of inherited retinal diseases include retinitis pigmentosa (RP), Leber congenital amaurosis, Stargardt's disease, Coats retinopathy, cone dystrophy, choroideremia, Usher syndrome, Best's disease, X-linked retinoschisis, hereditary color vision deficiency, and unspecified hereditary retinal dystrophy resulting from unidentified genetic abnormalities.

As used herein, the term "retinitis pigmentosa" (RP) refers to a retinal disease in which photoreceptor cells and retinal pigment epithelial cells are damaged (or degenerated). As photoreceptor cells are damaged, night blindness initially appears, visual fields gradually constrict, and eventually blindness occurs. The main cause is defects in genes involved in the mechanism by which light is converted into electrical signals within photoreceptor cells, and in some cases gene abnormalities have also been found in the retinal pigment epithelium, and these genetic abnormalities cause widespread retinal damage.

In retinitis pigmentosa, the earliest clinically apparent feature is the gradual destruction of rod cells and cone cells, with rod cells being affected earlier than cone cells. When destruction of photoreceptor cells progresses to cone cells, central vision is also lost and complete blindness results.

As used herein, the term "Leber congenital amaurosis" refers to an inherited retinal disease that can cause congenital blindness at birth or shortly after birth. In patients with Leber congenital amaurosis, rod and cone cells of the retina with normal function are absent, so both cone and rod responses are abolished on electroretinography. Leber congenital amaurosis is a disease caused by genetic abnormalities, and mutations in causative genes are found in about 40-50% of patients. Of the twelve known causative genes, eleven (GUCY2D, RPE65, SPATA7, AIPL1, LCA5, RPGRIP1, CRB1, CEP290, IMPDH1, RD3, and RDH12) carry mutations inherited in an autosomal recessive manner, and, more rarely, mutations inherited in an autosomal dominant manner (CRX) have also been reported.

As used herein, the term "Stargardt's disease" refers to a form of retinal dystrophy that is inherited in an autosomal recessive manner. Stargardt's disease usually appears between 8 and 15 years of age, and gradual loss of central visual acuity occurs due to bilateral macular degeneration. It is currently thought that mutations in a gene known as ABCA4 are responsible for Stargardt's disease. Mutations in ABCA4 cause accumulation of lipofuscin-like material in the retinal pigment epithelium, leading to RPE cell death and loss of photoreceptors (for example, rod and cone layers). Mutations in ABCA4 are associated with cone and rod dystrophies and with severe forms of retinal dystrophy.

As used herein, the term "Coats retinopathy" refers to a retinal vascular disease in which telangiectasia and aneurysmal dilatation of retinal capillaries lead to accumulation of exudates within and beneath the retina, causing exudative retinal detachment. It is known to result from somatic mutations of the NDP gene on the X chromosome, which cause deficiency of norrin protein required for retinal development.

As used herein, the term "cone dystrophy" refers to a disease in which cone cells, among the retinal cells responsible for color vision and central vision, undergo degeneration due to genetic abnormalities, resulting in loss of central vision. Cone dystrophy is broadly classified into pure cone dystrophy and cone-rod dystrophy. Modes of inheritance are diverse, including autosomal dominant, autosomal recessive, and X-linked patterns, and the disease can also arise in the absence of an obvious familial pattern due to de novo mutations.

As used herein, the term "choroideremia" refers to a rare X-linked progressive degeneration of the choroid, retinal pigment epithelium, and photoreceptors. In affected patients, mutations in the CHM gene lead to deficiency of Rab escort protein 1 (REP1), causing photoreceptors in the retina to lose function and gradually die. Symptoms usually appear in males and typically begin with night blindness in childhood, followed by progressive loss of peripheral vision, whereas central vision is relatively preserved in the early stages. In many affected men in their 40s, visual acuity remains good but the visual field is markedly constricted, and between about 50 and 70 years of age visual acuity is also lost. Some patients also show impaired color perception.

As used herein, the term "Usher syndrome" refers to an inherited disorder in which visual impairment progresses in association with hearing loss. The hearing loss in Usher syndrome is due to inner ear abnormalities, whereas the visual impairment is associated with retinitis pigmentosa (RP).

As used herein, the term "Best's disease" refers to an inherited retinal disease caused by mutations in the BEST1 (VMD2) gene. The disease progresses slowly and can lead to decreased central visual acuity. Mutations in the BEST1 (VMD2) gene impair the function of bestrophin, a calcium-activated chloride channel protein in the basolateral membrane of the retinal pigment epithelium, thereby disrupting fluid transport across the retinal pigment epithelium and resulting in serous retinal detachment and/or retinal pigment epithelial detachment.

As used herein, the term "X-linked retinoschisis" refers to a disorder in which splitting of the inner retina occurs due to mutations in the retinoschisis gene (RS1). Retinoschisis is characterized by abnormal separation within the retinal layers, among the ten layers that constitute the retina, particularly within the nerve fiber layer, and causes visual impairment. Juvenile X-linked retinoschisis is a rare X-linked recessive disease with a worldwide prevalence of about 1 in 120,000.

As used herein, the term "neuropathic pain" refers to pain resulting from injury, damage, and/or dysfunction affecting any level of the central nervous system (CNS) and/or the peripheral nervous system. As used herein, the term "neuropathic pain" encompasses any and all types of neuropathic pain, irrespective of the cause of the neuropathic pain and the full range of its symptoms.

Neuropathic pain includes central neuropathic pain and peripheral neuropathic pain. As used herein, the term "central neuropathic pain" refers to pain resulting from disorders, congenital defects, or injury of the central nervous system, that is, the brain or spinal cord. As used herein, the term "peripheral neuropathic pain" refers to pain resulting from damage to or infection of peripheral sensory nerves.

Symptoms of neuropathic pain can include persistent or chronic pain, spontaneous pain and allodynia (for example, a painful response to a normally non-painful stimulus), hyperalgesia (for example, a markedly painful response to a stimulus that is normally only mildly unpleasant, such as a pinprick), hyperesthesia (for example, excessive somatic sensitivity to stimuli, particularly cutaneous stimuli), or hyperpathia (for example, when a brief unpleasant sensation becomes a prolonged, severe pain). In some embodiments, symptoms may be long-lasting and may persist even after resolution of a primary underlying cause, if present. See, for example, Merck Manual, "Neuropathic Pain," merckmanuals.com/professional/neurologic-disorders/pain/neuropathic-pain; and Campbell J. N. and Meyer R. A., Neuron 52(1):77-92 (2006).

In some embodiments, types of neuropathic pain include (1) neuralgia, (2) deafferentation pain syndromes, (3) complex regional pain syndrome (CRPS), and (4) neuropathies (central or peripheral).

In some embodiments, neuropathic pain is neuralgia, which generally refers to pain radiating along the course of one or more specific nerves (for example, cranial nerves) in the absence of obvious pathological changes in the nerve structure. Neuralgia includes, but is not limited to, trigeminal neuralgia (TN), atypical trigeminal neuralgia (ATN), occipital neuralgia, glossopharyngeal neuralgia, postherpetic neuralgia (resulting from herpes zoster or herpes virus infection), pain due to peripheral nerve injury, sciatica, low back pain, and atypical facial pain. Chemical irritation, chronic kidney disease, diabetes, inflammation, trauma (including surgery), nerve compression by adjacent structures (for example, tumors), certain pharmaceuticals (for example, cisplatin, paclitaxel, or vincristine), porphyria (a blood disorder), and infections (for example, herpes zoster (shingles), HIV/AIDS, Lyme disease, or syphilis) can all lead to neuralgia.

In some embodiments, neuropathic pain is deafferentation pain syndrome, which can result from loss of sensory input from a part of the body (for example, caused by interruption of peripheral sensory fibers or nerves within the central nervous system). Deafferentation pain syndromes include, but are not limited to, brain or spinal cord injury, post-stroke pain, phantom limb pain, paraplegia, brachial plexus avulsion injury, and lumbar radiculopathies.

In some embodiments, neuropathic pain is complex regional pain syndrome (CRPS), which is a chronic pain condition that most commonly affects an arm or a leg. In some embodiments, CRPS occurs after trauma, surgery, stroke, or myocardial infarction. In certain embodiments, CRPS is CRPS type I (CRPS-I) (also referred to as reflex sympathetic dystrophy syndrome), in which affected individuals often have no confirmed nerve injury. In other embodiments, CRPS is CRPS type II (CRPS-II) (also referred to as causalgia), which is associated with confirmed nerve injury.

In some embodiments, neuropathic pain is a neuropathy, which refers to pain resulting from functional or pathological changes in nerves (for example, due to disease or injury). Neuropathy is often clinically characterized by abnormalities of sensory and/or motor neurons. In certain embodiments, the neuropathy is a central neuropathy (for example, functional or pathological changes in the central nervous system). In other embodiments, the neuropathy is a peripheral neuropathy (for example, functional or pathological changes affecting one or more peripheral nerves, including motor, sensory, autonomic nerves, or combinations thereof). In some embodiments, peripheral neuropathy involves functional or pathological changes affecting a single nerve or group of nerves (that is, mononeuropathy). In some embodiments, peripheral neuropathy involves functional or pathological changes affecting multiple nerves (locally or systemically) (i.e., polyneuropathy). In some embodiments, peripheral neuropathy affects both sides of the body in a nearly symmetric manner (i.e., symmetric polyneuropathy). In some embodiments, peripheral neuropathy affects anatomically heterogeneous regions of the body (for example, mononeuritis multiplex or multifocal mononeuropathy, mononeuropathy multiplex).

As used herein, the term "mononeuropathy" refers to a peripheral neuropathy that is characterized by loss of motor function and/or sensation in a region innervated by a single peripheral nerve or nerve group as a result of damage to or destruction of such nerve or nerve group. Mononeuropathy typically results from localized injury or trauma that causes sustained compression or pressure on a single nerve. Certain systemic disorders (for example, mononeuritis multiplex) may also give rise to mononeuropathy. In some embodiments, localized injury or trauma results in destruction of a myelin sheath covering all or part of a nerve or neuronal axon, thereby slowing or blocking impulse conduction along the nerve. Mononeuropathy may affect essentially any part of the body. Non-limiting examples of mononeuropathic pain include sciatic nerve dysfunction, generalized pelvic nerve dysfunction, radial nerve dysfunction, ulnar nerve dysfunction, cranial mononeuropathy VI, cranial mononeuropathy VII, cranial mononeuropathy III (compressive type), cranial neuropathy III (diabetic type), axillary nerve dysfunction, carpal tunnel syndrome, femoral nerve dysfunction, tibial nerve dysfunction, Bell's palsy, thoracic outlet syndrome, and sixth (abducens) nerve palsy (see, for example, Finnerup N. B. et al., Pain 157(8):1599-1606 (2016); National Institute of Neurological Disorders and Stroke, "Peripheral Neuropathy Fact Sheet," ninds.nih.gov/disorders/peripheralneuropathy/detailj3eripheralneuropathy.htm.). In some embodiments, the mononeuropathic pain is sciatica.

As used herein, the term "polyneuropathy" refers to a peripheral neuropathy that is characterized by loss of motor function and/or sensation in a region innervated by multiple peripheral nerves as a result of damage to or destruction of such nerves. Polyneuropathic pain includes, but is not limited to, pain associated with post-polio syndrome, post-mastectomy pain syndrome, diabetic neuropathy, alcoholic neuropathy, amyloidosis, toxin-induced neuropathy, AIDS-related neuropathy, hypothyroid neuropathy, uremic neuropathy, vitamin deficiency-related neuropathy, chemotherapy-induced pain, neuropathy associated with 2',3'-dideoxycytidine (ddC) therapy, Guillain-Barré syndrome, and Fabry disease (see, for example, Finnerup N. B. et al., Pain 157(8):1599-1606 (2016); National Institute of Neurological Disorders and Stroke, "Peripheral Neuropathy Fact Sheet," ninds.nih.gov/disorders/peripheralneuropathy/detail 3eripheralneuropathy.htm.). In some embodiments, the polyneuropathy is diabetic peripheral neuropathy, which may be caused by elevated blood glucose levels (hyperglycemia) and/or elevated blood lipid levels (for example, triglycerides) in a subject with diabetes, thereby inducing damage to peripheral nerves of the subject.

In some embodiments, the peripheral neuropathy described herein is classified according to the portion of the neuron that is damaged or affected (for example, axon, myelin sheath, or cell body). In certain embodiments, the peripheral neuropathy is a distal axonopathy, which results from metabolic and/or toxic disturbance of the axon. Metabolic disorders may include deficiency syndromes such as diabetes, renal failure, malnutrition, and alcoholism. In some embodiments, the metabolic disorder is diabetes, and the distal axonopathy is a diabetic neuropathy.

In other embodiments, the peripheral neuropathy is a myelinopathy, which arises from a primary insult to the myelin sheath and/or myelinating Schwann cells and results in acute failure of impulse conduction. The most common cause of myelinopathy is acute inflammatory demyelinating polyneuropathy (AIDP; also referred to as Guillain-Barré syndrome), but other causes include chronic inflammatory demyelinating polyneuropathy (CIDP), inherited metabolic disorders (for example, leukodystrophies), and toxins.

In further embodiments, the peripheral neuropathy is a neuronopathy, which results from destruction of neurons in the peripheral nervous system (PNS). Neuronopathy may result from, for example, motor neuron disease, sensory neuronopathy (for example, due to herpes zoster), toxins, or autonomic dysfunction, and may also result from exposure to neurotoxic chemotherapeutic agents such as vincristine.

Neuropathic pain may be caused by, or associated with, a variety of etiologies including, but not limited to, physical injury (for example, trauma or repetitive stress), diseases or disorders, exposure to toxic agents, or any combination thereof. In some embodiments, neuropathic pain is caused by, or associated with, traumatic injury or damage such as nerve compression injury (for example, nerve crush, nerve stretch, nerve entrapment, or incomplete nerve transection), spinal cord injury (for example, hemisection of the spinal cord), injury or damage to peripheral nerves (for example, motor, sensory, and/or autonomic nerves), limb amputation, contusion, inflammation (for example, inflammation of the spinal cord), or surgical procedures. In some embodiments, neuropathic pain arises from, or is associated with, repetitive stress involving repetitive, slow, and/or forceful activities requiring prolonged movement of any joint group. Without being bound by theory, such repetitive stress may cause ligaments, tendons, and muscles to become inflamed and swollen, thereby narrowing anatomical passages through which nerves traverse (for example, ulnar neuropathy and carpal tunnel syndrome, in which nerves at the elbow or wrist are entrapped or compressed). In other embodiments, neuropathic pain arises from, or is associated with, a disease or disorder including, but not limited to, ischemic events (for example, stroke or myocardial infarction), multiple sclerosis, metabolic and/or endocrine diseases or disorders (for example, diabetes, other metabolic diseases, and acromegaly, which is caused by excessive production of growth hormone and characterized by abnormal enlargement of skeletal elements including joints, resulting in nerve compression and pain), small vessel disease that reduces oxygen supply to peripheral nerves and causes nerve tissue damage (for example, vasculitis, that is, inflammation of blood vessels), autoimmune diseases (for example, Sjögren's syndrome, lupus, rheumatoid arthritis, and acute inflammatory demyelinating polyneuropathy also referred to as Guillain-Barré syndrome), kidney disease, cancers or tumors (for example, neoplastic tumors, neuromas, paraneoplastic syndromes, and toxicity caused by chemotherapeutic agents and/or radiation used in cancer treatment), infections (for example, herpes zoster (shingles), Epstein-Barr virus, West Nile virus, cytomegalovirus, herpes simplex virus, acquired immunodeficiency syndrome (AIDS), Lyme disease, diphtheria, and leprosy), inflammatory disorders, peripheral nerve disorders (for example, neuromas), and hereditary or de novo genetic disorders (for example, Charcot-Marie-Tooth disease, which is characterized by muscle weakness and wasting in the legs and feet, gait abnormalities, loss of tendon reflexes, and paralysis of the lower extremities), as well as mononeuropathy or polyneuropathy. In some embodiments, neuropathic pain arises from, or is associated with, infection by a pathogenic agent, including, for example, tick-borne infections, varicella-zoster virus, Epstein-Barr virus, West Nile virus, cytomegalovirus, herpes simplex virus, or AIDS. In other embodiments, neuropathic pain arises from, or is associated with, exposure to toxic agents such as drugs, alcohol, heavy metals (for example, lead, arsenic, or mercury), industrial agents (for example, fumes generated from solvents or adhesives), or nitrous oxide.

As used herein, the expression "neuropathic pain associated with a disease or disorder" refers to neuropathic pain that accompanies a disease or disorder (for example, any of those described herein), is caused by such a disease or disorder, or results therefrom.

As used herein, the terms "nucleic acid," "nucleic acid molecule," "nucleotide sequence," "polynucleotide," and grammatical variants thereof are used interchangeably and refer to a sequence of nucleotides linked via phosphodiester bonds. Polynucleotides are denoted herein in the 5' to 3' direction. The polynucleotides of the present disclosure can be deoxyribonucleic acid (DNA) molecules or ribonucleic acid (RNA) molecules. Nucleotide bases are represented herein by single-letter codes such as A (adenine), G (guanine), T (thymine), C (cytosine), I (inosine), and U (uracil).

As used herein, the terms "operably linked" and "operably associated" refer to a disposition of DNA sequences such that the sequences are in a functional relationship and cooperate to perform a desired function. For example, a given promoter can be operably linked to a coding sequence (for example, a transgene) when the promoter is positioned so as to drive transcription of the coding sequence. The promoter and coding sequence need not be immediately adjacent, provided that the functional relationship is maintained.

As used herein, the terms "pharmaceutically acceptable carrier" and "pharmaceutically acceptable excipient," and grammatical variants thereof, refer to any carrier or diluent that is approved by a regulatory agency of the United States Federal government or listed in the United States Pharmacopeia for use in animals, including humans, or that is otherwise pharmaceutically acceptable in a subject, and that does not abrogate the biological activity or properties of the administered compound to such an extent that administration of the composition is rendered unsuitable or causes an undesirable physiological effect that precludes administration to the subject. Pharmaceutically acceptable carriers and excipients useful for the manufacture of pharmaceutical compositions are generally safe, non-toxic, and suitable for administration in the context described herein.

As used herein, the term "pharmaceutical composition" refers to a composition comprising one or more described herein (for example, a polypeptide, a polynucleotide, a vector, a cell, and/or a recombinant virus) in admixture, suspension, or combination with one or more additional chemical components, such as a pharmaceutically acceptable carrier or excipient.

As used herein, the terms "promoter" and "promoter sequence" are used interchangeably and refer to a DNA sequence capable of regulating expression of a coding sequence or a functional RNA. In general, a coding sequence is located 3' to the promoter sequence. A promoter can be derived entirely from a natural gene, can comprise different elements derived from different promoters occurring in nature, and/or can further include synthetic DNA segments. Those skilled in the art understand that different promoters can direct gene expression in different tissues or cell types, at different developmental stages, or in response to different environmental or physiological conditions. A promoter that directs expression of a gene in general or most host cell types is commonly referred to as a "constitutive promoter." A promoter that directs expression of a gene in particular cell types is commonly referred to as a "cell-specific promoter" or "tissue-specific promoter." A promoter that directs expression of a gene at particular developmental stages or stages of cell differentiation is commonly referred to as a "development-specific promoter" or a "differentiation-specific promoter." A promoter that is activated and induces gene expression upon exposure of cells to an inducing agent, biological molecule, chemical, ligand, light, or the like is commonly referred to as an "inducible promoter" or a "regulated promoter." In many cases, because the precise boundaries of regulatory sequences are not fully defined, DNA fragments of different lengths can exhibit substantially the same promoter activity.

The promoter sequence typically borders a transcription start site at its 3' end and extends upstream (in the 5' direction) sufficiently to include the minimal number of nucleotides or elements required to initiate transcription at a detectable level above background. Within the promoter sequence, a protein-binding domain (consensus sequence) responsible for binding RNA polymerase, as well as a transcription start site (for example, conveniently defined by S1 nuclease mapping), may be found. In some embodiments, promoters suitable for use in the present disclosure include tissue-specific promoters.

As used herein, the terms "gene regulatory region" and "regulatory region" refer to a nucleotide sequence located upstream (5' untranslated sequence), within, or downstream (3' untranslated sequence) of a coding region, which affects transcription, RNA processing, RNA stability, or translation of the associated coding region. A regulatory region may include, for example, a promoter, a translation leader sequence, an intron, a polyadenylation recognition sequence, an RNA processing site, an effector binding site, or a stem-loop structure. When a coding region is intended to be expressed in a eukaryotic cell, a polyadenylation signal and transcription termination sequence are typically located 3' to the coding sequence.

In some aspects, a polynucleotide described herein (for example, a polynucleotide comprising a transgene encoding a polypeptide comprising an amino acid sequence of a TAFA protein, a fragment thereof, or a variant thereof, together with an untranslated nucleic acid sequence) further comprises a promoter and/or one or more additional expression (for example, transcription) regulatory elements operably associated with one or more coding regions. In an operable association, a coding region for a gene product is linked to one or more regulatory regions in a manner that places expression of the gene product under the influence or control of the regulatory region(s). For example, a coding region and a promoter are operably associated where activation of promoter function results in transcription of an mRNA encoding the gene product specified by the coding region, and where the linkage between the promoter and coding region does not interfere with the ability of the promoter to direct expression of the gene product or with the ability of the DNA template to be transcribed. In addition to promoters, other expression-regulatory elements such as enhancers, operators, repressors, and transcription termination signals may also be operably associated with a coding region that directs expression of a gene product.

As used herein, the terms "subject," "patient," "individual," and "host," and grammatical variants thereof, are used interchangeably and refer to any mammalian subject to whom any of the compositions described herein (for example, a polypeptide, a polynucleotide, a recombinant expression construct, a vector, a cell, a pharmaceutical composition, or a recombinant virus) is administered. Non-limiting examples include humans, companion animals (for example, dogs and cats), farm animals (for example, cattle, sheep, pigs, and horses), and laboratory animals (for example, non-human primates, rats, mice, rabbits, and guinea pigs). In preferred embodiments, the subject is a human. The methods described herein are applicable to both human therapeutic or prophylactic use and veterinary use.

As used herein, the phrase "subject in need thereof" includes a subject, such as a mammalian subject, for whom administration of a composition described herein would be beneficial.

As used herein, the term "therapeutically effective amount" refers to an amount of a reagent or pharmaceutical composition comprising a composition of the present disclosure (for example, a polypeptide comprising a fragment of a TAFA protein or a variant thereof, or a polynucleotide encoding the same) that is sufficient to achieve a desired therapeutic, pharmacological, and/or physiological effect in a subject in need thereof. Because prevention can be regarded as a form of treatment, a therapeutically effective amount can also be a "prophylactically effective amount."

As used herein, the term "transgene" refers to a polynucleotide encoded by a recombinant expression construct, including at least one polynucleotide (for example, a polynucleotide encoding a TAFA protein, or a polynucleotide encoding a polypeptide comprising a fragment of a TAFA protein or a variant thereof), a polynucleotide region, an expression product of such a polynucleotide or region, a polynucleotide encoding a polypeptide or multi-polypeptide product, or a facilitating or regulatory nucleic acid. In some aspects, the transgene is heterologous to the cell into which it is introduced (or transduced) (for example, it is not naturally expressed in the cell).

As used herein, the terms "treat," "treating," and "treatment" refer to any intervention that, for example, reduces the severity of a disease or condition, shortens the duration of a disease course, ameliorates or eliminates one or more symptoms associated with a disease or condition, or provides a beneficial effect to a subject having the disease or condition, even without necessarily curing the disease or condition. The terms also encompass preventing or inhibiting the onset of a disease or condition or of a symptom thereof.

As used herein, the terms "vector" and "construct" refer to any vehicle into which a nucleic acid or gene can be inserted and which is capable of delivering the nucleic acid sequence into a cell, wherein the nucleic acid sequence can be replicated and/or expressed. The nucleic acid sequence inserted into the vector may be exogenous or heterologous, and may be a transgene. Examples of constructs include, without limitation, plasmids, cosmids, and viruses (for example, AAV). Those skilled in the art can construct such vectors or constructs using standard recombinant techniques (see, for example, Maniatis et al., Molecular Cloning: A Laboratory Manual, Cold Spring Harbor Laboratory Press, 1988; Ausubel et al., Current Protocols in Molecular Biology, John Wiley & Sons, NY, 1994). As used herein, the terms "expression vector" and "expression construct" refer to a vector or construct that comprises a nucleotide sequence encoding at least a portion of a gene product that is transcribed, and in some cases, the resulting RNA is translated into a protein, polypeptide, or peptide. Expression constructs may include various regulatory elements, and in addition to regulatory sequences that control transcription and translation, vectors and expression vectors can further comprise nucleotide sequences that provide additional functions. Viruses useful in the present invention include, but are not limited to, retroviruses, herpes simplex viruses, lentiviruses, poxviruses, vaccinia viruses, rhabdoviruses, adenoviruses, helper-dependent adenoviruses, and adeno-associated viruses (AAV).

A vector can be engineered to encode a selectable marker or reporter that permits selection or identification of cells that contain the vector. Expression of the selectable marker or reporter enables identification and/or selection of host cells that have integrated and express other coding regions present in the vector. Non-limiting examples of selectable marker genes known and used in the art include genes conferring resistance to ampicillin, streptomycin, gentamicin, kanamycin, hygromycin, the herbicide bialaphos, or sulfonamides, and genes that serve as phenotypic markers such as anthocyanin regulatory genes and isopentenyl transferase genes. Non-limiting examples of reporters known and used in the art include luciferase (Luc), green fluorescent protein (GFP), chloramphenicol acetyltransferase (CAT), β-galactosidase (LacZ), and β-glucuronidase (Gus). A selectable marker can also function as a reporter.

In general, recombinant adeno-associated virus (AAV) is produced by triple transfection of a host cell (for example, a HEK293 cell). The three plasmids are: (1) an AAV transfer plasmid comprising a gene expression cassette flanked by inverted terminal repeats (ITRs); (2) a "Rep-Cap plasmid" providing Rep proteins required for replication of the AAV genome and capsid proteins that form the viral particles; and (3) a "helper plasmid" providing adenoviral proteins (E2a, E4) and RNAs (VA RNA) that support the AAV life cycle. AAV particles are generated when these three types of plasmids are co-transfected into HEK293 cells or other cells that provide adenoviral E1 and E3 gene functions.

As used herein, the term "dual helper plasmid" refers to a plasmid that provides two or more of the requirements for AAV production in a cell. As will be apparent from the present disclosure, in certain aspects, a dual helper plasmid described herein provides both requirement (2) the Rep-Cap function, and requirement (3) the helper function. For example, in some aspects, a dual helper plasmid comprises a rep gene, a cap gene, an E2a gene, an E4 gene, and a VA RNA gene.

The dual helper plasmids described herein not only comprise the above-described genes, but also arrange these genes in specific configurations within the plasmid. For example, in some aspects, the E2a gene, the E4 gene, and the VA RNA gene are sequentially linked within the dual helper plasmid, and the rep gene and cap gene (collectively referred to herein as the "rep-cap genes") are sequentially linked in a clockwise direction (5' to 3') between the 5' end of the E2a gene and the 3' end of the VA RNA gene. More specifically, in some aspects, the 5' end of the rep-cap genes is linked to the 5' end of the E2a gene, and the 3' end of the rep-cap genes is linked to the 3' end of the VA RNA gene. In other aspects, the E2a gene, the E4 gene, and the VA RNA gene are sequentially linked and the rep-cap genes are positioned between the 5' end of the E2a gene and the 3' end of the VA RNA gene in a counter-clockwise orientation (3' to 5'). More specifically, in some aspects, the 3' end of the rep-cap genes is linked to the 5' end of the E2a gene, and the 5' end of the rep-cap genes is linked to the 3' end of the VA RNA gene.

As used herein, the term "cell" includes eukaryotic and prokaryotic cells and refers to any transformable cell that is capable of replicating a vector and/or expressing a gene encoded by the vector. A cell can be transfected, transduced, or transformed with the vector, which, as used herein, refers to processes by which an exogenous polynucleotide (nucleic acid molecule) is delivered or introduced into a host cell. As used herein, the term "transformation" is used in a broad sense to encompass transfection and transduction.

Host cells of the invention are not particularly limited and are preferably insect or mammalian cells, more preferably insect cells such as Sf9 cells, and mammalian cells such as HEK293 cells, HeLa cells, ARPE-19 cells, RPE-1 cells, HepG2 cells, Hep3B cells, Huh-7 cells, C8D1a cells, Neuro2A cells, CHO cells, MES13 cells, BHK-21 cells, COS7 cells, COP5 cells, A549 cells, MCF-7 cells, HC70 cells, HCC1428 cells, BT-549 cells, PC3 cells, LNCaP cells, Capan-1 cells, Panc-1 cells, MIA PaCa-2 cells, SW480 cells, HCT166 cells, LoVo cells, A172 cells, MKN-45 cells, MKN-74 cells, Kato-III cells, NCI-N87 cells, HT-144 cells, SK-MEL-2 cells, SH-SY5Y cells, C6 cells, HT-22 cells, or NIH3T3 cells. In some aspects, the host cell is an isolated host cell.

### II. Polypeptides

In one aspect, the present disclosure provides a polypeptide having the ability to increase neurite length and/or the number of branch points.

In a preferred embodiment, the polypeptide comprises an amino acid sequence of a TAFA (TAFA Chemokine Like Family Member) protein, or an amino acid sequence of a fragment or a variant thereof. In a preferred embodiment, the polypeptide comprises, from the N-terminus to the C-terminus, an amino acid sequence of General Formula 7:

General Formula 7: X1-X2-X3-G-T-C-E-V-X4-A-X5-H-X6-C-C-N-X7-N-X8-I-E-E-X9-S-Q-T-X10-X11-C-S-C- X12-X13-G-X14-V-A-G-T-T-X15-X16-X17-P-S-C-V-X18-A-X19-I-V-X20-X21-X22-W-W- C-X23-M-X24-P-C-X25-X26-G-E-X27-C-K-X28-L-P-D-X29-X30-G-W-X31-C-X32-X33- G-X34-K-X35-K-T-T-X36-X37-X38-X39

In General Formula 1:
X1 is absent, V, I, or L;
X2 is K, E, R, or Q;
X3 is G, T, Q, P, or A;
X4 is V or I;
X5 is A, L, V, or I;
X6 is R or L;
X7 is K, R, or Q;
X8 is R or K;
X9 is R or L;
X10 is V or G;
X11 is K or N;
X12 is F or L;
X13 is P or S;
X14 is Q or K;
X15 is R, H, or Q;
X16 is A, N, S, or T;
X17 is A, Q, R, K, or T;
X18 is D or E;
X19 is S or A;
X20 is I, E, L, A, or V;
X21 is Q, G, or E;
X22 is K or R;
X23 is H, Q, or E;
X24 is E, Q, N, D, S, or H;
X25 is L, V, or M;
X26 is E, D, P, L, or A;
X27 is E or D;
X28 is V, T, A, or I;
X29 is L, N, R, Y, S, or Q;
X30 is S, K, or T;
X31 is S or M;
X32 is S, A, or Y;
X33 is S, T, or R;
X34 is N or H;
X35 is V or I;
X36 is R or K;
X37 is absent, V, A, G, M, or N;
X38 is absent, T, I, N, F, or S; and
X39 is absent, R, H, V, K, I, or Q.

In a preferred embodiment, the polypeptide comprises one or more amino acid sequences selected from the group consisting of the amino acid sequences set forth in SEQ ID NOS: 87 to 141.

In a preferred embodiment, the polypeptide may consist of from 8 to 61 amino acid residues.

In some embodiments, the polypeptide may consist of 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, or 61 amino acid residues.

In a preferred embodiment, the polypeptide comprises an amino acid sequence having at least 50% sequence identity with the amino acid sequence of SEQ ID NO: 1, SEQ ID NO: 15, or SEQ ID NO: 87.

In some embodiments, the polypeptide has at least 50%, at least 51%, at least 52%, at least 53%, at least 54%, at least 55%, at least 56%, at least 57%, at least 58%, at least 59%, at least 60%, at least 61%, at least 62%, at least 63%, at least 64%, at least 65%, at least 66%, at least 67%, at least 68%, at least 69%, at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, or at least 95% sequence identity with the amino acid sequence of SEQ ID NO: 1, SEQ ID NO: 15, or SEQ ID NO: 87.

In a preferred embodiment, the amino acid sequence of the polypeptide comprises, from the N-terminus to the C-terminus, the amino acid sequence of General Formula 1:

General Formula 1: X1-G-E-X2-C-K-X3-L

In General Formula 1,
X1 is E, D, P, L, or A;
X2 is D or E; and
X3 is T, V, I, or A.

In some embodiments, a polypeptide comprising the amino acid sequence of General Formula 1 comprises the amino acid sequence of SEQ ID NO: 142.

In some embodiments, a polypeptide comprising the amino acid sequence of General Formula 1 comprises an amino acid sequence selected from the group consisting of SEQ ID NOS: 1 to 14.

In some embodiments, a polypeptide comprising the amino acid sequence of General Formula 1 may consist of from 8 to 43 amino acid residues.

In some embodiments, such a polypeptide may consist of 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, or 43 amino acid residues.

In a preferred embodiment, the amino acid sequence of the polypeptide comprises, from the N-terminus to the C-terminus, the amino acid sequence of General Formula 2:

General Formula 2: I-V-X4-X5-X6-W-W-C-X7-M-X8-P-C-X9-X1-G-E-X2-C-K-X3-L

In General Formula 2,
X1 is E, D, P, L, or A;
X2 is D or E;
X3 is T, V, I, or A;
X4 is I, E, A, L, or V;
X5 is Q, E, or G;
X6 is K or R;
X7 is E, H, or Q;
X8 is E, Q, N, D, S, or H; and
X9 is L, M, or V.

In some embodiments, a polypeptide comprising the amino acid sequence of General Formula 2 may comprise the amino acid sequence of SEQ ID NO: 143.

In some embodiments, a polypeptide comprising the amino acid sequence of General Formula 2 may comprise an amino acid sequence selected from the group consisting of SEQ ID NOS: 28 to 51.

In a preferred embodiment, the amino acid sequence of the polypeptide comprises, from the N-terminus to the C-terminus, the amino acid sequence of General Formula 3:

General Formula 3: X1-G-E-X2-C-K-X3-L-P-D-X4-X5-G-W-S-C-S-X6-G-N-K-X7-K-T-T-K-V-T-R

In General Formula 3,
X1 is E, D, P, L, or A;
X2 is D or E;
X3 is T, V, I, or A;
X4 is Y, S, or L;
X5 is S or T;
X6 is S or T; and
X7 is V or I.

In some embodiments, a polypeptide comprising the amino acid sequence of General Formula 3 may comprise the amino acid sequence of SEQ ID NO:144.

In some embodiments, a polypeptide comprising the amino acid sequence of General Formula 3 may comprise an amino acid sequence selected from the group consisting of SEQ ID NOS: 52 to 58.

In some embodiments, the amino acid sequence of the polypeptide may comprise one or more amino acid sequences selected from the group consisting of SEQ ID NOS: 152 to 171.

In a preferred embodiment, the amino acid sequence of the polypeptide comprises, from the N-terminus to the C-terminus, the amino acid sequence of General Formula 4:

General Formula 4: G-X1-V-A-G-T-T-X2-X3-X4-P-S-C-V-X5-X6-X7

In General Formula 4,
X1 is Q or K;
X2 is R, H, or Q;
X3 is A, N, S, or T;
X4 is R, A, Q, K, or T;
X5 is D or E;
X6 is A or absent; and
X7 is S, A, or absent.

In some embodiments, a polypeptide comprising the amino acid sequence of General Formula 4 may comprise the amino acid sequence of SEQ ID NO: 145.

In some embodiments, a polypeptide comprising the amino acid sequence of General Formula 4 may comprise an amino acid sequence selected from the group consisting of SEQ ID NOS: 15 to 27.

In some embodiments, a polypeptide comprising the amino acid sequence of General Formula 4 may consist of from 15 to 46 amino acid residues.

In some embodiments, such a polypeptide may consist of 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, or 46 amino acid residues.

In a preferred embodiment, the amino acid sequence of the polypeptide comprises, from the N-terminus to the C-terminus, the amino acid sequence of General Formula 5:

General Formula 5: X8-I-E-E-X9-S-Q-T-X10-X11-C-S-C-X12-X13-G-X1-V-A-G-T-T-X2-X3-X4-P-S-C-V-X5-X 6-X7

In General Formula 5,
X1 is Q or K;
X2 is R, H, or Q;
X3 is A, N, S, or T;
X4 is R, A, Q, K, or T;
X5 is D or E;
X6 is A or absent;
X7 is S, A, or absent;
X8 is R or K;
X9 is R or L;
X10 is V or G;
X11 is K or N;
X12 is F or L; and
X13 is P or S.

In some embodiments, a polypeptide comprising the amino acid sequence of General Formula 5 may comprise the amino acid sequence of SEQ ID NO: 146.

In some embodiments, a polypeptide comprising the amino acid sequence of General Formula 5 may comprise an amino acid sequence selected from the group consisting of SEQ ID NOS: 59 to 74.

In a preferred embodiment, the amino acid sequence of the polypeptide comprises, from the N-terminus to the C-terminus, the amino acid sequence of General Formula 6:

General Formula 6: G-X1-V-A-G-T-T-X2-X3-X4-P-S-C-V-X5-X6-X7-I-V-X8-X9-K-W-W-C-X10-M-X11-P-C-X 12

In General Formula 6,
X1 is Q or K;
X2 is R, H, or Q;
X3 is A, N, S, or T;
X4 is R, A, Q, K, or T;
X5 is D or E;
X6 is A or absent;
X7 is S, A, or absent;
X8 is I, A, V, or L;
X9 is Q or E;
X10 is H or Q;
X11 is N, D, S, or H; and
X12 is L or M.

In some embodiments, a polypeptide comprising the amino acid sequence of General Formula 6 may comprise the amino acid sequence of SEQ ID NO: 147.

In some embodiments, a polypeptide comprising the amino acid sequence of General Formula 6 may comprise an amino acid sequence selected from the group consisting of SEQ ID NOS: 75 to 85.

In some embodiments, the amino acid sequence of the polypeptide may comprise one or more amino acid sequences selected from the group consisting of SEQ ID NOS: 172 to 184.

### III. Nucleic acids

In another aspect, the present disclosure provides a nucleic acid molecule encoding the polypeptide described above.

The polypeptide, TAFA protein, or fragment or variant thereof is as described in section II.

A nucleic acid molecule useful in the present disclosure is not particularly limited as long as the nucleic acid can be translated into a polypeptide when transduced into a cell. In some embodiments, the nucleic acid encodes a polypeptide (for example, a TAFA polypeptide or a polypeptide comprising any one of the amino acid sequences of SEQ ID NOS: 142 to 147) or a fusion protein.

In some embodiments, the nucleic acid encodes a protein useful for the prevention or treatment of a disease or disorder such as those described herein. In some embodiments, the nucleic acid encodes a peptide for the prevention or treatment of a particular disease, which is intended for continuous expression within the body of a subject or patient.

In some embodiments, the nucleic acid molecule has at least 50%, at least 60%, at least 70%, at least 80%, at least 85%, at least 90%, at least 95%, at least 96%, at least 97%, at least 98%, at least 99%, or 100% sequence identity with a nucleic acid encoding the TAFA protein or a fragment or variant thereof.

In some embodiments, the nucleic acid encoding the polypeptide further comprises a sequence encoding a signal peptide.

In some embodiments, the polynucleotides described herein further comprise a regulatory element. Thus, in some embodiments, the polynucleotide comprises (1) a regulatory element, (2) an untranslated nucleic acid sequence as described herein (for example, an EF-1α intron or a fragment thereof), and (3) a transgene (for example, a nucleic acid molecule encoding a TAFA polypeptide or a polypeptide comprising any one of the amino acid sequences of SEQ ID NOS: 142 to 147).

As used herein, the term "regulatory element" refers to a nucleic acid sequence that regulates (for example, increases or decreases) the expression of an operably linked nucleic acid. Regulatory elements useful in the present disclosure include, for example, enhancers (for example, a CMV enhancer), promoters (for example, a CMV promoter, an EF-1α promoter, or a β-actin promoter), exons (for example, exon 1 or exon 2), splicing donor sequences, acceptor sequences, or combinations thereof. In some embodiments, the regulatory element further comprises a sequence for transcription termination (for example, a poly A sequence), a sequence for stable expression of a transgene (for example, a WPRE sequence), a sequence for reducing the development of transgene-specific immunity (for example, a miRNA target sequence), or a combination thereof.

### IV. Vectors comprising nucleic acids encoding polypeptides

In another aspect, the present disclosure provides a vector comprising the nucleic acid molecule described above.

The nucleic acid molecule is as described in section III.

As described herein, such vectors are useful for recombinant expression in host cells and in cells targeted for therapeutic intervention. In some embodiments, a vector useful for delivering the polynucleotides described herein (for example, a nucleic acid molecule encoding a TAFA polypeptide or a polypeptide comprising any one of the amino acid sequences of SEQ ID NOS: 142 to 147) comprises a viral vector. Examples of viruses that can be used as vectors in the present disclosure include, but are not limited to, retroviruses, herpes simplex viruses, lentiviruses, poxviruses, vaccinia viruses, rhabdoviruses, adenoviruses, helper-dependent adenoviruses, adeno-associated viruses (AAVs), baculoviruses, and combinations thereof. In some embodiments, vectors that can be used in the present disclosure comprise non-viral vectors. Non-limiting examples of such vectors include plasmids, cosmids, yeast artificial chromosomes (YACs), bacteriophages, and combinations thereof.

In some embodiments, the vector comprises one or more sequences selected from the group consisting of a promoter sequence, an enhancer sequence, an exon sequence, an intron sequence, a signal sequence-coding sequence, a splicing donor sequence, and one or more adeno-associated viral inverted terminal repeat (ITR) sequences.

In another aspect, the present disclosure provides a recombinant viral particle comprising the vector and a capsid protein.

In a preferred embodiment, the virus may be an AAV.

### V. AAV

In some embodiments, the polynucleotides described herein (for example, a nucleic acid molecule encoding a TAFA polypeptide or a polypeptide comprising any one of the amino acid sequences of SEQ ID NOS: 142 to 147) are delivered to cells, for example, using an AAV. As a single-strand DNA virus, adeno-associated virus (AAV) is a helper-dependent human parvovirus. The AAV genome has a size of about 4.7 kbp and comprises an N-terminal region encoding the rep gene involved in viral replication and expression of viral genes, a C-terminal region encoding the cap gene encoding the capsid protein of the virus, and inverted terminal repeats (ITRs) of about 145 bases inserted at each end. The 145 bp ITRs have a T-shaped structure, function as the origin of replication during replication of the viral genome, and act as the primary packaging signal. The ITR is the only cis-acting sequence required to make recombinant AAV (rAAV) constructs. The ITR has enhancer activity in the presence of Rep protein but very weak activity in the absence of Rep protein. Accordingly, when cloning a transgene into a recombinant AAV construct, these features are taken into consideration and expression constructs are prepared with appropriate enhancers, promoters, pA, and the like (RJ Samulski and N Muzyczka, Annu. Rev. Virolo. 2014. 1:427-451). Four proteins are translated from the rep gene, designated rep78, rep68, rep52, and rep40 according to their molecular weights, and they perform essential functions in AAV DNA replication. Four proteins are translated from the cap gene; of these, VP1, VP2, and VP3 are structural proteins that constitute AAV particles, and the assembly-activating protein (AAP) promotes assembly of AAV particles by the structural proteins. For efficient replication of adeno-associated virus, some proteins and RNAs derived from helper viruses such as adenovirus or herpes simplex virus are required (Muzyczka N. Curr Top Microbiol Immunol 158, 97-129, 1992).

AAV is an attractive vector for delivering transgenes into cells. Infection of cultured cells with AAV is generally non-cytopathic, and natural infection of humans and other animals is asymptomatic and subclinical. Furthermore, AAV can infect many different types of mammalian cells and thereby has the potential to target many different tissues in vivo. AAV also has additional advantages that make it a particularly attractive viral system for gene delivery, including induction of weaker immune responses than other forms of gene delivery and sustained expression from non-integrating, episomal vector DNA in both dividing and non-dividing cells. In addition, AAV can withstand conditions used to inactivate adenovirus (for example, 56-65°C for several hours), so that low-temperature storage of rAAV-based vaccines is less critical.

The types or serotypes of adeno-associated virus that can be used in the present disclosure include, but are not limited to, AAVrh.10 (AAVrh10), AAV-DJ (AAVDJ), AAV-DJ8 (AAVDJ8), AAV1, AAV2, AAV2G9, AAV3, AAV3a, AAV3b, AAV3-3, AAV4, AAV4-4, AAV5, AAV6, AAV6.1, AAV6.2, AAV6.1.2, AAV7, AAV7.2, AAV8, AAV9, AAV9.11, AAV9.13, AAV9.16, AAV9.24, AAV9.45, AAV9.47, AAV9.61, AAV9.68, AAV9.84, AAV9.9, AAV10, AAV11, AAV12, AAV16.3, AAV24.1, AAV27.3, AAV42.12, AAV42-1b, AAV42-2, AAV42-3a, AAV42-3b, AAV42-4, AAV42-5a, AAV42-5b, AAV42-6b, AAV42-8, AAV42-10, AAV42-11, AAV42-12, AAV42-13, AAV42-15, AAV42-aa, AAV43-1, AAV43-12, AAV43-20, AAV43-21, AAV43-23, AAV43-25, AAV43-5, AAV44.1, AAV44.2, AAV44.5, AAV223.1, AAV223.2, AAV223.4, AAV223.5, AAV223.6, AAV223.7, AAV1-7/rh.48, AAV1-8/rh.49, AAV2-15/rh.62, AAV2-3/rh.61, AAV2-4/rh.50, AAV2-5/rh.51, AAV3.1/hu.6, AAV3.1/hu.9, AAV3-9/rh.52, AAV3-11/rh.53, AAV4-8/r11.64, AAV4-9/rh.54, AAV4-19/rh.55, AAV5-3/rh.57, AAV5-22/rh.58, AAV7.3/hu.7, AAV16.8/hu.10, AAV16.12/hu.11, AAV29.3/bb.1, AAV29.5/bb.2, AAV106.1/hu.37, AAV114.3/hu.40, AAV127.2/hu.41, AAV127.5/hu.42, AAV128.3/hu.44, AAV130.4/hu.48, AAV145.1/hu.53, AAV145.5/hu.54, AAV145.6/hu.55, AAV161.10/hu.60, AAV161.6/hu.61, AAV33.12/hu.17, AAV33.4/hu.15, AAV33.8/hu.16, AAV52/hu.19, AAV52.1/hu.20, AAV58.2/hu.25, AAVA3.3, AAVA3.4, AAVA3.5, AAVA3.7, AAVC1, AAVC2, AAVC5, AAVF3, AAVF5, AAVH2, AAVrh.72, AAVhu.8, AAVrh.68, AAVrh.70, AAVpi.1, AAVpi.3, AAVpi.2, AAVrh.60, AAVrh.44, AAVrh.65, AAVrh.55, AAVrh.47, AAVrh.69, AAVrh.45, AAVrh.59, AAVhu.12, AAVH6, AAVLK03, AAVH-1/hu.1, AAVH-5/hu.3, AAVLG-10/rh.40, AAVLG-4/rh.38, AAVLG-9/hu.39, AAVN721-8/rh.43, AAVCh.5, AAVCh.5R1, AAVcy.2, AAVcy.3, AAVcy.4, AAVcy.5, AAVCy.5R1, AAVCy.5R2, AAVCy.5R3, AAVCy.5R4, AAVcy.6, AAVhu.1, AAVhu.2, AAVhu.3, AAVhu.4, AAVhu.5, AAVhu.6, AAVhu.7, AAVhu.9, AAVhu.10, AAVhu.11, AAVhu.13, AAVhu.15, AAVhu.16, AAVhu.17, AAVhu.18, AAVhu.20, AAVhu.21, AAVhu.22, AAVhu.23.2, AAVhu.24, AAVhu.25, AAVhu.27, AAVhu.28, AAVhu.29, AAVhu.29R, AAVhu.31, AAVhu.32, AAVhu.34, AAVhu.35, AAVhu.37, AAVhu.39, AAVhu.40, AAVhu.41, AAVhu.42, AAVhu.43, AAVhu.44, AAVhu.44R1, AAVhu.44R2, AAVhu.44R3, AAVhu.45, AAVhu.46, AAVhu.47, AAVhu.48, AAVhu.48R1, AAVhu.48R2, AAVhu.48R3, AAVhu.49, AAVhu.51, AAVhu.52, AAVhu.54, AAVhu.55, AAVhu.56, AAVhu.57, AAVhu.58, AAVhu.60, AAVhu.61, AAVhu.63, AAVhu.64, AAVhu.66, AAVhu.67, AAVhu.14/9, AAVhu.t19, AAVrh.2, AAVrh.2R, AAVrh.8, AAVrh.8R, AAVrh.12, AAVrh.13, AAVrh.13R, AAVrh.14, AAVrh.17, AAVrh.18, AAVrh.19, AAVrh.20, AAVrh.21, AAVrh.22, AAVrh.23, AAVrh.24, AAVrh.25, AAVrh.31, AAVrh.32, AAVrh.33, AAVrh.34, AAVrh.35, AAVrh.36, AAVrh.37, AAVrh.37R2, AAVrh.38, AAVrh.39, AAVrh.40, AAVrh.46, AAVrh.48, AAVrh.48.1, AAVrh.48.1.2, AAVrh.48.2, AAVrh.49, AAVrh.51, AAVrh.52, AAVrh.53, AAVrh.54, AAVrh.56, AAVrh.57, AAVrh.58, AAVrh.61, AAVrh.64, AAVrh.64R1, AAVrh.64R2, AAVrh.67, AAVrh.73, AAVrh.74, AAVrh8R, AAVrh8R A586R variant, AAVrh8R R533A variant, AAAV, BAAV, caprine AAV, bovine AAV, AAVhE1.1, AAVhEr1.5, AAVhER1.14, AAVhEr1.14, AAVhEr1.8, AAVhEr1.16, AAVhEr1.18, AAVhEr1.35, AAVhEr1.7, AAVhEr1.36, AAVhEr2.29, AAVhEr2.4, AAVhEr2.16, AAVhEr2.16, AAVhEr2.30, AAVhEr2.31, AAVhEr2.31, AAVhEr2.36, AAVhER1.23, AAVhEr3.1, AAV2.5T, AAV-PAEC, AAV-LK01, AAV-LK02, AAV-LK03, AAV-LK04, AAV-LK05, AAV-LK06, AAV-LK07, AAV-LK08, AAV-LK09, AAV-LK10, AAV-LK11, AAV-LK12, AAV-LK13, AAV-LK14, AAV-LK15, AAV-LK16, AAV-LK17, AAV-LK18, AAV-LK19, AAV-PAEC2, AAV-PAEC4, AAV-PAEC6, AAV-PAEC7, AAV-PAEC8, AAV-PAEC11, AAV-PAEC12, AAV-2-pre-miRNA-101, AAV-8h, AAV-8b, AAV-h, AAV-b, AAV SM 10-2, AAV Shuffle 100-1, AAV Shuffle 100-3, AAV Shuffle 100-7, AAV Shuffle 10-2, AAV Shuffle 10-6, AAV Shuffle 10-8, AAV Shuffle 100-2, AAV SM 10-1, AAV SM 10-8, AAV SM 100-3, AAV SM 100-10, B P61 AAV, B P62 AAV, B P63 AAV, AAVrh.50, AAVrh.43, AAVrh.62, AAVrh.48, AAVhu.19, AAVhu.11, AAVhu.53, AAV4-8/rh.64, AAVLG-9/hu.39, AAV54.5/hu.23, AAV54.2/hu.22, AAV54.7/hu.24, AAV54.1/hu.21, AAV54.4R/hu.27, AAV46.2/hu.28, AAV46.6/hu.29, AAV128.1/hu.43, true type AAV (ttAAV), UPENN AAV10, and Japanese AAV10 serotypes.

In some embodiments, the serotype of the adeno-associated virus is AAV1, AAV2, AAV3, AAV4, AAV5, AAV6, AAV7, AAV8, AAV9, or AAVrh10. In some embodiments, the serotype of the AAV is AAV2. In some embodiments, the serotype of the AAV is AAV5. In some embodiments, the serotype of the AAV is AAV8. In some embodiments, the serotype of the AAV is AAV9.

In some embodiments, for efficient expression or production of the adeno-associated virus, or for other purposes such as retargeting, one or more amino acid sequences of the Rep and/or Cap proteins are mutated, or new amino acid sequences are added or deleted, and the sequence of the gene encoding them may likewise be mutated. Such modified AAVs are included within the AAVs of the present disclosure as long as they retain AAV function.

### VI. Cells

In some embodiments, the present disclosure provides cells comprising any of the polynucleotides described herein (for example, a nucleic acid molecule encoding a TAFA polypeptide or a polypeptide comprising any one of the amino acid sequences of SEQ ID NOS: 142 to 147) or cells comprising a vector containing such polynucleotides.

The nucleic acid molecules are as described in section III, and the vectors are as described in section IV.

For example, in some embodiments, cells are transduced, transfected, or transformed using a vector (for example, an AAV vector) comprising a transgene described herein (for example, a nucleic acid molecule encoding a TAFA polypeptide or a polypeptide comprising any one of the amino acid sequences of SEQ ID NOS: 142 to 147) and an untranslated nucleic acid sequence for expression of the transgene.

Without intending to be bound by any particular theory, in some embodiments, cells described herein (for example, cells transduced with a polynucleotide comprising an untranslated nucleic acid sequence) are useful for producing a protein encoded by a transgene described herein (for example, a TAFA polypeptide or a polypeptide comprising any one of the amino acid sequences of SEQ ID NOS: 142 to 147). As described herein, in some embodiments, an untranslated nucleic acid sequence (for example, an EF-1α intron or a fragment thereof) can enhance expression of the protein encoded by the transgene in a cell. Accordingly, in some embodiments, cells described herein (for example, cells transduced with a polynucleotide comprising a transgene and an untranslated nucleic acid sequence) exhibit greater expression of the encoded protein compared to a reference cell. In some embodiments, the reference cell is transduced with a corresponding polynucleotide lacking the untranslated nucleic acid sequence.

In some embodiments, cells described herein can produce the encoded protein in vitro. In certain embodiments, cells described herein can produce the encoded protein in vivo (for example, in a subject administered a polynucleotide described herein). In some embodiments, cells described herein can produce the encoded protein both in vitro and in vivo.

In some embodiments, cells that can be used to produce a protein encoded by a transgene (for example, in vitro) comprise host cells. As used herein, the term "host cell" is intended to include cells of any organism that can be transduced with the expression construct or vector (for example, AAV vector) to replicate the expression construct or express a gene encoded by the expression construct. Such cells include eukaryotic cells and prokaryotic cells. As used herein, the term "transduction" is intended to include transfection and transformation. Host cells can be transduced, transfected, or transformed with the expression construct, which refers to the process by which an exogenous nucleic acid molecule is delivered or introduced into the host cell. In some embodiments, the host cell is an isolated host cell comprising the AAV vector. In some embodiments, the host cell is an isolated host cell transformed with the AAV vector.

In some embodiments, the host cell is a eukaryotic cell. In some embodiments, the host cell is selected from the group consisting of a mammalian cell, an insect cell, a yeast cell, a transgenic mammalian cell, and a plant cell. In some embodiments, the host cell is a prokaryotic cell. In some embodiments, the prokaryotic cell is a bacterial cell.

In some embodiments, the host cell is an insect cell. In some embodiments, the insect cell is an Sf9 cell. In some embodiments, the host cell is a mammalian cell. Non-limiting examples of mammalian cells that can be used in the present disclosure include HEK293, HeLa, ARPE-19, RPE-1, HepG2, Hep3B, Huh-7, C8D1a, Neuro2A, CHO, MES13, BHK-21, COS7, COP5, A549, MCF-7, HC70, HCC1428, BT-549, PC3, LNCaP, Capan-1, Panc-1, MIA PaCa-2, SW480, HCT166, LoVo, A172, MKN-45, MKN-74, Kato-III, NCI-N87, HT-144, SK-MEL-2, SH-SY5Y, C6, HT-22, PC-12, NIH3T3 cells, and combinations thereof.

In some embodiments, cells that can be used to produce a protein encoded by a transgene comprise human cells (for example, in vivo). In some embodiments, the human cells are cells of a subject administered a nucleic acid molecule described herein. In certain embodiments, the human cells are derived from a donor (for example, a healthy human subject).

In some embodiments, the present disclosure provides a composition comprising an AAV vector or a host cell comprising the AAV vector or transformed with the AAV vector.

### VII. Compositions

In another aspect, the present disclosure provides a composition comprising the polypeptide, the nucleic acid molecule encoding the polypeptide, the vector comprising the nucleic acid molecule, the recombinant viral particle comprising the vector and capsid protein, the cell comprising the nucleic acid molecule or vector, or a combination thereof.

The polypeptides, nucleic acid molecules, vectors, recombinant viral particles, and cells are as described in sections II to VI.

In some embodiments, the composition is a pharmaceutical composition.

In some embodiments, the present disclosure provides a pharmaceutical composition comprising (a) a polypeptide described herein (for example, a TAFA polypeptide or a polypeptide comprising any one of the amino acid sequences of SEQ ID NOS: 142 to 147) or a nucleic acid molecule encoding the same, and (b) one or more pharmaceutically acceptable carriers. In some embodiments, the present disclosure provides a pharmaceutical composition comprising (a) a vector (for example, an rAAV) or recombinant viral particle described herein and (b) one or more pharmaceutically acceptable carriers. In some embodiments, the present disclosure provides a pharmaceutical composition comprising (a) a cell described herein and (b) one or more pharmaceutically acceptable carriers.

In some embodiments, the pharmaceutical composition described herein comprises a polypeptide consisting of 8 to 61 amino acid residues.

In some embodiments, the pharmaceutical composition described herein comprises a polypeptide including an amino acid sequence having at least 50% sequence identity with the amino acid sequence of SEQ ID NO: 1, SEQ ID NO: 15, or SEQ ID NO: 87.

In some embodiments, the pharmaceutical composition is a pharmaceutical composition for the prevention, amelioration, or treatment of retinal neurodegenerative diseases.

In some embodiments, the retinal neurodegenerative disease is caused by damage to all or part of the retina or macula. In some embodiments, the retinal neurodegenerative disease is a disease caused by dysfunction or damage of retinal or macular cells. In some embodiments, the retinal neurodegenerative disease may be a disease caused by dysfunction or damage of photoreceptor cells (for example, rod and cone cell layers) and/or retinal pigment epithelial (RPE) cells of the retina or macula. In some embodiments, the retinal neurodegenerative disease is retinopathy, choroidal neovascularization, maculopathy, macular degeneration, retinal degeneration, macular edema, retinal edema, macular swelling, retinal swelling, color vision abnormality, retinal cell degeneration, retinal vascular occlusion, retinal detachment, an inherited retinal disease, or a combination thereof. In some embodiments, the macular degeneration is age-related macular degeneration, Best macular dystrophy, Sorsby fundus dystrophy, malattia Leventinese, Doyne honeycomb retinal dystrophy, Stargardt's disease (Stargardt macular dystrophy), myopic macular degeneration, or pigment epithelial detachment-related macular degeneration. In some embodiments, the macular degeneration is age-related macular degeneration. In some embodiments, the age-related macular degeneration is wet or dry age-related macular degeneration. In some embodiments, the retinopathy is retinal dystrophy. In some embodiments, the retinopathy is diabetic retinopathy. In some embodiments, the diabetic retinopathy is non-proliferative diabetic retinopathy (NPDR), proliferative diabetic retinopathy (PDR), diabetic maculopathy, diabetic macular edema, or a combination thereof. In some embodiments, the retinal neurodegenerative disease is an inherited retinal disease. In some embodiments, the color vision abnormality is color blindness or color weakness. In some embodiments, the color vision abnormality is deuteranopia, protanopia, tritanopia, deuteranomaly, protanomaly, or tritanomaly. In some embodiments, the color vision abnormality is achromatopsia, trichromatic abnormality, or total color blindness. In one embodiment, the polypeptide of the present disclosure or a nucleic acid molecule encoding the same is useful for preventing or treating a color vision abnormality by reversing or restoring destruction, loss, or dysfunction of cone cells, one type of photoreceptor.

In some embodiments, the inherited retinal disease is retinitis pigmentosa (RP), Leber congenital amaurosis, Stargardt's disease, Coats retinopathy, cone dystrophy, choroideremia, Usher syndrome, Best disease, X-linked retinoschisis, inherited color vision abnormality, or unspecified hereditary retinal dystrophy.

In some embodiments, the pharmaceutical composition is a pharmaceutical composition for the prevention, amelioration, or treatment of neuropathic pain.

In some embodiments, the neuropathic pain is central neuropathic pain, that is, pain resulting from injury or damage affecting all levels of the central nervous system, including the central somatosensory nervous system (for example, pain due to brain injury or spinal cord injury), or pain caused by, or associated with, diseases or disorders such as seizures, multiple sclerosis, or lateral medullary infarction. In some embodiments, central neuropathic pain may be spontaneous or stimulus-evoked. In some embodiments, central neuropathic pain may include mechanical allodynia and cold allodynia. Symptoms of central neuropathic pain typically include, for example, burning, aching, shooting pain, pressure, painful cold, paresthesia, and dysesthesia (for example, tingling, pins-and-needles pain, cold sensation, and a feeling of pressure). The distribution of central neuropathic pain may include, for example, areas ranging from a small region in a peripheral territory to a wide region, or areas that, in the case of spinal cord injury or stroke, cover half of the body, include one side of the face, or involve the side of the body or limb contralateral to the lesion. Central neuropathic pain due to spinal cord injury includes "at-level pain," perceived in a segmental pattern at the level of injury, and "below-level pain," perceived below the level of injury. In some embodiments, the methods described herein reduce, reverse, relieve, ameliorate, inhibit, attenuate, or prevent central neuropathic pain, pain-related symptoms, the underlying cause of the pain, or any combination thereof.

In some embodiments, the neuropathic pain is peripheral neuropathic pain, that is, pain caused by, or associated with, damage or injury affecting any part of the peripheral nervous system (for example, damage to motor nerves, sensory nerves, autonomic nerves, or any combination thereof) or pain caused by, or associated with, a disease or disorder. Damage or injury to motor nerves is associated with symptoms such as muscle weakness (for example, weakness of the back, leg, hip, or facial muscles), painful cramps, fasciculations (uncontrolled muscle twitching under the skin), muscle atrophy (marked reduction in muscle mass), and decreased reflexes. Damage or injury to sensory nerves causes various symptoms, including pain and hypersensitivity of pain receptors in the skin, resulting in allodynia (for example, severe pain induced by normally non-painful stimuli).

In some embodiments, the neuropathic pain treatable with the composition of the present disclosure is neuralgia, which includes, without limitation, trigeminal neuralgia (TN) (for example, pain in the facial or oral trigeminal nerve territory), atypical trigeminal neuralgia (ATN), occipital neuralgia, postherpetic neuralgia (for example, unilateral pain distributed in one or more dermatomes or in the ophthalmic division of the trigeminal nerve), peripheral nerve injury pain (for example, pain in the innervation territory of a lesioned nerve, typically distal pain in the innervation territory of an injured nerve in response to trauma, surgery, or compression), glossopharyngeal neuralgia (for example, severe pain in the throat, tongue, and retroauricular region caused by irritation of the ninth cranial nerve), sciatica, low back pain, and atypical facial pain. In some embodiments, the neuralgia is caused by, or associated with, chemical irritation, inflammation, trauma (including surgery), compression of a nerve by an adjacent structure (for example, a tumor), infection, or a combination thereof. In some embodiments, the neuropathic pain is deafferentation pain syndrome, which includes, without limitation, pain due to brain or spinal cord injury, post-stroke pain, phantom limb pain, paraplegia, brachial plexus avulsion injury, and lumbar radiculopathy. In some embodiments, the neuropathic pain is complex regional pain syndrome (CRPS) including CRPS1 and CRPS2, without limitation. In some embodiments, symptoms associated with CRPS include severe pain, changes in nails, bones, and skin, and increased sensitivity to touch in the affected limb. In some embodiments, the neuropathic pain is a neuropathy (for example, central or peripheral). Non-limiting examples of neuropathic pain include mononeuropathic pain (mononeuropathy) and polyneuropathic pain (polyneuropathy). In some embodiments, the neuropathic pain is diabetic peripheral neuropathy.

In some embodiments, the compositions of the present disclosure prevent, ameliorate, or treat hyperalgesia. As used herein, the term "hyperalgesia" refers to an increased or exaggerated response to a painful stimulus (for example, pinprick or a hot plate). In some embodiments, hyperalgesia relates to mechanical stimuli such as pinprick (mechanical hyperalgesia). In other embodiments, hyperalgesia relates to thermal stimuli such as a hot plate (thermal hyperalgesia).

Pharmaceutically acceptable carriers useful in the present disclosure are those commonly used in formulation. Examples of pharmaceutically acceptable carriers include, but are not limited to, lactose, dextrose, sucrose, sorbitol, mannitol, starch, gum acacia, calcium phosphate, alginate, gelatin, calcium silicate, microcrystalline cellulose, polyvinylpyrrolidone, cellulose, water, syrup, methyl cellulose, methyl hydroxybenzoate, propyl hydroxybenzoate, talc, magnesium stearate, and mineral oil. The pharmaceutical composition of the present disclosure may further comprise one or more additives selected from the group consisting of lubricating agents, wetting agents, sweetening agents, flavoring agents, emulsifying agents, suspending agents, and preservatives. Details of suitable pharmaceutically acceptable carriers and formulations can be found in Remington's Pharmaceutical Sciences (19th ed., 1995).

The pharmaceutical compositions of the present disclosure are formulated to be compatible with their intended route of administration. Suitable non-oral routes of administration include, for example, intravenous injection, transdermal administration, subcutaneous injection, intramuscular injection, intraocular (for example, sub-Tenon, subconjunctival, suprachoroidal, sub-choroidal space, subretinal, intravitreal, and any other intraocular administration route capable of delivering the composition to similar locations), topical ocular, intracerebroventricular, intrathecal, intra-amniotic, intra-arterial, intra-articular, intracardiac, intracavernosal, intracerebral, cisternal, intracoronary, intracranial, intradural, epidural, intrahippocampal, intranasal, intraosseous, intraperitoneal, intrapleural, intraspinal, intrathoracic, intrathymic, intrauterine, intravaginal, intraventricular, intravesical, subconjunctival, intratumoral, local, intraperitoneal, and combinations thereof. In some aspects, intraocular administration includes suprachoroidal, subretinal, and intravitreal administration. In some aspects, local administration includes topical ocular, intranasal, transdermal, oral, or rectal administration. Because the amino acid sequence of the polypeptide of the present disclosure (for example, a TAFA protein fragment or variant thereof) may consist of 8 to 61 amino acids, preferably 8 to 30 amino acids, the intended prophylactic or therapeutic effect can be achieved by administration via topical ocular or intranasal routes.

In some embodiments, the pharmaceutical composition is administered in a daily dose of 0.0001 to 100 mg/kg.

The pharmaceutical composition of the present disclosure can be formulated with one or more pharmaceutically acceptable carriers and/or excipients. The composition may be provided in unit dosage form or dispensed in multi-dose containers. The formulation may be in the form of a solution, suspension, or emulsion in an oily or aqueous medium, or in the form of an extract, powder, granule, tablet, or capsule. The formulation may further comprise a dispersing agent or stabilizer.

### VIII. Kits

The present disclosure also provides kits comprising one or more polypeptides as described herein (for example, a TAFA polypeptide or a polypeptide comprising any one of the amino acid sequences of SEQ ID NOS: 142 to 147), one or more polynucleotides (for example, comprising a transgene and an untranslated nucleic acid sequence), one or more vectors as described herein (for example, an AAV vector), one or more cells as described herein (for example, host cells comprising the AAV vector or transformed with the AAV vector), any of the compositions described herein, or any combination thereof. In some embodiments, the kit further comprises instructions for use.

As used herein, the terms "kit" and "system" are intended, in some embodiments, to refer to at least one or more polynucleotides described herein, one or more vectors (for example, an AAV vector) described herein, one or more host cells described herein, any pharmaceutical composition described herein, or any combination thereof, optionally in combination with one or more additional types of elements or components (for example, other biochemical reagents, containers, packaging such as packaging intended for commercial sale, instructions for use, and the like).

### IX. Uses and methods

### IX.A. Methods of production

The present disclosure also provides methods of producing compositions comprising the polypeptide (for example, a TAFA polypeptide or a polypeptide comprising any one of the amino acid sequences of SEQ ID NOS: 142 to 147), the nucleic acid molecule encoding the polypeptide, the vector comprising the nucleic acid molecule, the recombinant viral particle comprising the vector and capsid protein, the cell comprising the vector, the cell transformed with the vector, or any combination thereof.

In some embodiments, such methods comprise synthesizing, under suitable conditions, a TAFA protein fragment or variant thereof as described herein.

In some embodiments, such methods comprise culturing, under suitable conditions, cells described herein (for example, cells transduced with a polynucleotide comprising a transgene and an untranslated nucleic acid molecule) and recovering the encoded protein. In certain embodiments, a method of producing a polypeptide encoded by a transgene comprises administering to a subject in need thereof a polynucleotide of the present disclosure (for example, comprising a transgene and an untranslated nucleic acid molecule), such that the encoded polypeptide is produced in the subject. Additional disclosure relating to such in vivo methods of producing a polypeptide is provided elsewhere in the present disclosure (see, for example, the therapeutic uses below).

In some embodiments, the present disclosure provides methods of producing recombinant adeno-associated virus (rAAV) particles comprising a polynucleotide described herein (for example, comprising a transgene and an untranslated nucleic acid sequence). In some embodiments, a method of producing such recombinant AAV comprises culturing a cell transfected with an AAV vector described herein under conditions suitable for producing recombinant AAV. In some embodiments, the method further comprises a step of isolating the produced recombinant viral particle.

In some embodiments, the present disclosure provides a recombinant viral particle produced by the method.

In some embodiments, the recombinant viral particle can be produced by transfecting a cell with (i) an AAV vector comprising the transgene (for example, a nucleic acid molecule encoding a TAFA polypeptide or a polypeptide comprising any one of the amino acid sequences of SEQ ID NOS: 142 to 147) and (ii) a construct comprising rep and cap genes. Additionally, (iii) a helper construct for transducing a transgene into a host cell may be used. In such embodiments, the helper construct may comprise the E2A gene, which promotes AAV genome replication and transcription of AAV genes, the E4 gene, which allows AAV mRNA to move from the nucleus to the cytoplasm, and a VA region that produces two VA RNAs involved in translational control.

In some embodiments, the above three constructs can be replaced by two constructs for transducing host cells. In such embodiments, an AAV construct comprises the transgene and the untranslated nucleic acid sequence, and a separate construct comprises the rep and cap genes, the E2A gene, the E4 gene, and the VA region. Additional methods for producing AAV particles are generally known in the art and are described, for example, in Clement et al., Mol Ther Methods Clin Dev 3:16002 (2016), Clark, Kidney Int. 61:S9-15 (2002), and Xiao et al., J Virol 72(3):2224-32 (1998), each of which is incorporated by reference herein in its entirety.

The present disclosure also provides recombinant viral particles comprising (a) a capsid protein and (b) the AAV vector.

### IX.B. Therapeutic uses

The polypeptides described herein (for example, TAFA polypeptides or polypeptides comprising any one of the amino acid sequences of SEQ ID NOS: 142 to 147), nucleic acids (for example, comprising a transgene and an untranslated nucleic acid sequence), vectors harboring such nucleic acids and recombinant viruses (for example, rAAV), cells comprising the nucleic acids or vectors, and methods described herein have numerous in vitro and in vivo utilities. For example, the polypeptides, polynucleotides, vectors (for example, AAV vectors), described herein can be administered to cells in culture (in vitro or ex vivo) or to human subjects (in vivo) to prevent or treat disease. Accordingly, in some embodiments, the present disclosure provides the therapeutic use of any of the polypeptides, polynucleotides (for example, comprising a transgene and an untranslated nucleic acid sequence), recombinant expression constructs or vectors, cells, pharmaceutical compositions, or recombinant viruses described herein. In some embodiments, the present disclosure provides a method of expressing a transgene in a subject in need thereof, comprising administering to the subject a polynucleotide as disclosed herein (for example, comprising a transgene and an untranslated nucleic acid sequence), a vector as disclosed herein, a recombinant virus (for example, rAAV) as disclosed herein, a cell as disclosed herein, or a pharmaceutical composition as disclosed herein, wherein expression of the transgene is increased in the subject after administration.

As described herein, an untranslated nucleic acid sequence of the present disclosure can increase expression of the transgene when the transgene is translated. Accordingly, in some embodiments, the present disclosure provides methods of increasing expression of a transgene in a cell, comprising contacting the cell with any of the polynucleotides (for example, nucleic acid molecules encoding a TAFA polypeptide or a polypeptide comprising any one of the amino acid sequences of SEQ ID NOS: 142 to 147), vectors, or recombinant viruses (for example, rAAV) described herein. The contacting can be carried out ex vivo or in vivo. When the contacting is performed in vivo, the method may further comprise administering to the subject, before contacting, any of the polynucleotides, vectors, or recombinant viruses described herein.

In some embodiments, after such contacting, expression of the transgene (for example, a nucleic acid molecule encoding a TAFA polypeptide or a polypeptide comprising any one of the amino acid sequences of SEQ ID NOS: 142 to 147) is increased by at least about 1-fold, at least about 1.1-fold, at least about 1.2-fold, at least about 1.3-fold, at least about 1.4-fold, at least about 1.5-fold, at least about 1.6-fold, at least about 1.7-fold, at least about 1.8-fold, at least about 1.9-fold, at least about 2-fold, at least about 2.5-fold, at least about 3-fold, at least about 3.5-fold, at least about 4-fold, at least about 5-fold, at least about 6-fold, at least about 7-fold, at least about 8-fold, at least about 9-fold, or at least about 10-fold compared to a baseline level. In some embodiments, the baseline expression is expression of the transgene in the cell prior to contacting. In some embodiments, the baseline expression is expression of the transgene in a corresponding cell that has not been contacted with the polypeptide, polynucleotide, vector, or recombinant virus described herein.

Another aspect of the present disclosure provides methods for preventing or treating a disease in a subject in need thereof, comprising administering to the subject an effective amount of any of the polypeptides (for example, TAFA polypeptides or polypeptides comprising any one of the amino acid sequences of SEQ ID NOS:142 to 147), polynucleotides (for example, nucleic acid molecules encoding the TAFA polypeptides or polypeptides comprising any one of the amino acid sequences of SEQ ID NOS:142 to 147), vectors, cells, recombinant viruses, or pharmaceutical compositions described herein. As is apparent from the present disclosure, the compositions described herein (for example, polypeptides, polynucleotides, recombinant expression constructs, cells, pharmaceutical compositions, or recombinant viruses) can be used to prevent or treat any disease of interest.

In some embodiments, the method further comprises administering an additional therapeutic agent (for example, an inhibitor of vascular endothelial growth factor (VEGF) or a therapeutic agent for neuropathic pain) to the subject. In some embodiments, the additional therapeutic agent may be administered before, concurrently with, or after administration of the polypeptide, polynucleotide, vector, cell, recombinant virus, or pharmaceutical composition.

Diseases that can be prevented, ameliorated, or treated by the present disclosure are not limited, and include any disease for which it is desirable to reduce the frequency of drug administration. Non-limiting examples of such diseases include retinal neurodegenerative diseases. In some aspects, the retinal neurodegenerative disease is selected from retinopathy, choroidal neovascularization, maculopathy, macular degeneration, retinal degeneration, macular edema, retinal edema, macular swelling, retinal swelling, color vision abnormality, retinal cell degeneration, retinal vascular occlusion, retinal detachment, inherited retinal disease, and combinations thereof.

In some aspects, the retinal neurodegenerative disease that can be prevented or treated by the present disclosure includes macular degeneration. In some aspects, the macular degeneration includes age-related macular degeneration (AMD). Age-related macular degeneration can be divided into dry (atrophic) macular degeneration and wet (neovascular or exudative) macular degeneration. Age-related macular degeneration can also be classified into early AMD, intermediate AMD, and late or advanced AMD (geographic atrophy). In some aspects, the retinal or macular disease that can be prevented or treated by the present disclosure includes diabetic retinopathy. In some aspects, the diabetic retinopathy is non-proliferative diabetic retinopathy (NPDR). In some aspects, the diabetic retinopathy is proliferative diabetic retinopathy (PDR). In some aspects, the diabetic retinopathy is diabetic maculopathy. In some aspects, the diabetic retinopathy is diabetic macular edema. In some aspects, the diabetic retinopathy is any retinopathy associated with ischemic damage within the retina. Unless otherwise specified, the present disclosure can be used to prevent or treat all forms of AMD and/or diabetic retinopathy.

Additional non-limiting examples of diseases include neuropathic pain.

In some embodiments, the neuropathic pain is central neuropathic pain, that is, pain resulting from injury or damage affecting all levels of the central nervous system, including the central somatosensory nervous system (for example, brain injury or spinal cord injury), or pain caused by, or associated with, diseases or disorders such as seizures, multiple sclerosis, or lateral medullary infarction. In some embodiments, central neuropathic pain may be spontaneous or stimulus-evoked. In some embodiments, central neuropathic pain may include mechanical allodynia and cold allodynia. Symptoms of central neuropathic pain typically include, for example, burning, aching, shooting pain, pressure, painful cold, paresthesia, and dysesthesia (for example, tingling, pins-and-needles pain, cold sensation, and a feeling of pressure). The distribution of central neuropathic pain may include, for example, areas ranging from a small region in a peripheral territory to a wide region, or areas that, in the case of spinal cord injury or stroke, cover half of the body, include one side of the face, or involve the side of the body or limb contralateral to the lesion. Central neuropathic pain due to spinal cord injury includes "at-level pain," perceived in a segmental pattern at the level of injury, and "below-level pain," perceived below the level of injury. In some embodiments, the methods described herein reduce, reverse, relieve, ameliorate, inhibit, attenuate, or prevent central neuropathic pain, pain-related symptoms, the underlying cause of the pain, or any combination thereof.

In some embodiments, the neuropathic pain is peripheral neuropathic pain, that is, pain caused by, or associated with, damage or injury affecting any part of the peripheral nervous system (for example, damage to motor nerves, sensory nerves, autonomic nerves, or any combination thereof) or pain caused by, or associated with, a disease or disorder. Damage or injury to motor nerves is associated with symptoms such as muscle weakness (for example, weakness of the back, leg, hip, or facial muscles), painful cramps, fasciculations (uncontrolled muscle twitching under the skin), muscle atrophy (marked reduction in muscle mass), and decreased reflexes. Damage or injury to sensory nerves causes various symptoms, including pain and hypersensitivity of pain receptors in the skin, resulting in allodynia (for example, severe pain induced by normally non-painful stimuli).

In some embodiments, the methods of the present invention treat one or more types of neuropathic pain by administering to a subject in need thereof the polypeptide, a nucleic acid molecule encoding the polypeptide, a vector comprising the nucleic acid molecule, a recombinant viral particle comprising the vector and capsid protein, a cell comprising the vector, a cell transformed with the vector, or any combination thereof. In some embodiments, the neuropathic pain treatable by the methods of the present invention is neuralgia, which includes, without limitation, trigeminal neuralgia (TN) (for example, pain in the facial or oral trigeminal nerve territory), atypical trigeminal neuralgia (ATN), occipital neuralgia, postherpetic neuralgia (for example, unilateral pain distributed in one or more dermatomes or in the ophthalmic division of the trigeminal nerve), peripheral nerve injury pain (for example, pain in the innervation territory of a lesioned nerve, typically distal pain in the innervation territory of an injured nerve in response to trauma, surgery, or compression), glossopharyngeal neuralgia (for example, severe pain in the throat, tongue, and retroauricular region caused by irritation of the ninth cranial nerve), sciatica, low back pain, and atypical facial pain. In some embodiments, the neuralgia is caused by, or associated with, chemical irritation, inflammation, trauma (including surgery), compression of a nerve by an adjacent structure (for example, a tumor), infection, or a combination thereof. In some embodiments, the neuropathic pain is deafferentation pain syndrome, which includes, without limitation, brain or spinal cord injury, post-stroke pain, phantom limb pain, paraplegia, brachial plexus avulsion injury, and lumbar radiculopathy. In some embodiments, the neuropathic pain is complex regional pain syndrome (CRPS) including CRPS1 and CRPS2, without limitation. In some embodiments, symptoms associated with CRPS include severe pain, changes in nails, bones, and skin, and increased sensitivity to touch in the affected limb. In some embodiments, the neuropathic pain is a neuropathy (for example, central or peripheral).

Non-limiting examples of neuropathic pain include mononeuropathic pain (mononeuropathy) and polyneuropathic pain (polyneuropathy).

In some embodiments, the neuropathic pain arises from, or is associated with, physical injury including, for example: (1) traumatic injury or damage including nerve compression (for example, nerve crush, nerve stretch, nerve entrapment, or partial nerve transection); (2) spinal cord injury (for example, hemisection of the spinal cord); (3) damage or injury to peripheral nerves (for example, motor, sensory, or autonomic nerves, or any combination thereof); (4) limb amputation; contusion; inflammation (for example, inflammation of the spinal cord); or surgical procedures; and (5) repetitive stress, for example, repetitive, slow, and/or forceful activities requiring prolonged movement of a joint group (for example, ulnar neuropathy and carpal tunnel syndrome). In some embodiments, the methods treat neuropathic pain caused by, or associated with, exposure to toxic agents.

In some embodiments, the neuropathic pain arises from, or is associated with, one or more diseases or disorders including, for example: (1) ischemic events (for example, stroke or myocardial infarction); (2) multiple sclerosis; (3) metabolic and/or endocrine diseases or disorders (for example, diabetes, metabolic disease, and acromegaly, which is caused by excessive production of growth hormone and characterized by abnormal enlargement of skeletal parts including joints, leading to nerve entrapment and pain); (4) small vessel disease that reduces oxygen supply to peripheral nerves and causes nerve tissue damage (for example, vasculitis, that is, inflammation of blood vessels); (5) autoimmune diseases (for example, Sjögren's syndrome, lupus, rheumatoid arthritis, and acute inflammatory demyelinating polyneuropathy, also known as Guillain-Barré syndrome); (6) renal impairment; (7) cancer or tumors (for example, neoplastic tumors, neuromas, paraneoplastic syndromes, and toxicity from chemotherapeutic agents and radiation therapy for cancer); (8) infections (for example, herpes varicella-zoster (shingles), Epstein-Barr virus, West Nile virus, cytomegalovirus, and herpes simplex virus, infection by viruses such as HIV/AIDS, or infection by bacteria such as those causing Lyme disease and diphtheria, and by Mycobacterium leprae causing leprosy); (9) inflammatory disorders; (10) peripheral nerve disorders (for example, neuroma); (11) genetic or de novo genetic disorders (for example, Charcot-Marie-Tooth disease); (12) mononeuropathy; (13) polyneuropathy; or combinations thereof. In some embodiments, the neuropathic pain is caused by, or associated with, diabetes (type I or type II). In some embodiments, the neuropathic pain is diabetic peripheral neuropathy.

In some embodiments, the neuropathic pain arises from, or is associated with, exposure to infectious agents such as tick-borne infections, herpes varicella-zoster, Epstein-Barr virus, West Nile virus, cytomegalovirus, and herpes simplex virus, HIV/AIDS, or exposure to toxic substances such as drugs, alcohol, heavy metals (for example, lead, arsenic, mercury), industrial substances (for example, fumes from solvents or adhesives), and nitrous oxide.

In some embodiments, the neuropathic pain arises from, or is associated with, physical trauma, infection, diabetes, cancer therapy, alcoholism, amputation, multiple sclerosis, shingles, spinal surgery, sciatica (pain along the sciatic nerve), low back pain, neuralgia such as trigeminal neuralgia (for example, pain in the facial or oral trigeminal nerve territory), neuropathic pain such as painful polyneuropathy (for example, foot pain that can extend to the lower leg, thigh, and hands), or combinations thereof. In some embodiments, the neuropathic pain is trigeminal neuralgia. In some embodiments, the neuropathic pain is associated with muscle weakness of the back, leg, hip, or face. In some embodiments, the neuropathic pain is caused by compression of nerves, for example, nerves in the leg, foot, or hip, or nerves of the facial muscles. In some embodiments, the neuropathic pain involves sciatic nerve injury. In some embodiments, the neuropathic pain is sciatica.

In some embodiments, the methods of the present invention can reverse, relieve, ameliorate, inhibit, attenuate, or prevent one or more symptoms associated with neuropathic pain. Accordingly, in one aspect, the present invention provides a method for ameliorating hyperalgesia, comprising administering to a subject in need thereof the polypeptide, a nucleic acid molecule encoding the polypeptide, a vector comprising the nucleic acid molecule, a recombinant viral particle comprising the vector and capsid protein, a cell comprising the vector, a cell transformed with the vector, or any combination thereof. As used herein, the term "hyperalgesia" refers to an increased or exaggerated response to a painful stimulus (for example, pinprick or a hot plate). In some embodiments, hyperalgesia relates to mechanical stimuli such as pinprick (mechanical hyperalgesia). In other embodiments, hyperalgesia relates to thermal stimuli such as a hot plate (thermal hyperalgesia). In some embodiments, the subject in need thereof has a chronic constriction injury (for example, sciatica). In some embodiments, the subject in need thereof has diabetic peripheral neuropathy.

In some embodiments, when the polypeptide (for example, a TAFA protein; a polypeptide comprising an amino acid sequence of a TAFA protein fragment or variant thereof), the nucleic acid molecule encoding the polypeptide, the vector comprising the nucleic acid molecule, the recombinant viral particle comprising the vector and capsid protein, the cell comprising the vector, the cell transformed with the vector, or any combination thereof is administered to a subject in need thereof (for example, a subject with neuropathic pain who has not been treated), the subject can exhibit a higher threshold to mechanical stimulation compared to a reference control. As used herein, the term "threshold to mechanical stimulation" refers to the amount of pressure from a mechanical stimulus (for example, a pulling force) required before the subject responds. Thus, a subject with a higher threshold can endure or tolerate a substantially greater amount of mechanical stimulation than a subject with a lower threshold. In some embodiments, the methods of the present invention increase the subject's threshold to mechanical stimulation by at least 5%, at least 10%, at least 20%, at least 30%, at least 40%, at least 50%, at least 60%, at least 70%, at least 80%, at least 90%, at least 100%, at least 150%, or at least 200% compared to a reference control (for example, the subject's threshold before administration).

In some embodiments, when the polypeptide, the nucleic acid molecule encoding the polypeptide, the vector comprising the nucleic acid molecule, the recombinant viral particle comprising the vector and capsid protein, the cell comprising the vector, the cell transformed with the vector, or any combination thereof is administered to a subject in need thereof, the latency to thermal stimulation (for example, a hot plate), that is, the time interval between stimulus and response, is increased compared to a reference control (a subject with neuropathic pain who has not received the treatment). In some embodiments, the methods of the present invention increase the subject's latency to thermal stimulation by at least 5%, at least 10%, at least 20%, at least 30%, at least 40%, at least 50%, at least 60%, at least 70%, at least 80%, at least 90%, at least 100%, at least 150%, or at least 200% compared to a reference control (for example, the subject's latency before administration).

In another aspect, the present invention provides a method for ameliorating sensory nerve conduction velocity in a subject in need thereof. As used herein, the term "sensory nerve conduction velocity" (SNCV) refers to the speed at which electrical signals travel through a peripheral nerve. Healthy nerves transmit electrical signals more rapidly and robustly than damaged nerves (see, for example, Chouhan S., J Clin Diagn Res 10(1):CC01-3 (2016)). Thus, tests that measure SNCV (for example, sensory nerve conduction velocity tests) can be useful for identifying potential nerve damage and/or dysfunction in a subject. In some embodiments, the methods of the present invention increase SNCV in a subject with neuropathic pain by at least 5%, at least 10%, at least 20%, at least 30%, at least 40%, at least 50%, at least 60%, at least 70%, at least 80%, at least 90%, at least 100%, at least 150%, or at least 200% compared to a reference control (for example, the subject's SNCV before administration).

Animal models for studying neuropathic pain are available. Non-limiting examples of such animal models include: (1) the spinal nerve ligation (SNL) model, in which one or more spinal nerves running to the paw are ligated and transected (see Kim SH and Chung JM., Pain 50:355-363 (1992)); (2) the partial sciatic ligation (PSL) model, in which a portion of the sciatic nerve is tightly ligated (see Seltzer et al., Pain 43:205-218 (1990)); (3) the chronic constriction injury (CCI) model, in which four loose ligatures made of chromic gut are placed around the sciatic nerve, and the immune response to the sutures induces nerve swelling and constriction; (4) the spared nerve injury (SNI) model, in which the common peroneal and tibial nerves are transected while leaving the sural nerve intact (see Devor I. and Woolf C.J., Pain 87:149-158 (2000)); and (5) STZ-induced diabetic rats, in which streptozotocin (STZ) injection induces pancreatic edema and degeneration of beta cells in the islets of Langerhans and thereby experimental diabetes in rats (see, for example, Akbarzadeh A. et al., Indian J. Clin. Biochem. 22(2):60-64 (2007)). These models induce hyperalgesia in animals, which is manifested by enhanced responses to mechanical and/or thermal stimuli.

Mechanical hyperalgesia in animals can be assessed using the Von Frey test, in which a series of Von Frey monofilaments of different bending forces are applied to the plantar surface of the paw. The paw withdrawal threshold decreases sharply after nerve injury (see Li et al., Pain 85:493-502 (2000)). Accordingly, in some embodiments, the methods of the present invention increase the paw withdrawal threshold in an animal model of neuropathic pain (for example, a chronic constriction injury model).

Tests for thermal hyperalgesia can include the use of a radiant heat source (for example, a hot plate) focused on the plantar surface, and the response time to paw withdrawal is measured. After nerve injury, paw withdrawal occurs more rapidly than before injury (see Kim SH and Chung JM., Pain 50:355-363 (1992)). In some embodiments, the methods of the present invention increase the paw withdrawal latency in an animal model of neuropathic pain (for example, a diabetic peripheral neuropathy model).

Another aspect of the present disclosure provides a gene therapy or method for preventing or treating a disease that can achieve sustained expression of a transgene.

Using the viral delivery systems described herein, administration of the compositions of the present disclosure can be carried out at intervals of about 1 week, about 2 weeks, about 3 weeks, about 1 month, about 2 months, about 3 months, about 4 months, about 5 months, about 6 months, about 7 months, about 8 months, about 9 months, about 10 months, about 11 months, about 1 year, about 2 years, about 3 years, about 4 years, about 5 years, or about 10 years or more. In some embodiments, the interval is about 2 to about 3 months. In some embodiments, the interval is about 6 months. In some embodiments, the interval is about 1 year. In some embodiments, the interval is at least about 1 year. In some embodiments, the interval is at least about 2 years. In some embodiments, the interval is at least about 3 years. In some embodiments, the interval is at least about 4 years. In some embodiments, the interval is at least about 5 years. In some embodiments, the interval is at least about 10 years. That is, by using the viral delivery systems described herein, the frequency of administration of the compositions can be dramatically reduced, thereby sparing physicians, patients, or subjects the inconvenience associated with repeated dosing. Depending on the patient's symptoms or needs, the compositions may initially be administered 2-3 times at intervals of 1-2 weeks, and then once every 2-3 months, every 6 months, or every year or more, or once every 2-10 years or more.

### [Effects of the Invention]

The features and advantages of the present disclosure can be summarized as follows:
(i) The invention provides a polypeptide (for example, a polypeptide comprising the amino acid sequence of a TAFA protein, a TAFA protein fragment, or a variant thereof) that has the ability to increase neurite length and/or the number of branch points.
(ii) The invention also provides therapeutic uses of a pharmaceutical composition comprising the polypeptide, a nucleic acid molecule encoding the polypeptide, a vector comprising the nucleic acid molecule, a recombinant viral particle comprising the vector and a capsid protein, a cell comprising the vector, a cell transformed with the vector, or any combination thereof.
(iii) By restoring damaged nerves, the composition of the present disclosure can be usefully employed for the prevention or treatment of retinal neurodegenerative diseases and neuropathic pain.

### BRIEF DESCRIPTION OF THE DRAWINGS

Fig. 1A shows the analysis of changes in neurite length induced by full-length TAFA4 protein in differentiated neuronal cells derived from the mouse dorsal root ganglion progenitor cell line MED17.11, and Fig. 1B shows the analysis of changes in the number of branch points induced by full-length TAFA4 protein in the same neuronal cells. An asterisk (*) indicates p value < 0.05.
Fig. 2 shows the analysis, by fluorescence angiography (FA), of the efficacy in ameliorating retinal damage in a NaIO₃-induced retinal injury model following subretinal administration of either control AAV8 (control group) or AAV8.mouse TAFA4 (test group). OD (oculus dexter) denotes the right eye, and OS (oculus sinister) denotes the left eye.
Fig. 3 shows the measurement of changes in A-wave and B-wave amplitudes of the electroretinogram (ERG) between the control group (control AAV8) and the test group (AAV8.mouse TAFA4).
Fig. 4 shows a comparison of interspecies sequence identity of full-length TAFA4 protein.
Fig. 5 shows a comparison of interspecies sequence identity of mature TAFA4 protein.
Fig. 6 shows the analysis, by fluorescence angiography (FA), of the efficacy in ameliorating retinal damage in a NaIO₃-induced retinal injury model following subretinal administration of either control AAV8 (control group) or test groups (AAV8.human TAFA4, AAV8.gecko TAFA4, or AAV8.fish TAFA4). OD (oculus dexter) denotes the right eye, and OS (oculus sinister) denotes the left eye.
Fig. 7 shows the measurement of changes in A-wave and B-wave amplitudes of the electroretinogram (ERG) between the control group (control AAV8) and the test groups (AAV8.human TAFA4, AAV8.gecko TAFA4, and AAV8.fish TAFA4).
Fig. 8 shows the analysis, by fluorescence angiography (FA), of the efficacy in ameliorating retinal damage in a NaIO₃-induced retinal injury model following subretinal administration of either control AAV8 (control group) or test groups (AAV8.TAFA1, AAV8.TAFA2, AAV8.TAFA3, or AAV8.TAFA4). OD (oculus dexter) denotes the right eye, and OS (oculus sinister) denotes the left eye.
Fig. 9 shows the measurement of changes in A-wave and B-wave amplitudes of the electroretinogram (ERG) between the control group (control AAV8) and the test groups (AAV8.TAFA1, AAV8.TAFA2, AAV8.TAFA3, and AAV8.TAFA4).
Figs. 10A to 10D show comparison of interspecies amino acid sequence identity of full-length TAFA1, TAFA2, TAFA3, and TAFA4. Fig. 10A compares amino acid residues 1-70, Fig. 10B compares amino acid residues 71-140, Fig. 10C compares amino acid residues 141-210, and Fig. 10D compares amino acid residues 211-244.
Fig. 11A shows the analysis of changes in neurite length induced by TAFA4 peptide fragments (TAFA4 fragments 1-3, F1-F3) in differentiated neuronal cells derived from the MED17.11 cell line, and Fig. 11B shows the analysis of changes in the number of branch points induced by TAFA4 peptide fragments (F1-F3) in the same cells.
Fig. 12 shows the sequences of TAFA4-derived peptides (TAFA protein and its fragments F1-F8) according to the embodiments of the present invention.
Fig. 13A shows the analysis of changes in neurite length induced by TAFA4 peptide fragments (TAFA4 fragments 4-8, F4-F8) in differentiated neuronal cells derived from the MED17.11 cell line, and Fig. 13B shows the analysis of changes in the number of branch points induced by TAFA4 peptide fragments (F4-F8) in the same cells. Double asterisks (**) indicate p value < 0.01.
Fig. 14 shows the analysis of analgesic effects of TAFA4 and TAFA4 fragments 1, 2, 3, 5, 6, and 7 (F1, F2, F3, F5, F6, and F7) in a neuropathic pain model.

### DETAILED DESCRIPTION OF THE INVENTION

Hereinafter, the present invention will be described in further detail with reference to examples. These examples are provided solely for the purpose of more specifically illustrating the present invention, and it will be apparent to those of ordinary skill in the art that the scope of the present invention is not limited by these examples in light of the spirit of the invention.

### Examples

### Example 1. Evaluation of the efficacy of TAFA4 on neuronal cells

To evaluate the biological efficacy of TAFA4 on neuronal cells, the mouse dorsal root ganglion progenitor cell line MED17.11 was differentiated into neuronal cells, and changes in neurite length and the number of branch points induced by recombinant human TAFA4 (hereinafter "rhTAFA4") were assessed.

The MED17.11 cell line was cultured in a 33°C incubator (5% CO₂), and undifferentiated cells were maintained in DMEM/F-12 medium (Gibco) supplemented with 10% fetal bovine serum (Gibco), 1% penicillin/streptomycin (Gibco), 5 ng/mL interferon-γ (R&D Systems), and 0.5% chicken embryonic extract (Sera Lab). The method for evaluating the efficacy of TAFA4 was as follows. Undifferentiated MED17.11 cells were harvested using 0.25% trypsin-EDTA (Gibco). After centrifugation, the supernatant was removed, and the cell pellet was resuspended in differentiation-inducing medium. The differentiation-inducing medium consisted of DMEM/F-12 supplemented with 10% fetal bovine serum (Gibco), 1% penicillin/streptomycin (Gibco), 10 ng/mL fibroblast growth factor 2 (R&D Systems), 0.5 mM dibutyryl cAMP (Sigma), 25 µM forskolin (Sigma), 5 µg/mL Y-27632 (Chemdea), 100 ng/mL beta nerve growth factor (R&D Systems), and 10 ng/mL glial-derived neurotrophic factor (R&D Systems). MED17.11 cells resuspended in differentiation-inducing medium were dispensed into a 96-well cell culture plate(Thermo Fisher) at 1 × 10⁴ cells/mL, 100 µL per well. The test group was treated with 1 µM rhTAFA4 (R&D Systems, Table 1), and the control group (CTL) was treated with an equal volume of phosphate-buffered saline (PBS) (Gibco), followed by incubation at 37°C in an incubator (5% CO₂). During incubation, images of the cells were acquired using an Incucyte system (Sartorius). Neurite length and the number of branch points in MED17.11 cells were analyzed from the acquired images using the Incucyte software.

Three independent experiments were performed in total, and statistical significance was analyzed by Student's *t*-test.

**[Table 1]**

| Amino acid sequence of recombinant human TAFA4 peptide | | |
|---|---|---|
| Name of peptide | Length (a.a.) | Amino acid sequence (Sequence, N'→C') |
| Recombinant human TAFA4 (SEQ ID NO: 86) | 105 | SQHLRGHAGH HQIKQGTCEV VAVHRCCNKN RIEERSQTVK CSCFPGQVAG TTRAQPSCVE ASIVIQKWWC HMNPCLEGED CKVLPDYSGW SCSSGNKVKT TKVTR |

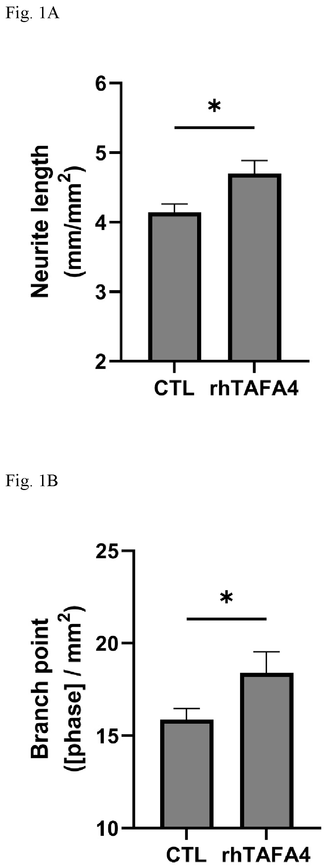

At 4 days after treatment, neurite length and the number of branch points of MED17.11 cells were found to be significantly increased in the rhTAFA4-treated group compared with the control group (CTL) (Fig. 1A and Fig. 1B).

### Example 2. Evaluation of the efficacy of TAFA in a retinal injury model

### 2-A. Production of AAVs carrying TAFA1-4 and interspecies variant genes

Recombinant AAVs comprising a transgene were produced according to the method described in Korean Patent Application No. 10-2023-0068976, and genes encoding TAFA1-4 and their interspecies variants were inserted as the transgenes.

### 2-B. Evaluation of the efficacy of TAFA4 in an NaIO₃ (sodium iodate)-induced retinal injury model

NaIO₃ (sodium iodate) has been reported to induce direct damage to retinal neurons and reduce the area of neurites and retinal ganglion cells (see Zui Tao et al., Molecular Neurobiology, 2013 Feb;47 1 241-60).

As confirmed in Example 1 above, TAFA4 significantly increased neurite length and the number of branch points compared with the control. To evaluate whether TAFA4 ameliorates retinal neurodegenerative disease in an in vivo model, fluorescence angiography (FA) and electroretinogram (ERG) changes following administration of AAV8.mouse TAFA4 were evaluated in a mouse model of NaIO₃-induced retinal injury.

Control AAV8 without a transgene or AAV8.mouse TAFA4 (CAT311 promoter-mTAFA4) was administered via subretinal injection (SRI) to C57BL/6 mice (Orient Bio).

To allow sufficient expression of mouse TAFA4, 5 × 10⁸ vg of AAV8 was administered by subretinal injection (SRI) into both eyes of each mouse, and the mice were maintained for 56 days. NaIO₃ was then administered via tail vein injection at a dose of 20 mg/kg to induce an AMD model. At 10 days after model induction (day 66 after SRI), a fluorescent contrast agent was injected through the tail vein. Images were focused on the fundus using a Micron-IV imaging camera (Phoenix), and fluorescence angiography (FA) images were acquired. At 11 days after model induction (day 67 after SRI), scotopic ERG was performed. Mice were placed on the ERG stage, and ERG probes were applied to the tail, head, and cornea to measure A-wave and B-wave amplitudes. ERG data were analyzed using the LabScribeERG (iWorx Data Acquisition Software) program.

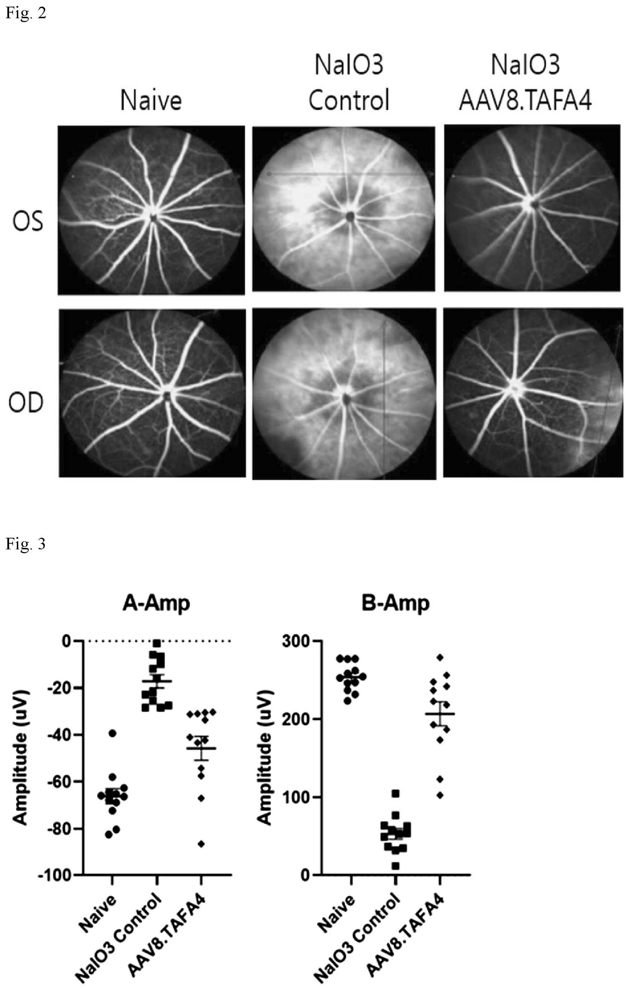

In FA images acquired at 10 days after model induction, leakage of fluorescent contrast agent due to outer retinal damage was observed in the retinas of NaIO₃-treated mice group (control AAV8-treated) compared with healthy group (naïve, mice control AAV8-treated) in which AMD was not induced. In contrast, the group administered AAV8.mouse TAFA4 (test group) showed retinal findings that were almost normal and similar to those of the naïve group (Fig. 2). Consistent with the FA findings, scotopic ERG evaluated on day 11 after model induction showed retinal abnormalities induced by NaIO₃, with marked reductions in the evoked A-wave and B-wave amplitudes. In contrast, in the group administered AAV8.mouse TAFA4 (test group), both A-wave and B-wave amplitudes were restored, and in particular, the B-wave amplitude recovered to a level close to normal (Fig. 3).

### 2-C. Evaluation of the efficacy of interspecies TAFA4 variants in a NaIO₃ (sodium iodate)-induced retinal injury model

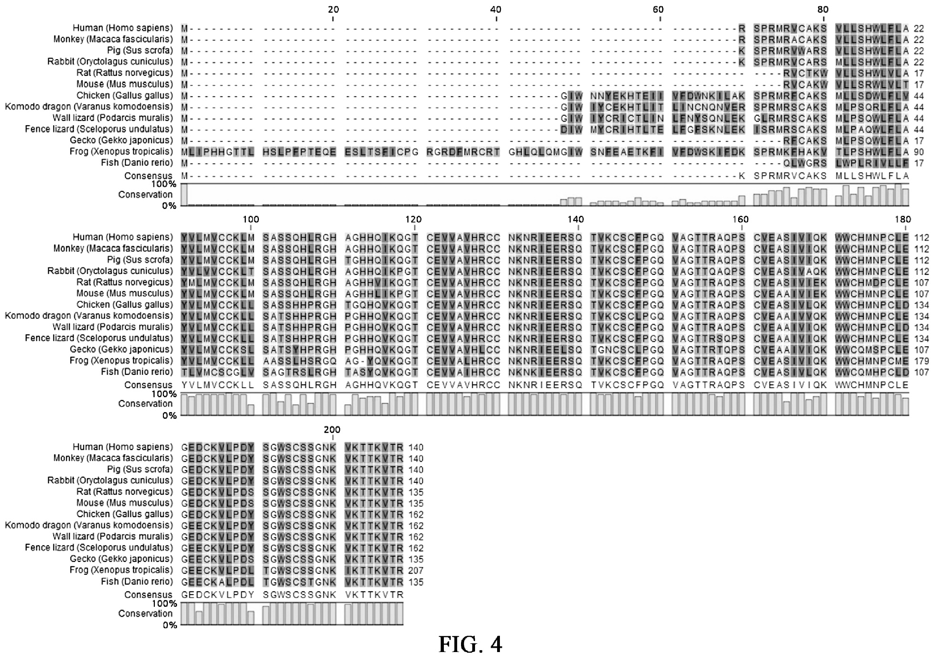

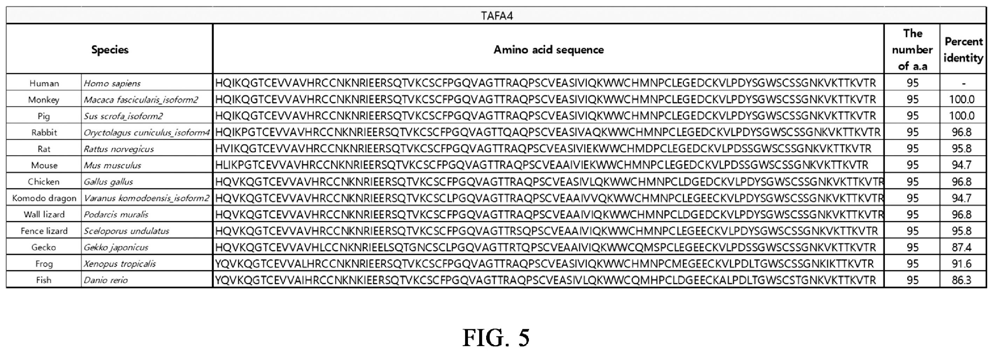

To determine whether interspecies variants of TAFA4 also exhibit similar efficacy, the sequence identity of full-length TAFA4 proteins from mammals (human, monkey, pig, rabbit, rat, mouse), birds (chicken), reptiles (komodo dragon, wall lizard, fence lizard, gecko), amphibians (frog), and fish was compared, and the consensus sequence among species was identified (Fig. 4). The amino acid sequence of mature human TAFA4 protein (95 a.a.) showed more than 90% sequence identity with that of mammals and amphibians, more than 95% with that of birds, and more than 85% with that of reptiles and fish, confirming very high sequence identity across species (Fig. 5).

Since the neuroprotective effect of AAV8.mouse TAFA4 against retinal injury was confirmed in the NaIO₃-induced retinal damage model, additional experiments were conducted to determine whether TAFA4 from other species also has retinal protective effects. Specifically, we evaluated the efficacy of human TAFA4 and TAFA4 from reptile (gecko TAFA4; sequence identity 87.4%) and fish (fish TAFA4; sequence identity 86.3%), which exhibit the lowest sequence identity with human TAFA4. In a mouse model of AMD induced by NaIO₃ administration, changes in fluorescence angiography (FA) and electroretinogram (ERG) following administration of AAV8.human TAFA4, AAV8.gecko TAFA4, and AAV8.fish TAFA4 were evaluated.

C57BL/6 mice (Orient Bio) were administered either control AAV8 or each species-derived AAV8.TAFA4 by subretinal injection (SRI).

To allow sufficient expression of TAFA4, 1×10⁹ vg of AAV8 was injected subretinally into both eyes of the mice, and after waiting up to 42 days, NaIO₃ was administered intravenously at a dose of 20 mg/kg to induce the AMD model. On day 9 after model induction (day 51 after SRI), fluorescein dye was injected via the tail vein. Thereafter, the fundus was brought into focus using a Micron-IV imaging camera (Phoenix), and FA images were acquired. On day 7 after model induction (day 49 after SRI), scotopic ERG was performed. Mice were placed on the ERG stage, and ERG probes were placed in contact with the tail, head, and cornea, after which A-wave and B-wave amplitudes were measured. ERG analysis was performed using the LabScribeERG (iWorx Data Acquisition Software) program.

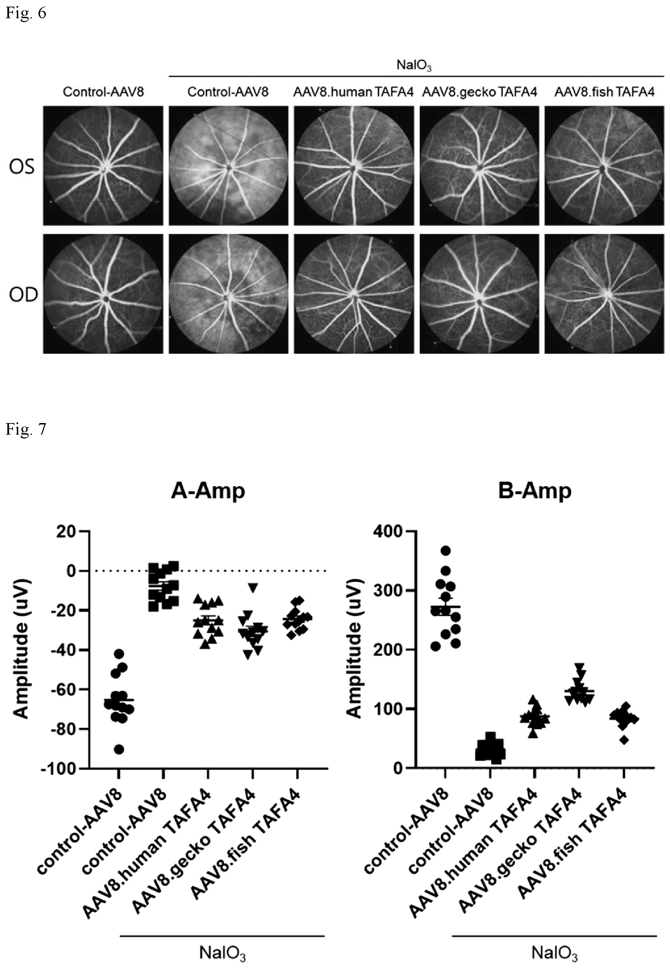

On FA images taken on day 9 after model induction, leakage of fluorescein dye due to outer retinal damage was observed in the NaIO₃-treated group (control group, control AAV8-treated) compared with healthy animals (naïve, control AAV8-treated). In contrast, the groups administered AAV8.human TAFA4, AAV8.gecko TAFA4, or AAV8.fish TAFA4 (test groups) showed an almost normal retinal appearance similar to that of the naïve group (Fig. 6). Likewise, scotopic ERG evaluated on day 11 after model induction showed marked reductions in the evoked A-wave and B-wave amplitudes due to NaIO₃-induced retinal abnormalities. In contrast, in the groups administered AAV8.human TAFA4, AAV8.gecko TAFA4, or AAV8.fish TAFA4 (test groups), NaIO₃-induced changes in A-wave and B-wave amplitudes were restored (Fig. 7).

### 2-D. Retinal protective effects of TAFA4 paralogs according to sequence identity in a NaIO₃ (sodium iodate)-induced retinal injury model

High sequence identity between TAFA4 and other TAFA family proteins (TAFA1-3) has been reported. Therefore, we examined whether human TAFA1, TAFA2, and TAFA3 also exhibit retinal protective effects in a mouse model of NaIO₃-induced retinal damage. In a mouse AMD model induced by NaIO₃, changes in FA and ERG following administration of AAV8.TAFA1, AAV8.TAFA2, AAV8.TAFA3, and AAV8.TAFA4 were evaluated.

C57BL/6 mice (Orient Bio) were administered control AAV8, AAV8.TAFA1, AAV8.TAFA2, AAV8.TAFA3, or AAV8.TAFA4 via subretinal injection (SRI).

To assess the retinal protective effects of TAFA1, TAFA3, and TAFA4, 1×10⁹ vg of AAV8 was injected subretinally into both eyes of the mice, and after waiting up to 42 days, NaIO₃ was administered intravenously at a dose of 20 mg/kg to induce the AMD model. On day 9 after model induction (day 51 after SRI), fluorescein dye was injected via the tail vein, the fundus was visualized using a Micron-IV imaging camera (Phoenix), and FA images were obtained. On day 7 after model induction (day 49 after SRI), scotopic ERG was performed. Mice were placed on the ERG stage, ERG probes were placed in contact with the tail, head, and cornea, and A-wave and B-wave amplitudes were measured. ERG analysis was conducted using LabScribeERG (iWorx Data Acquisition Software).

To assess the retinal protective effect of TAFA2, 1×10⁹ vg of AAV8 was injected subretinally into both eyes of the mice, and after waiting up to 56 days, NaIO₃ was administered intravenously at a dose of 20 mg/kg to induce the AMD model. On day 9 after model induction (day 65 after SRI), fluorescein dye was injected via the tail vein, the fundus was visualized using a Micron-IV imaging camera (Phoenix), and FA images were obtained. On day 7 after model induction (day 63 after SRI), scotopic ERG was performed, and A-wave and B-wave amplitudes were measured and analyzed as described above.

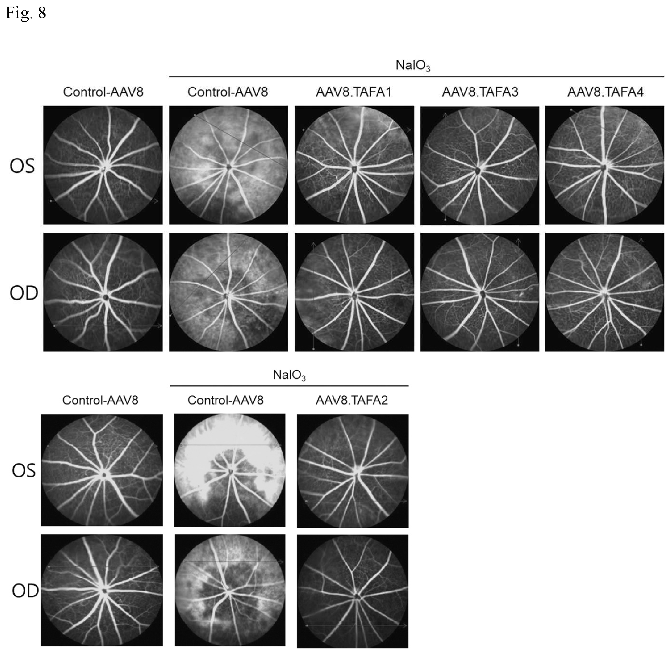

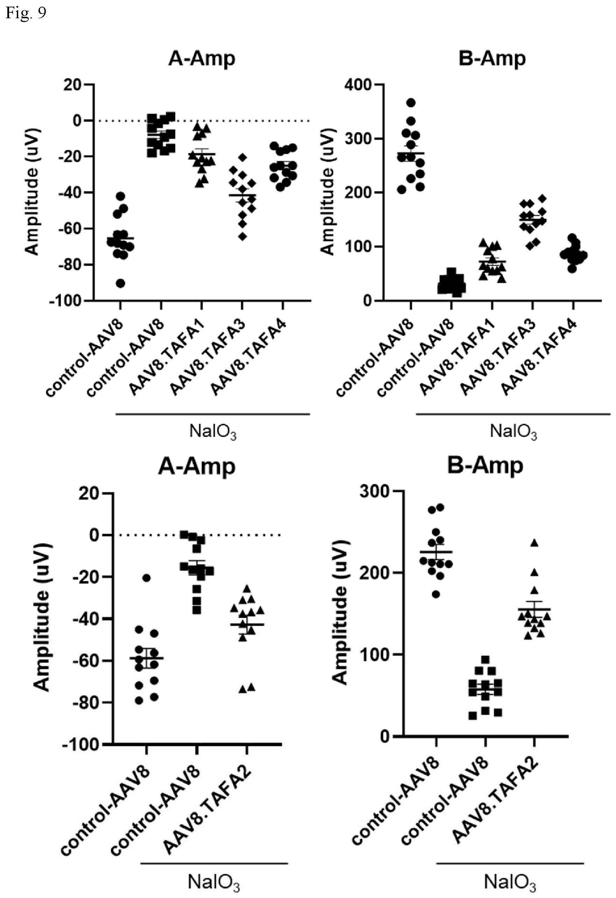

On FA images, leakage of fluorescein dye due to outer retinal damage was observed in the NaIO₃-treated group (control group, control AAV8-treated) compared with healthy animals (naïve, control AAV8-treated). In contrast, the groups administered AAV8.TAFA1, AAV8.TAFA2, AAV8.TAFA3, or AAV8.TAFA4 (test groups) showed an almost normal retinal appearance similar to that of the naïve group (Fig. 8). Scotopic ERG also revealed that NaIO3 caused marked reductions in the evoked A-wave and B-wave amplitudes, indicative of retinal abnormalities. In contrast, in the groups administered AAV8.TAFA1, AAV8.TAFA2, AAV8.TAFA3, or AAV8.TAFA4 (test groups), NaIO₃-induced changes in A-wave and B-wave amplitudes were restored (Fig. 9).

### 2-E. Comparison of sequence identity of interspecies TAFA1-4 variants

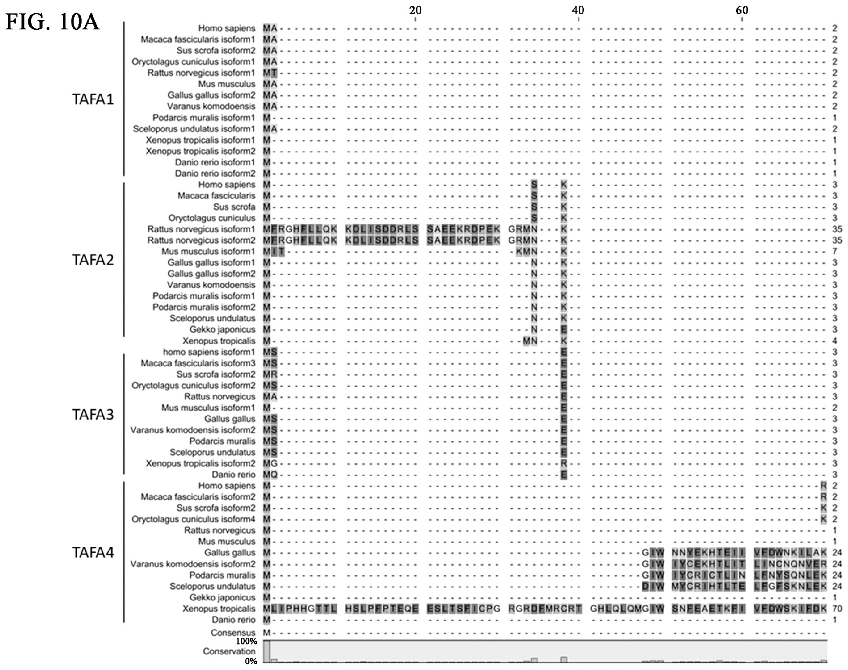

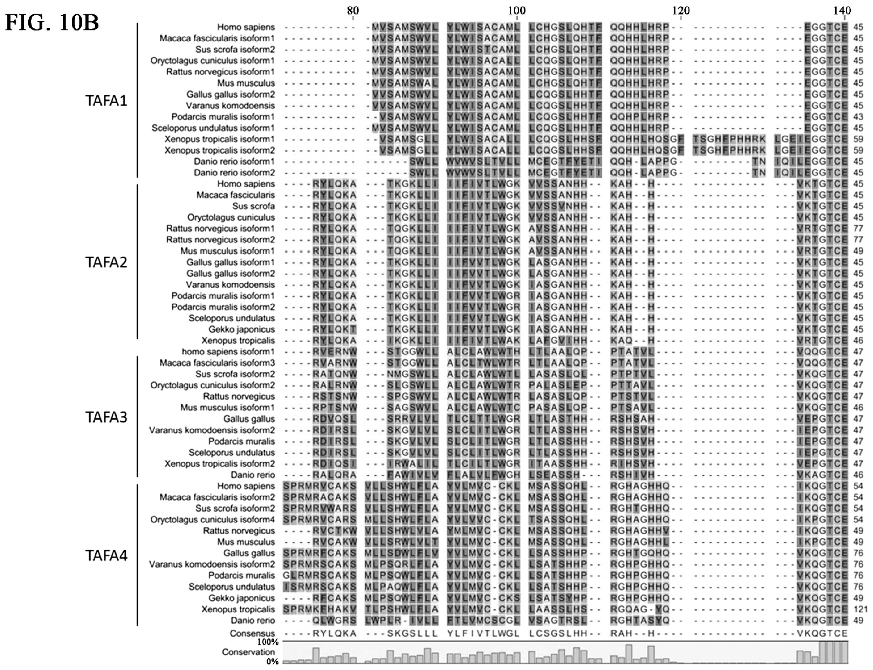

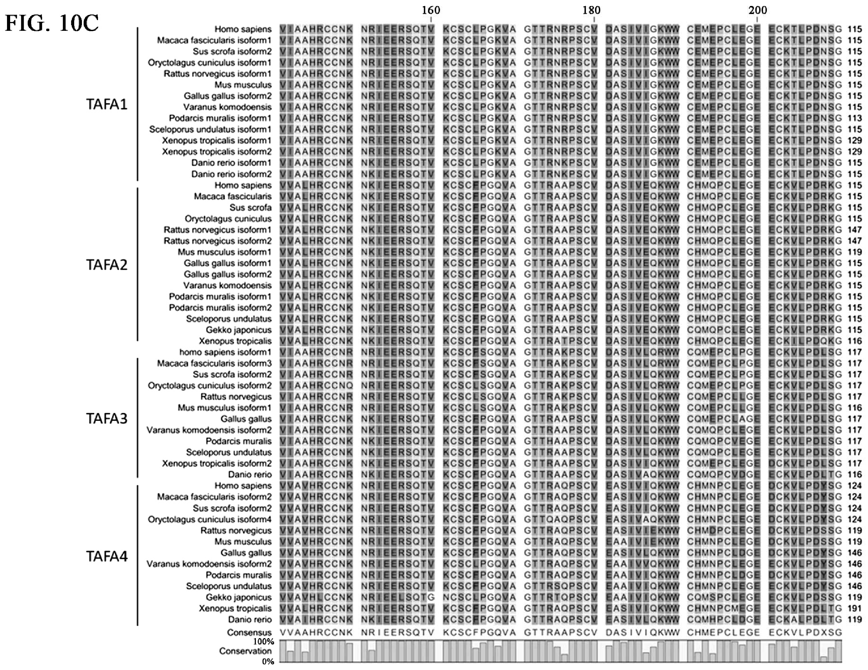

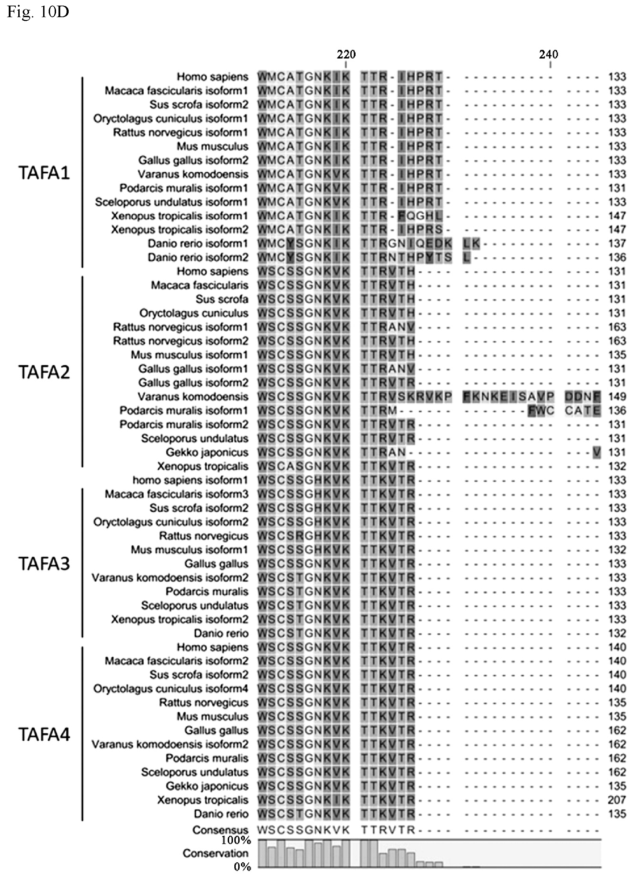

As confirmed in sections 2-C and 2-D, retinal protective effects were observed in interspecies variants of TAFA4 and in TAFA4 paralogs TAFA1-3 in the NaIO₃-induced retinal damage model. Therefore, we hypothesized that common sequences shared among various TAFA proteins may confer retinal protective activity, and we compared the sequence identity of full-length TAFA1, TAFA2, TAFA3, and TAFA4 proteins from vertebrates-mammals (human, monkey, pig, rabbit, rat, mouse), birds (chicken), reptiles (komodo dragon, wall lizard, fence lizard, gecko), amphibians (frog), and fish-to identify consensus sequences. The amino acid sequences of full-length TAFA1-4 in vertebrates are shown in FIGS. 10A-10D.

The 93-amino-acid sequence of human TAFA4 (IKQGTCEVVAVHRCCNKNRIEERSQTVKCSCFPGQVAGTTRAQPSCVEASIVIQKWW CHMNPCLEGEDCKVLPDYSGWSCSSGNKVKTTKVTR, SEQ ID NO: 87) showed very high sequence identity to TAFA4 sequences in other species and to consensus sequences of TAFA1-3 from human to fish. Sequence identities within the consensus region are summarized in Table 2.

**[Table 2]**

| Sequence identity between the common 93-amino-acid sequence of human TAFA4 and TAFA1, TAFA2, TAFA3, and TAFA4 from each species | | | | | |
|---|---|---|---|---|---|
| **TAFA family** | **species** | **scientific name** | **length** | **SEQ ID NO** | **percent identity** |
| TAFA4 | Human | *Homo sapiens* | 93 | 87 | 100.0% |
| TAFA1 | Human | *Homo sapiens* | 91 | 88 | 75.8% |
| TAFA1 | Monkey | *Macaca fascicularis* | 91 | 89 | 75.8% |
| TAFA1 | Pig | *Sus scrofa* | 91 | 90 | 75.8% |
| TAFA1 | Rabbit | *Oryctolagus cuniculus* | 91 | 91 | 75.8% |
| TAFA1 | Rat | *Rattus norvegicus* | 91 | 92 | 75.8% |
| TAFA1 | Mouse | *Mus musculus* | 91 | 93 | 75.8% |
| TAFA1 | Chicken | *Gallus gallus* | 91 | 94 | 75.8% |
| TAFA1 | Komodo dragon | *Varanus komodoensis* | 91 | 95 | 76.9% |
| TAFA1 | Wall lizard | *Podarcis muralis* | 91 | 96 | 76.9% |
| TAFA1 | Fence lizard | *Sceloporus undulatus* | 91 | 97 | 76.9% |
| TAFA1 | Frog | *Xenopus tropicalis_isoform1* | 92 | 98 | 76.1% |
| TAFA1 | Frog | *Xenopus tropicalis_isoform2* | 92 | 99 | 76.1% |
| TAFA1 | Fish | *Danio rerio_isoform1* | 93 | 100 | 74.2% |
| TAFA1 | Fish | *Danio rerio_isoform2* | 93 | 101 | 75.3% |
| TAFA2 | Human | *Homo sapiens* | 93 | 102 | 86.0% |
| TAFA2 | Monkey | *Macaca fascicularis* | 93 | 103 | 86.0% |
| TAFA2 | Pig | *Sus scrofa* | 93 | 104 | 86.0% |
| TAFA2 | Rabbit | *Oryctolagus cuniculus* | 93 | 105 | 86.0% |
| TAFA2 | Rat | *Rattus norvegicus_isoform1* | 93 | 106 | 82.8% |
| TAFA2 | Rat | *Rattus norvegicus_isoform2* | 93 | 107 | 84.9% |
| TAFA2 | Mouse | *Mus musculus* | 93 | 108 | 84.9% |
| TAFA2 | Chicken | *Gallus gallus_isoform1* | 93 | 109 | 83.9% |
| TAFA2 | Chicken | *Gallus gallus_isoform2* | 93 | 110 | 87.1% |
| TAFA2 | Komodo dragon | *Varanus komodoensis* | 93 | 111 | 84.9% |
| TAFA2 | Wall lizard | *Podarcis muralis_isoform1* | 91 | 112 | 85.7% |
| TAFA2 | Wall lizard | *Podarcis muralis_isoform2* | 93 | 113 | 87.1% |
| TAFA2 | Fence lizard | *Sceloporus undulatus* | 93 | 114 | 87.1% |
| TAFA2 | Gecko | *Gekko japonicus* | 92 | 115 | 83.7% |
| TAFA2 | Frog | *Xenopus tropicalis* | 93 | 116 | 84.9% |
| TAFA2 | Fish | *Danio rerio* | 93 | 117 | 82.8% |
| TAFA3 | Human | *Homo sapiens* | 93 | 118 | 82.8% |
| TAFA3 | Monkey | *Macaca fascicularis* | 93 | 119 | 82.8% |
| TAFA3 | Pig | *Sus scrofa* | 93 | 120 | 84.9% |
| TAFA3 | Rabbit | *Oryctolagus cuniculus* | 93 | 121 | 82.8% |
| TAFA3 | Rat | *Rattus norvegicus* | 93 | 122 | 82.8% |
| TAFA3 | Mouse | *Mus musculus* | 93 | 123 | 83.9% |
| TAFA3 | Chicken | *Gallus gallus* | 93 | 124 | 84.9% |
| TAFA3 | Komodo dragon | *Varanus komodoensis* | 93 | 125 | 86.0% |
| TAFA3 | Wall lizard | *Podarcis muralis* | 93 | 126 | 83.9% |
| TAFA3 | Fence lizard | *Sceloporus undulatus* | 93 | 127 | 86.0% |
| TAFA3 | Frog | *Xenopus tropicalis* | 93 | 128 | 87.1% |
| TAFA3 | Fish | *Danio rerio* | 93 | 129 | 81.7% |
| TAFA4 | Monkey | *Macaca fascicularis* | 93 | 130 | 100.0% |
| TAFA4 | Pig | *Sus scrofa* | 93 | 131 | 100.0% |
| TAFA4 | Rabbit | *Oryctolagus cuniculus* | 93 | 132 | 96.8% |
| TAFA4 | Rat | *Rattus norvegicus* | 93 | 133 | 96.8% |
| TAFA4 | Mouse | *Mus musculus* | 93 | 134 | 95.7% |
| TAFA4 | Chicken | *Gallus gallus* | 93 | 135 | 96.8% |
| TAFA4 | Komodo dragon | *Varanus komodoensis* | 93 | 136 | 94.6% |
| TAFA4 | Wall lizard | *Podarcis muralis* | 93 | 137 | 96.8% |
| TAFA4 | Fence lizard | *Sceloporus undulatus* | 93 | 138 | 95.7% |
| TAFA4 | Gecko | *Gekko japonicus* | 93 | 139 | 87.1% |
| TAFA4 | Frog | *Xenopus tropicalis* | 93 | 140 | 92.5% |
| TAFA4 | Fish | *Danio rerio* | 93 | 141 | 87.1% |

| | | | | | |
|---|---|---|---|---|---|
| Sequence identity was determined using Clustal Omega. | | | | | |

### Example 3. Evaluation of the efficacy of TAFA4 peptide fragments

Based on the results of Examples 1 and 2, the inventors synthesized three TAFA4 peptide fragments (TAFA4 fragments 1-3, F1-F3) as shown in Table 3 to identify fragments of TAFA4 exhibiting biological activity. Peptides were synthesized by Abclon.

**[Table 3]**

| Amino acid sequences of TAFA4 peptide fragments 1-3 | | |
|---|---|---|
| **peptide name** | **length (a.a.)** | **amino acid sequence, N'→C'** |
| TAFA4 fragment 1 (SEQ ID NO: 148) | 35 | HQIKQGTCEV VAVHRCCNKN RIEERSQTVK CSCFP |
| TAFA4 fragment 2 (SEQ ID NO: 75) | 31 | GQVAGTTRAQ PSCVEASIVI QKWWCHMNPC L |
| TAFA4 fragment 3 (SEQ ID NO: 52) | 29 | EGEDCKVLPD YSGWSCSSGN KVKTTKVTR |

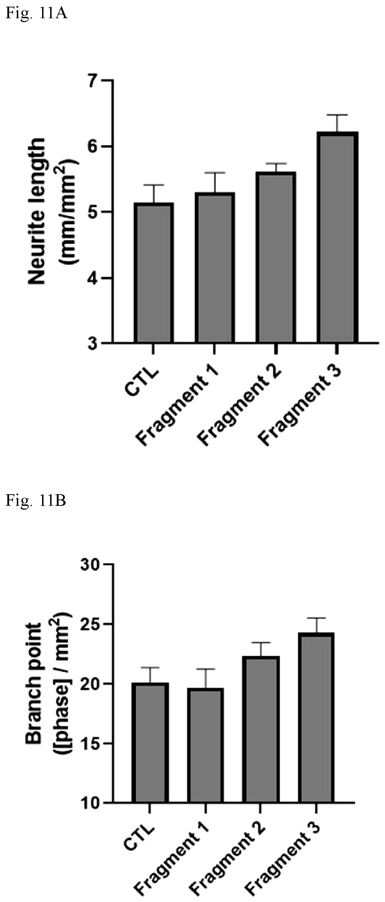

As in Example 1, MED17.11 cells resuspended in differentiation medium were plated at 1×10⁴ cells/mL, 100 µL per well, in 96-well cell culture plates. TAFA4 peptide fragments 1-3 were added at 5 µM, and an equal volume of dimethyl sulfoxide (DMSO, Sigma) was added to the control group, followed by incubation at 37°C in a 5% CO₂ incubator. After 4 days of culture, neurite length and the number of branch points were analyzed using an Incucyte system. Three independent experiments were performed, and an increasing trend in both neurite length and branch points was observed in the groups treated with TAFA4 fragment 2 (F2) and TAFA4 fragment 3 (F3) (FIGS. 11A and 11B).

The interspecies sequences of TAFA4 fragments 2 (F2) and 3 (F3) are shown in Tables 4 and 5, respectively.

**[Table 4]**

| Interspecies amino acid sequences of TAFA4 fragment 2 | | | |
|---|---|---|---|
| **species (scientific name)** | SEQ ID NO | SEQUENCE | a.a. |
| Human (*Homo sapiens*), Monkey (*Macaca fascicularis*), Pig (*Sus scrofa*) | SEQ ID NO: 75 | GQVAGTTRAQPSCVEASIVIQKWW CHMNPCL | 31 |
| Rabbit (*Oryctolagus cuniculus*) | SEQ ID NO: 76 | GQVAGTTQAQPSCVEASIVAQKW WCHMNPCL | 31 |
| Rat (*Rattus norvegicus*) | SEQ ID NO: 77 | GQVAGTTRAQPSCVEASIVIEKWW CHMDPCL | 31 |
| Mouse (*Mus musculus*) | SEQ ID NO: 78 | GQVAGTTRAQPSCVEAAIVIEKWW CHMNPCL | 31 |
| Chicken (*Gallus gallus*) | SEQ ID NO: 79 | GQVAGTTRAQPSCVEASIVLQKW WCHMNPCL | 31 |
| Komodo dragon (*Varanus komodoensis*) | SEQ ID NO: 80 | GQVAGTTRAQPSCVEAAIVVQKW WCHMNPCL | 31 |
| Wall lizard (*Podarcis muralis*) | SEQ ID NO: 81 | GQVAGTTRAQPSCVEAAIVIQKW WCHMNPCL | 31 |
| Fence lizard (*Sceloporus undulatus*) | SEQ ID NO: 82 | GQVAGTTRSQPSCVEAAIVIQKWW CHMNPCL | 31 |
| Gecko (*Gekko japonicus*) | SEQ ID NO: 83 | GQVAGTTRTQPSCVEAAIVIQKWW CQMSPCL | 31 |
| Frog (*Xenopus tropicalis*) | SEQ ID NO: 84 | GQVAGTTRAQPSCVEASIVIQKWW CHMNPCM | 31 |
| Fish (*Danio rerio*) | SEQ ID NO: 85 | GQVAGTTRAQPSCVEASIVLQKW WCQMHPCL | 31 |

The sequence reflecting fragment 2 and its interspecies variants corresponds to SEQ ID NO:147.

**[Table 5]**

| Interspecies amino acid sequences of TAFA4 fragment 3 | | | |
|---|---|---|---|
| **species (scientific name)** | SEQ ID NO | SEQUENCE | a.a. |
| Human (*Homo sapiens*), Monkey (*Macaca fascicularis*), Pig (*Sus scrofa*), Rabbit (*Oryctolagus cuniculus*) | SEQ ID NO: 52 | EGEDCKVLPDYSGWSCSSGNKVK TTKVTR | 29 |
| Rat (*Rattus norvegicus*), Mouse (*Mus musculus*) | SEQ ID NO: 53 | EGEDCKVLPDSSGWSCSSGNKVK TTKVTR | 29 |
| Chicken (*Gallus gallus*), Wall lizard (*Podarcis muralis*) | SEQ ID NO: 54 | DGEDCKVLPDYSGWSCSSGNKVK TTKVTR | 29 |
| Komodo dragon (*Varanus komodoensis*), Fence lizard (*Sceloporus undulatus*) | SEQ ID NO: 55 | EGEECKVLPDYSGWSCSSGNKVK TTKVTR | 29 |
| Gecko (*Gekko japonicus*) | SEQ ID NO: 56 | EGEECKVLPDSSGWSCSSGNKVK TTKVTR | 29 |
| Frog (*Xenopus tropicalis*) | SEQ ID NO: 57 | EGEECKVLPDLTGWSCSSGNKIKT TKVTR | 29 |
| Fish (*Danio rerio*) | SEQ ID NO: 58 | DGEECKALPDLTGWSCSTGNKVK TTKVTR | 29 |

The sequence reflecting fragment 3 and its interspecies variants corresponds to SEQ ID NO:144.

### Example 4. Evaluation of the efficacy of subdivided TAFA4 peptide fragments

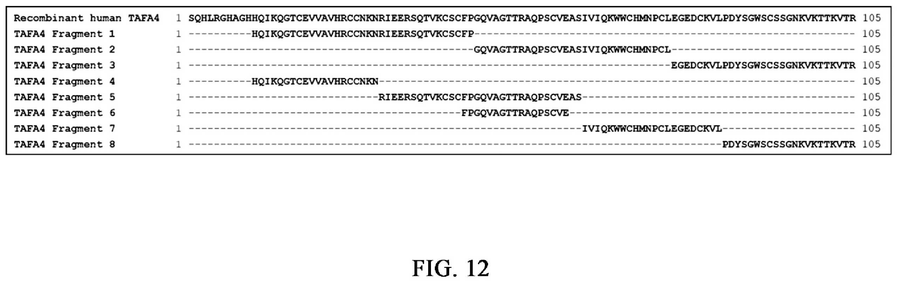

Based on Example 3, in which TAFA4 fragments 2 (F2) and 3 (F3) showed activity, further subdivided TAFA4 peptide fragments (TAFA4 fragments 4-8, F4-F8) were synthesized as shown in Table 6 to identify minimal TAFA4 peptide fragments exhibiting biological activity. Peptides were synthesized by Abclon, and the positions of each fragment are shown in Fig. 12.

**[Table 6]**

| Amino acid sequences of TAFA4 peptide fragments 4-8 | | |
|---|---|---|
| **peptide name** | **length** | **amino acid sequence, N'→C'** |
| | **(a.a.)** | |
| TAFA4 fragment 4 (SEQ ID NO: 149) | 20 | HQIKQGTCEV VAVHRCCNKN |
| TAFA4 fragment 5 (SEQ ID NO: 59) | 32 | RIEERSQTVK CSCFPGQVAG TTRAQPSCVE AS |
| TAFA4 fragment 6 (SEQ ID NO: 150) | 17 | FPGQVAGTTR AQPSCVE |
| TAFA4 fragment 7 (SEQ ID NO: 28) | 22 | IVIQKWWCHM NPCLEGEDCK VL |
| TAFA4 fragment 8 (SEQ ID NO: 151) | 21 | PDYSGWSCSS GNKVKTTKVT R |

Using the same experimental procedure as in Example 4, 5 µM of each TAFA4 peptide fragment or an equal volume of DMSO were added and incubated at 37°C (5% CO₂). After 4 days, cell images were acquired using an Incucyte (Sartorius), and neurite length and branch points were analyzed. Two independent experiments were performed, and statistical significance was evaluated by one-way ANOVA with Dunnett's post-hoc test.

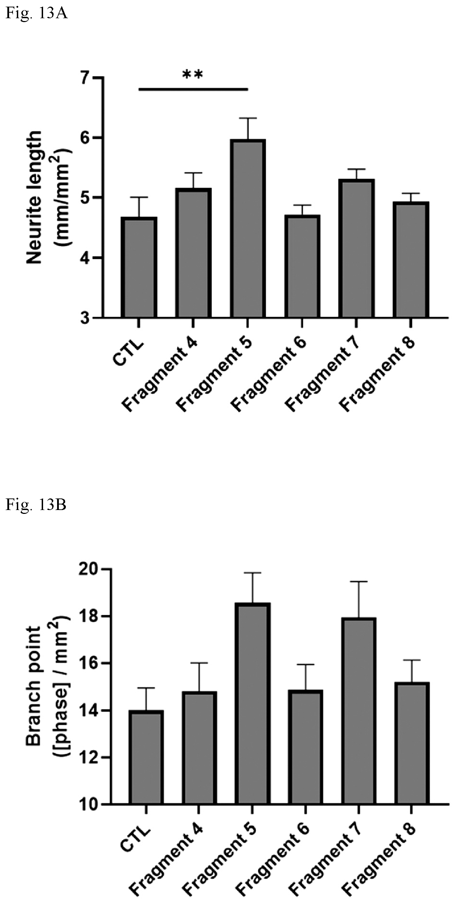

Neurite length tended to increase with TAFA4 fragments 5 (F5) and 7 (F7), with fragment 5 (F5) showing a statistically significant increase (Fig. 13A). Similarly, only TAFA4 fragments 5 (F5) and 7 (F7) showed a tendency to increase the number of branch points (Fig. 13B).

The interspecies sequence homology of TAFA4 fragments 5 (F5) and 7 (F7) is shown in Tables 7 and 8.

**[Table 7]**

| Comparison of interspecies sequence identity of TAFA4 peptide fragment 5 | | | |
|---|---|---|---|
| **Species (scientific name)** | **SEQ ID NO** | **amino acid sequence, N'→C'** | **percent identity** |
| Human (*Homo sapiens*), Monkey (*Macaca fascicularis*), Pig (*Sus scrofa*), Rat (*Rattus norvegicus*), Chicken (*Gallus gallus*), Frog (*Xenopus tropicalis*) | SEQ ID NO: 59 | RIEERSQTVK CSCFPGQVAG TTRAQPSCVE AS | 100.0% |
| Rabbit (*Oryctolagus cuniculus*) | SEQ ID NO: 60 | RIEERSQTVK CSCFPGQVAG TTQAQPSCVE AS | 96.9% |
| Mouse (*Mus musculus*), Wall lizard (*Podarcis muralis*) | SEQ ID NO: 61 | RIEERSQTVK CSCFPGQVAG TTRAQPSCVE AA | 96.9% |
| Komodo dragon (*Varanus komodoensis*) | SEQ ID NO: 62 | RIEERSQTVK CSCLPGQVAG TTRAQPSCVE AA | 93.8% |
| Fence lizard (*Sceloporus undulatus*) | SEQ ID NO: 63 | RIEERSQTVK CSCFPGQVAG TTRSQPSCVE AA | 93.8% |
| Gecko (*Gekko japonicus*) | SEQ ID NO: 64 | RIEELSQTGN CSCLPGQVAG TTRTQPSCVE AA | 81.3% |
| Fish (*Danio rerio*) | SEQ ID NO: 65 | KIEERSQTVK CSCFPGQVAG TTRAQPSCVE AS | 96.9% |

**[Table 8]**

| Comparison of interspecies sequence identity of TAFA4 peptide fragment 7 | | | |
|---|---|---|---|
| **Species (scientific name)** | **SEQ ID NO** | **amino acid sequence, N'→C'** | **percent identity** |
| Human (*Homo sapiens*), Monkey (*Macaca fascicularis*), Pig (*Sus scrofa*) | SEQ ID NO: 28 | IVIQKWWCHM NPCLEGEDCK VL | 100.0% |
| Rabbit (*Oryctolagus cuniculus*) | SEQ ID NO: 29 | IVAQKWWCHM NPCLEGEDCK VL | 95.5% |
| Rat(*Rattus norvegicus*) | SEQ ID NO: 30 | IVIEKWWCHM DPCLEGEDCK VL | 90.9% |
| Mouse(*Mus musculus*) | SEQ ID NO: 31 | IVIEKWWCHM NPCLEGEDCK VL | 95.5% |
| Chicken(*Gallus gallus*) | SEQ ID NO: 32 | IVLQKWWCHM NPCLDGEDCK VL | 90.9% |
| Komodo dragon(*Varanus komodoensis*) | SEQ ID NO: 33 | IVVQKWWCHM NPCLEGEECK VL | 90.9% |
| Wall lizard(*Podarcis muralis*) | SEQ ID NO: 34 | IVIQKWWCHM NPCLDGEDCK VL | 95.5% |
| Fence lizard(*Sceloporus undulatus*) | SEQ ID NO: 35 | IVIQKWWCHM NPCLEGEECK VL | 95.5% |
| Gecko(*Gekko japonicus*) | SEQ ID NO: 36 | IVIQKWWCQM SPCLEGEECK VL | 86.4% |
| Frog(*Xenopus tropicalis*) ( 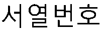 32) | SEQ ID NO: 37 | IVIQKWWCHM NPCMEGEECK VL | 90.9% |
| Fish(*Danio rerio*) | SEQ ID NO: 38 | IVLQKWWCQM HPCLDGEECK AL | 72.7% |

### Example 5. Evaluation of the efficacy of TAFA4 and its fragments on neuropathic pain in vivo

As described above, rhTAFA4 and TAFA4 fragments 2, 3, 5, and 7 (F2, F3, F5, and F7) increased neurite length and the number of branch points in MED17.11 cells. Based on this, we evaluated the efficacy of rhTAFA4 and TAFA4 fragments in a neuropathic pain model in vivo.

Six-week-old mice were anesthetized, and the skin between the gluteus maximus and biceps femoris of the left hind limb was incised to expose the sciatic nerve. After separating the exposed nerve from surrounding tissues, the proximal portion of the nerve where it branches into the tibial, peroneal, and sural nerves was ligated three times at 0.5-1 mm intervals using 6-0 silk (AILEE). The skin was sutured, and the animals were allowed to recover for one week. One week later, pain was assessed using the von Frey test. In the von Frey test, each monofilament was applied six times to the center of the plantar surface of the left hind paw, starting from the lowest force (0.008 g). If no pain response (lifting, shaking, licking, etc.) was observed, progressively higher-force monofilaments were used. The g value of the monofilament that elicited pain responses in at least three out of six applications was recorded, and the 50% threshold was calculated.

After confirming pain due to sciatic nerve ligation, rhTAFA4 and TAFA4 fragments were administered to evaluate the degree of pain alleviation. rhTAFA4 and TAFA4 fragments 1, 2, 3, 5, 6, and 7 (F1, F2, F3, F5, F6, and F7) were diluted in PBS to 200 µg/mL, and 10 µL (2 µg) per mouse was administered intrathecally at the L4-L5 level. One hour after administration, the von Frey test was repeated to evaluate pain relief.

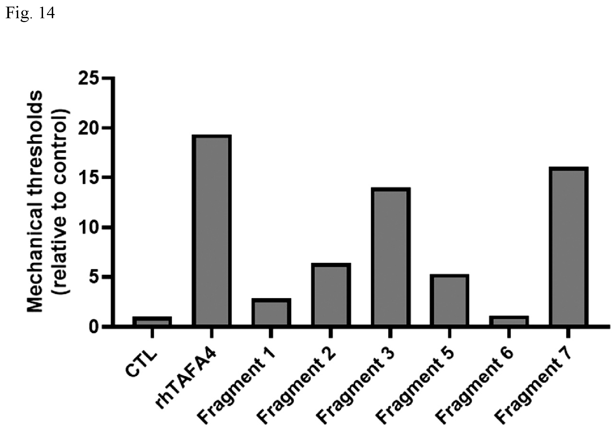

In the control group administered the same volume of PBS, pain due to nerve ligation persisted, indicating the presence of mechanical allodynia. In contrast, pain was alleviated in the group administered rhTAFA4. Pain also persisted in the groups administered TAFA4 fragments 1 and 6 (F1 and F6), whereas pain was alleviated in the groups administered TAFA4 fragments 2, 3, 5, and 7 (F2, F3, F5, and F7), which had shown clear trends of increasing neurite length and branch points (Fig. 14).

Fragments 2 and 5 that exhibited efficacy share the sequence GQVAGTTRAQPSCVEAS (17 amino acids, referred to as fragment 2.5), and fragments 3 and 7 share the sequence EGEDCKVL (8 amino acids, referred to as fragment 3.7). Therefore, the increases in neurite length and branch points observed in Examples 1-4, and the analgesic effects observed in this example, are considered to be attributable to polypeptides comprising the common sequence of fragment 2.5 or fragment 3.7.

Comparison of the sequence of TAFA4 fragment 2.5 with interspecies variant sequences of TAFA1-3 revealed that human TAFA4 fragment 2.5 differs by only one to four amino acids, indicating very high homology.

Similarly, comparison of the sequence of TAFA4 fragment 3.7 with interspecies variant sequences of TAFA1-3 showed that human TAFA4 fragment 3.7 differs by only one to three amino acids, confirming that fragment 3.7 also has very high homology.

The results of comparing TAFA4 fragments with interspecies variant sequences of TAFA1-3 are summarized in Tables 9 (fragment 2.5) and 10 (fragment 3.7).

**[Table 9]**

| TAFA protein fragment 2.5 exhibiting efficacy and its interspecies variant sequences | | | |
|---|---|---|---|
| **TAFA** | **Species** | **SEQ ID NO** | **Sequence, N'→C'** |
| TAFA4 | Human | SEQ ID NO: 15 | GQVAG TTRAQPSCVE AS |
| | Monkey | | |
| | Pig | | |
| | Rat | | |
| | Chicken | | |
| | Frog | | |
| | Fish | | |
| | Rabbit | SEQ ID NO: 16 | GQVAG TT**Q**AQPSCVE AS |
| | Mouse | SEQ ID NO: 17 | GQVAG TTRAQPSCVE A**A** |
| | Komodo dragon | | |
| | Wall lizard | | |
| | Fence lizard | SEQ ID NO: 18 | GQVAG TTR**S**QPSCVE A**A** |
| | Gecko | SEQ ID NO: 19 | GQVAG TTR**T**QPSCVE A**A** |
| TAFA1 | Human | SEQ ID NO: 20 | G**K**VAG TT**RNR**PSCV**D** AS |
| | Monkey | | |
| | Pig | | |
| | Rabbit | | |
| | Rat | | |
| | Mouse | | |
| | Chicken | | |
| | Komodo dragon | | |
| | Wall lizard | | |
| | Fence lizard | | |
| | Frog | | |
| | Fish | SEQ ID NO: 21 | G**K**VAG TTR**NK**PSCV**D** AS |
| | Gecko | SEQ ID NO: 22 | G**K**VAG TT**RNR**PSCV**D** -- |
| TAFA2 | Human | SEQ ID NO: 23 | GQVAG TTRA**A**PSCV**D** AS |
| | Monkey | | |
| | Pig | | |
| | Rabbit | | |
| | Rat | | |
| | Mouse | | |
| | Chicken | | |
| | Komodo dragon | | |
| | Wall lizard | | |
| | Fence lizard | | |
| | Gecko | | |
| | Fish | | |
| | Frog | SEQ ID NO: 24 | GQVAG TTRA**T**PSCV**D** AS |
| TAFA3 | Human | SEQ ID NO: 25 | GQVAG TTRA**K**PSCV**D** AS |
| | Monkey | | |
| | Pig | | |
| | Rabbit | | |
| | Rat | | |
| | Mouse | | |
| | Chicken | SEQ ID NO: 26 | GQVAG TTR**AA**PSCV**D** AS |
| | Komodo dragon | | |
| | Fence lizard | | |
| | Frog | | |
| | Fish | | |
| | Wall lizard | SEQ ID NO: 27 | GQVAG TT**H**A**A**PSCV**D** AS |

| | | | |
|---|---|---|---|
| Amino acids that differ are shown in bold. The sequence of fragment 2.5, including its interspecies variants, corresponds to SEQ ID NO:145. | | | |

**[Table 10]**

| TAFA protein fragment 3.7 exhibiting efficacy and its interspecies variant sequences | | | |
|---|---|---|---|
| **TAFA** | **Species** | **SEQ ID NO** | **Sequence, N'→C'** |
| TAFA4 | Human | SEQ ID NO: 1 | EGEDCK VL |
| | Monkey | | |
| | Pig | | |
| | Rabbit | | |
| | Rat | | |
| | Mouse | | |
| | Chicken | SEQ ID NO: 2 | **D**GEDCK VL |
| | Wall lizard | | |
| | Komodo dragon | SEQ ID NO: 3 | EGE**E**CK VL |
| | Fence lizard | | |
| | Gecko | | |
| | Frog | | |
| | Fish | SEQ ID NO: 4 | **D**GE**E**CK **A**L |
| TAFA1 | Human | SEQ ID NO: 5 | EGE**E**CK **T**L |
| | Monkey | | |
| | Pig | | |
| | Rabbit | | |
| | Rat | | |
| | Mouse | | |
| | Chicken | | |
| | Komodo dragon | | |
| | Wall lizard | | |
| | Fence lizard | | |
| | Frog | | |
| | Fish | | |
| TAFA2 | Human | SEQ ID NO: 6 | EG**EE**CK VL |
| | Monkey | | |
| | Pig | | |
| | Rabbit | | |
| | Rat | | |
| | Mouse | | |
| | Chicken | | |
| | Komodo dragon | | |
| | Wall lizard | | |
| | Fence lizard | | |
| | Gecko | | |
| | Frog | SEQ ID NO: 7 | EGE**E**CK **I**L |
| | Fish | SEQ ID NO: 8 | **D**GE**E**CK VL |
| TAFA3 | Human | SEQ ID NO: 9 | **P**GE**E**CK VL |
| | Monkey | | |
| | Pig | | |
| | Rabbit | | |
| | Rat | SEQ ID NO: 10 | **L**GE**E**CK VL |
| | Mouse | | |
| | Chicken | SEQ ID NO: 11 | **A**GE**E**CK VL |
| | Komodo dragon | SEQ ID NO: 12 | EGE**E**CK VL |
| | Wall lizard | | |
| | Fence lizard | | |
| | Frog | SEQ ID NO: 13 | EGEDCK VL |
| | Fish | SEQ ID NO: 14 | **D**GE**E**CK VL |

| | | | |
|---|---|---|---|
| Amino acids that differ are shown in bold. The sequence of fragment 3.7, including its interspecies variants, corresponds to SEQ ID NO:142. | | | |

### Example 6. Comparison of homology between TAFA4 peptide fragments and TAFA1-3 peptide fragments

In Example 3, we confirmed that interspecies homology of TAFA4 fragments was at least 70% (≥81.3% for F5 and ≥72.7% for F7). High sequence identity has been reported between TAFA4 and other TAFA family proteins. Therefore, we hypothesized that common sequences among various TAFA peptide fragments may confer efficacy in increasing neurite length and branch points, and we compared sequence homology between TAFA1, TAFA2, and TAFA3 peptide fragments from vertebrates-mammals (human, monkey, pig, rabbit, rat, mouse), birds (chicken), reptiles (komodo dragon, wall lizard, fence lizard, gecko), amphibians (frog), and fish-and human TAFA4 fragments 5 (F5) and 7 (F7), as summarized in Tables 11-16, to identify consensus sequences.

**[Table 11]**

| Comparisons of sequence identity between human TAFA4 fragment 5 and TAFA1 fragment 5 | | | |
|---|---|---|---|
| **species (scientific name)** | **SEQ ID NO** | **amino acid sequence, N'→C'** | **percent identity** |
| Human (Homo sapiens), Monkey (Macaca fascicularis), Pig (Sus scrofa), Rabbit (Oryctolagus cuniculus), Rat (Rattus norvegicus), Mouse (Mus musculus), Chicken (Gallus gallus), Komodo dragon (Varanus komodoensis), Wall lizard (Podarcis muralis), Fence lizard (Sceloporus undulatus), Frog (Xenopus tropicalis) | SEQ ID NO: 66 | RIEERSQTVK CSCLPGKVAG TTRNRPSCVD AS | 84.4% |
| Gecko (Gekko japonicus) | SEQ ID NO: 67 | RIEERSQTVK CSCLPGKVAG TTRNRPSCVD -- | 78.1% |
| Fish (Danio rerio) | SEQ ID NO: 68 | KIEERSQTVK CSCLPGKVAG TTRNKPSCVD AS | 81.3% |

**[Table 12]**

| Comparisons of sequence identity between human TAFA4 fragment 5 and TAFA2 fragment 5 | | | |
|---|---|---|---|
| **species (scientific name)** | **SEQ ID NO** | **amino acid sequence, N'→C'** | **percent identity** |
| Human (Homo sapiens), Monkey (Macaca fascicularis), Pig (Sus scrofa), Rabbit (Oryctolagus cuniculus), Rat (Rattus norvegicus), Mouse (Mus musculus), Chicken (Gallus gallus), Komodo dragon (Varanus komodoensis), Wall lizard (Podarcis muralis), Fence lizard (Sceloporus undulatus), Gecko (Gekko japonicus), Fish (Danio rerio) | SEQ ID NO: 69 | KIEERSQTVK CSCFPGQVAG TTRAAPSCVD AS | 90.6% |
| Frog (Xenopus tropicalis) | SEQ ID NO: 70 | KIEERSQTVK CSCFPGQVAG TTRATPSCVD AS | 90.6% |

**[Table 13]**

| Comparisons of sequence identity between human TAFA4 fragment 5 and TAFA3 fragment 5 | | | |
|---|---|---|---|
| **species (scientific name)** | **SEQ ID NO** | **amino acid sequence, N'→C'** | **percent identity** |
| Human (Homo sapiens), Monkey (Macaca fascicularis), Pig (Sus scrofa) | SEQ ID NO: 71 | RIEERSQTVK CSCFSGQVAG TTRAKPSCVD AS | 90.6% |
| Rabbit (Oryctolagus cuniculus), Rat (Rattus norvegicus), Mouse (Mus musculus) | SEQ ID NO: 72 | RIEERSQTVK CSCLSGQVAG TTRAKPSCVD AS | 87.5% |
| Chicken (Gallus gallus), Komodo dragon (Varanus komodoensis), Fence lizard (Sceloporus undulatus), Frog (Xenopus tropicalis), Fish (Danio rerio) | SEQ ID NO: 73 | KIEERSQTVK CSCFPGQVAG TTRAAPSCVD AS | 90.6% |
| Wall lizard (Podarcis muralis) | SEQ ID NO: 74 | KIEERSQTVK CSCFPGQVAG TTHAAPSCVD AS | 87.5% |

| | | | |
|---|---|---|---|
| The sequence of fragment 5, including its interspecies variants, corresponds to SEQ ID NO:146. | | | |

**[Table 14]**

| Comparisons of sequence identity between human TAFA4 fragment 7 and TAFA1 fragment 7 | | | |
|---|---|---|---|
| **species (scientific name)** | **SEQ ID NO** | **amino acid sequence, N'→C'** | **percent identity** |
| Human (Homo sapiens), Monkey (Macaca fascicularis), Pig (Sus scrofa), Rabbit (Oryctolagus cuniculus), Rat (Rattus norvegicus), Mouse (Mus musculus), Chicken (Gallus gallus), Komodo dragon (Varanus komodoensis), Wall lizard (Podarcis muralis), Fence lizard (Sceloporus undulatus), Frog (Xenopus tropicalis), Fish (Danio rerio) | SEQ ID NO: 39 | IVIGKWWCEM EPCLEGEECK TL | 77.3% |

**[Table 15]**

| Comparisons of sequence identity between human TAFA4 fragment 7 and TAFA2 fragment 7 | | | |
|---|---|---|---|
| **species (scientific name)** | **SEQ ID NO** | **amino acid sequence, N'→C'** | **percent identity** |
| Human (Homo sapiens), Monkey (Macaca fascicularis), Pig (Sus scrofa), Rabbit (Oryctolagus cuniculus), Rat (Rattus norvegicus), Mouse (Mus musculus), Chicken (Gallus gallus), Komodo dragon (Varanus komodoensis), Wall lizard (Podarcis muralis), Fence lizard (Sceloporus undulatus) | SEQ ID NO: 40 | IVEQKWWCHM QPCLEGEECK VL | 86.4% |
| Gecko (Gekko japonicus) | SEQ ID NO: 41 | IVEQKWWCQM QPCLEGEECK VL | 81.8% |
| Frog (Xenopus tropicalis) | SEQ ID NO: 42 | IVEQKWWCHM QPCLEGEECK IL | 81.8% |
| Fish (Danio rerio) | SEQ ID NO: 43 | IVAQKWWCQM QPCMDGEECK VL | 72.7% |

**[Table 16]**

| Comparisons of sequence identity between human TAFA4 fragment 7 and TAFA3 fragment 7 | | | |
|---|---|---|---|
| **species (scientific name)** | **SEQ ID NO** | **amino acid sequence, N'→C'** | **percent identity** |
| Human (Homo sapiens), Monkey (Macaca fascicularis), Rabbit (Oryctolagus cuniculus) | SEQ ID NO: 44 | IVLQRWWCQM EPCLPGEECK VL | 72.7% |
| Pig (Sus scrofa) | SEQ ID NO: 45 | IVLQKWWCQM EPCLPGEECK VL | 77.3% |
| Rat (Rattus norvegicus), Mouse (Mus musculus) | SEQ ID NO: 46 | IVLQKWWCQM EPCLLGEECK VL | 77.3% |
| Chicken (Gallus gallus) | SEQ ID NO: 47 | IVLQKWWCQM EPCLAGEECK VL | 77.3% |
| Komodo dragon (Varanus komodoensis), Fence lizard (Sceloporus undulatus) | SEQ ID NO: 48 | IVLQKWWCQM QPCLEGEECK VL | 81.8% |
| Wall lizard (Podarcis muralis) | SEQ ID NO: 49 | IVLQKWWCQM QPCVEGEECK VL | 77.3% |
| Frog (Xenopus tropicalis) | SEQ ID NO: 50 | IVIQKWWCQM EPCLEGEDCK VL | 90.9% |
| Fish (Danio rerio) | SEQ ID NO: 51 | IVAQKWWCQM QPCVDGEECK VL | 72.7% |

| | | | |
|---|---|---|---|
| The sequence of fragment 7, including its interspecies variants, corresponds to SEQ ID NO:143. | | | |

Additionally, the interspecies sequences of fragment 3 of TAFA1-3 (Table 17) and of fragment 2 of TAFA1-3 (Table 18) are as follows:

**[Table 17]**

| Interspecies sequences of fragment 3 of TAFA1-3 | | | |
|---|---|---|---|
| **TAFA** | **Species** | **SEQ ID NO** | **Sequence, N'→ C'** |
| TAFA1 | Human, Monkey, Pig, Rabbit, Rat, Mouse, Chicken, Frog(isoform2) | SEQ ID NO: 152 | EGEECKTLPDNSGWMCATGNKIKT TR-IH |
| TAFA1 | Komodo dragon, Wall lizard, Fence lizard | SEQ ID NO: 153 | EGEECKTLPDNSGWMCATGNKVKT TR-IH |
| TAFA1 | Frog(isoform1) | SEQ ID NO: 154 | EGEECKTLPDNSGWMCATGNKIKT TR-FQ |
| TAFA1 | Fish(isoform1) | SEQ ID NO: 155 | EGEECKTLPDNSGWMCYSGNKIKT TRGNI |
| TAFA1 | Fish(isoform2) | SEQ ID NO: 156 | EGEECKTLPDNSGWMCYSGNKIKT TRNTH |
| TAFA2 | Human, Monkey, Pig, Rabbit, Rat(isoform2), Mouse | SEQ ID NO: 157 | EGEECKVLPDRKGWSCSSGNKVKT TRVTH |
| TAFA2 | Rat(isoform1), Chicken(form1_) | SEQ ID NO: 158 | EGEECKVLPDRKGWSCSSGNKVKT TRANV |
| TAFA2 | Chicken(isoform2), Wall lizard(2), Fence lizard | SEQ ID NO: 159 | EGEECKVLPDRKGWSCSSGNKVKT TRVTR |
| TAFA2 | Komodo dragon | SEQ ID NO: 160 | EGEECKVLPDRKGWSCSSGNKVKT TRVSK |
| TAFA2 | Wall lizard | SEQ ID NO: 161 | EGEECKVLPDRKGWSCSSGNKVKT TRM-- |
| TAFA2 | Gecko | SEQ ID NO: 162 | EGEECKVLPDRKGWSCSSGNKVKT TRAN- |
| TAFA2 | Frog | SEQ ID NO: 163 | EGEECKILPDQKGWSCASGNKVKT TKVTR |
| TAFA2 | Fish | SEQ ID NO: 164 | DGEECKVLPDLKGWSCSTGNKVKT TKVTR |
| TAFA3 | Human, Monkey, Pig, Rabbit | SEQ ID NO: 165 | PGEECKVLPDLSGWSCSSGHKVKTT KVTR |
| TAFA3 | Rat | SEQ ID NO: 166 | LGEECKVLPDLSGWSCSRGHKVKT TKVTR |
| TAFA3 | Mouse | SEQ ID NO: 167 | LGEECKVLPDLSGWSCSSGHKVKT TKVTR |
| TAFA3 | Chicken | SEQ ID NO: 168 | AGEECKVLPDLSGWSCSSGNKVKT TKVTR |
| TAFA3 | Komodo dragon, Wall lizard, Fence lizard | SEQ ID NO: 169 | EGEECKVLPDLSGWSCSTGNKVKT TKVTR |
| TAFA3 | Frog | SEQ ID NO: 170 | EGEDCKVLPDLSGWSCSTGNKVKT TKVTR |
| TAFA3 | Fish | SEQ ID NO: 171 | DGEECKVLPDLTGWSCSTGNKVKT TKVTR |

**[Table 18]**

| Interspecies sequences of fragment 2 of TAFA1-3 | | | |
|---|---|---|---|
| **TAFA** | **Species** | **SEQ ID NO** | **Sequence, N'→ C'** |
| TAFA 1 | Human, Monkey, Pig, Rabbit, Rat, Mouse, Chicken, Komodo dragon, Wall lizard, Fence lizard, Frog | SEQ ID NO: 172 | GKVAGTTRNRPSCVDASIVIGKWWCEMEPC L |
| TAFA 1 | Fish | SEQ ID NO: 173 | GKVAGTTRNKPSCVDASIVIGKWWCEMEP CL |
| TAFA 2 | Human, Monkey, Pig, Rabbit, Rat, Mouse, Chicken, Komodo dragon, Wall lizard, Fence lizard | SEQ ID NO: 174 | GQVAGTTRAAPSCVDASIVEQKWWCHMQP CL |
| TAFA 2 | Gecko | SEQ ID NO: 175 | GQVAGTTRAAPSCVDASIVEQKWWCQMQP CL |
| TAFA 2 | Frog | SEQ ID NO: 176 | GQVAGTTRATPSCVDASIVEQKWWCHMQP CL |
| TAFA 2 | Fish | SEQ ID NO: 177 | GQVAGTTRAAPSCVDASIVAQKWWCQMQP CM |
| TAFA 3 | Human, Monkey, Rabbit | SEQ ID NO: 178 | GQVAGTTRAKPSCVDASIVLQRWWCQMEP CL |
| TAFA 3 | Pig, Rat, Mouse | SEQ ID NO: 179 | GQVAGTTRAKPSCVDASIVLQKWWCQMEP CL |
| TAFA 3 | Chicken | SEQ ID NO: 180 | GQVAGTTRAAPSCVDASIVLQKWWCQMEP CL |
| TAFA 3 | Komodo dragon, Fence lizard | SEQ ID NO: 181 | GQVAGTTRAAPSCVDASIVLQKWWCQMQP CL |
| TAFA 3 | Wall lizard | SEQ ID NO: 182 | GQVAGTTHAAPSCVDASIVLQKWWCQMQP CV |
| TAFA 3 | Frog | SEQ ID NO: 183 | GQVAGTTRAAPSCVDASIVIQKWWCQMEP CL |
| TAFA 3 | Fish | SEQ ID NO: 184 | GQVAGTTRAAPSCVDASIVAQKWWCQMQP CV |

All publications, patents, patent applications, and other documents cited herein are incorporated by reference in their entireties for all purposes, regardless of whether each individual publication, patent, patent application, or other document is explicitly stated to be incorporated by reference for all purposes.

While the present disclosure has been described with reference to the foregoing embodiments, those skilled in the art will appreciate that various modifications and changes can be made, such as addition, alteration, deletion, or insertion of components, without departing from the spirit of the disclosure as set forth in the claims. Such modifications and changes are to be understood as falling within the scope of the present disclosure.

## Claims

1. A polypeptide having the ability to increase neurite length or the number of branch points, wherein the polypeptide:
(i) consists of 8 to 61 amino acid residues; and
(ii) comprises a sequence having at least 50% sequence identity to the amino acid sequence of SEQ ID NO: 1 or SEQ ID NO: 15.

2. The polypeptide according to claim 1, wherein the amino acid sequence of the polypeptide comprises the amino acid sequence of the following General Formula 1 from the N-terminus to the C-terminus:
General Formula 1: X1-G-E-X2-C-K-X3-L
wherein in General Formula 1,
X1 is E, D, P, L, or A,
X2 is D or E, and
X3 is T, V, I, or A.

3. The polypeptide according to claim 2, wherein the amino acid sequence of the polypeptide comprises the amino acid sequence of SEQ ID NO: 142.

4. The polypeptide according to claim 2, wherein the amino acid sequence of the polypeptide comprises an amino acid sequence selected from the group consisting of SEQ ID NOs: 1 to 14.

5. The polypeptide according to claim 2, wherein the amino acid sequence of the polypeptide consists of 8 to 43 amino acid residues.

6. The polypeptide according to claim 1, wherein the amino acid sequence of the polypeptide comprises the amino acid sequence of the following General Formula 2 from the N-terminus to the C-terminus:
General Formula 2: I-V-X4-X5-X6-W-W-C-X7-M-X8-P-C-X9-X1-G-E-X2-C-K-X3-L
wherein in General Formula 2,
X1 is E, D, P, L, or A,
X2 is D or E,
X3 is T, V, I, or A,
X4 is I, E, A, L, or V,
X5 is Q, E, or G,
X6 is K or R,
X7 is E, H, or Q,
X8 is E, Q, N, D, S, or H, and
X9 is L, M, or V.

7. The polypeptide according to claim 6, wherein the amino acid sequence of the polypeptide comprises the amino acid sequence of SEQ ID NO: 143.

8. The polypeptide according to claim 6, wherein the amino acid sequence of the polypeptide comprises an amino acid sequence selected from the group consisting of SEQ ID NOs: 28 to 51.

9. The polypeptide according to claim 1, wherein the amino acid sequence of the polypeptide comprises the amino acid sequence of the following General Formula 3 from the N-terminus to the C-terminus:
General Formula 3: X1-G-E-X2-C-K-X3-L-P-D-X4-X5-G-W-S-C-S-X6-G-N-K-X7-K-T-T-K-V-T-R
wherein in General Formula 3,
X1 is E, D, P, L, or A,
X2 is D or E,
X3 is T, V, I, or A,
X4 is Y, S, or L,
X5 is S or T,
X6 is S or T, and
X7 is V or I.

10. The polypeptide according to claim 9, wherein the amino acid sequence of the polypeptide comprises the amino acid sequence of SEQ ID NO: 144.

11. The polypeptide according to claim 9, wherein the amino acid sequence of the polypeptide comprises an amino acid sequence selected from the group consisting of SEQ ID NOs: 52 to 58.

12. The polypeptide according to claim 1, wherein the amino acid sequence of the polypeptide comprises the amino acid sequence of the following General Formula 4 from the N-terminus to the C-terminus:
General Formula 4: G-X1-V-A-G-T-T-X2-X3-X4-P-S-C-V-X5-X6-X7
wherein in General Formula 4,
X1 is Q or K,
X2 is R, H, or Q,
X3 is A, N, S, or T,
X4 is R, A, Q, K, or T,
X5 is D or E,
X6 is A or absent, and
X7 is S, A, or absent.

13. The polypeptide according to claim 12, wherein the amino acid sequence of the polypeptide comprises the amino acid sequence of SEQ ID NO: 145.

14. The polypeptide according to claim 12, wherein the amino acid sequence of the polypeptide comprises an amino acid sequence selected from the group consisting of SEQ ID NOs: 15 to 27.

15. The polypeptide according to claim 12, wherein the amino acid sequence of the polypeptide consists of 15 to 46 amino acid residues.

16. The polypeptide according to claim 1, wherein the amino acid sequence of the polypeptide comprises the amino acid sequence of the following General Formula 5 from the N-terminus to the C-terminus:
General Formula 5: X8-I-E-E-X9-S-Q-T-X10-X11-C-S-C-X12-X13-G-X1-V-A-G-T-T-X2-X3-X4-P-S-C- V-X5-X6-X7
wherein in General Formula 5,
X1 is Q or K,
X2 is R, H, or Q,
X3 is A, N, S, or T,
X4 is R, A, Q, K, or T,
X5 is D or E,
X6 is A or absent,
X7 is S, A, or absent,
X8 is R or K,
X9 is R or L,
X10 is V or G,
X11 is K or N,
X12 is F or L, and
X13 is P or S.

17. The polypeptide according to claim 16, wherein the amino acid sequence of the polypeptide comprises the amino acid sequence of SEQ ID NO: 146.

18. The polypeptide according to claim 16, wherein the amino acid sequence of the polypeptide comprises an amino acid sequence selected from the group consisting of SEQ ID NOs: 59 to 74.

19. The polypeptide according to claim 1, wherein the amino acid sequence of the polypeptide comprises the amino acid sequence of the following General Formula 6 from the N-terminus to the C-terminus:
General Formula 6: G-X1-V-A-G-T-T-X2-X3-X4-P-S-C-V-X5-X6-X7-I-V-X8-X9-K-W-W-C-X10-M-X1 1-P-C-X12
wherein in General Formula 6,
X1 is Q or K,
X2 is R, H, or Q,
X3 is A, N, S, or T,
X4 is R, A, Q, K, or T,
X5 is D or E,
X6 is A or absent,
X7 is S, A, or absent,
X8 is I, A, V, or L,
X9 is Q or E,
X10 is H or Q,
X11 is N, D, S, or H, and
X12 is L or M.

20. The polypeptide according to claim 19, wherein the amino acid sequence of the polypeptide comprises the amino acid sequence of SEQ ID NO: 147.

21. The polypeptide according to claim 19, wherein the amino acid sequence of the polypeptide comprises an amino acid sequence selected from the group consisting of SEQ ID NOs: 75 to 85.

22. The polypeptide according to claim 1, wherein the amino acid sequence of the polypeptide comprises an amino acid sequence selected from the group consisting of SEQ ID NOs: 152 to 184.

23. A nucleic acid molecule encoding the polypeptide according to any one of claims 1 to 22.

24. A vector comprising the nucleic acid molecule according to claim 23.

25. A recombinant viral particle comprising the vector according to claim 24 and a capsid protein.

26. The recombinant viral particle according to claim 25, wherein the virus is an AAV.

27. A cell comprising the vector according to claim 24.

28. A cell transformed with the vector according to claim 24.

29. A composition comprising the polypeptide according to any one of claims 1 to 22, a nucleic acid molecule encoding the polypeptide, a vector comprising the nucleic acid molecule, a recombinant viral particle comprising the vector and a capsid protein, a cell comprising the vector, a cell transformed with the vector, or any combination thereof.

30. The composition according to claim 29, wherein the composition is a pharmaceutical composition.

31. The composition according to claim 30, wherein the pharmaceutical composition is a composition for preventing or treating a retinal neurodegenerative disease.

32. The composition according to claim 31, wherein the retinal neurodegenerative disease comprises retinopathy, choroidal neovascularization, maculopathy, macular degeneration, retinal degeneration, macular edema, retinal edema, macular swelling, retinal swelling, color vision deficiency, retinal cell degeneration, retinal vascular occlusion, retinal detachment, inherited retinal disease, or any combination thereof.

33. The composition according to claim 30, wherein the pharmaceutical composition is a composition for preventing or treating neuropathic pain.

34. The composition according to claim 33, wherein the neuropathic pain is allodynia, hyperalgesia, hyperesthesia, or dysesthesia.

35. The composition according to claim 33, wherein the neuropathic pain is central neuropathic pain or peripheral neuropathic pain.

36. A method for producing a composition, comprising a step of preparing a composition comprising the polypeptide according to any one of claims 1 to 22, a nucleic acid molecule encoding the polypeptide, a vector comprising the nucleic acid molecule, a recombinant viral particle comprising the vector and a capsid protein, a cell comprising the vector, a cell transformed with the vector, or any combination thereof.

37. A therapeutic use for the manufacture of a medicament comprising the polypeptide according to any one of claims 1 to 22, a nucleic acid molecule encoding the polypeptide, a vector comprising the nucleic acid molecule, a recombinant viral particle comprising the vector and a capsid protein, a cell comprising the vector, a cell transformed with the vector, or any combination thereof.

38. A method for preventing or treating a disease or disorder in a subject in need thereof, comprising administering to the subject a composition comprising the polypeptide according to any one of claims 1 to 22, a nucleic acid molecule encoding the polypeptide, a vector comprising the nucleic acid molecule, a recombinant viral particle comprising the vector and a capsid protein, a cell comprising the vector, a cell transformed with the vector, or any combination thereof.
